# EUROPEAN PATENT APPLICATION

(11) **EP 4 656 637 A1**
(43) Date of publication of application: **03.12.2025**
(21) Application number: 24747197.2
(22) Date of filing: 18.01.2024
(51) Int. Cl.: C07D 401/14, C07D 471/04, C07D 519/00, H10K 50/16, H10K 50/17, H10K 50/165, H10K 85/60

(54) **COMPOUND, AND LIGHT-EMITTING ELEMENT, DISPLAY DEVICE, LIGHTING DEVICE AND PHOTOSENSITIZER EACH CONTAINING SAME**

(30) Priority: 26.01.2023 JP 2023010002
(71) Applicant: Toray Industries, Inc., Tokyo 103-8666 (JP)
(72) Inventor: KOTANI Ryota, Otsu-shi, Shiga 520-8558 (JP); NAGAO Kazumasa, Otsu-shi, Shiga 520-8558 (JP)
(74) Representative: Hoefer & Partner Patentanwälte mbB
(86) International application number: PCT/JP2024/001236
(87) International publication number: WO 2024/157868

(57) **Abstract**

The main object of the present invention is to provide an organic EL element that has high luminance efficiency and long service life. It is a compound as represented by the general formula (1) given below: (In the general formula (1), R¹ and R² are each independently one selected from the group consisting of substituted or unsubstituted aryl groups and substituted or unsubstituted heteroaryl groups. R¹ and R² may be linked to each other to form a ring structure. R³ is one selected from the group consisting of substituted or unsubstituted alkyl groups, substituted or unsubstituted aryl groups, and substituted or unsubstituted heteroaryl groups. L denotes a substituted or unsubstituted arylene group or a substituted or unsubstituted heteroarylene group. A denotes a structure as represented by the general formula (2) or (3) given below.) (In the general formulae (2) and (3), R⁴ to R¹⁴ are each independently one selected from the group consisting of a hydrogen atom, substituted or unsubstituted alkyl groups, substituted or unsubstituted alkoxy groups, substituted or unsubstituted aryl groups, and substituted or unsubstituted heteroaryl groups. Here, at least one of R⁴ to R¹¹ or R¹² to R¹⁴ is bonded directly to L. R⁷ and R⁸ may be linked to each other to form a ring structure. X¹ to X⁸ are each independently a carbon atom or a nitrogen atom. Here, at least either of X¹ and X² is a nitrogen atom, and at least one of X³ to X⁸ is a nitrogen atom.)

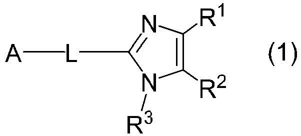

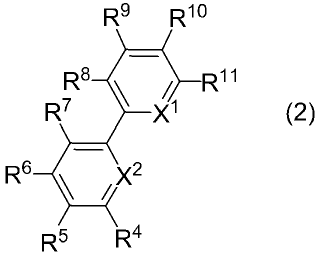

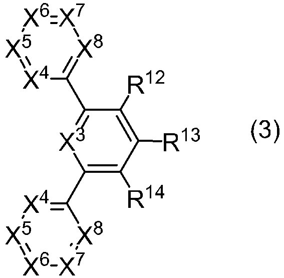

## Description

### TECHNICAL FIELD

The present invention relates to a compound, and to a light emitting element, a display device, an illumination device, and a photosensitizer including the compound.

### BACKGROUND ART

In recent years, efforts toward practical application of organic EL devices, including their adoption in displays for televisions and smartphones, have advanced steadily. However, existing organic EL devices still have many technical problems. In particular, major issues include the achievement of highly efficient light emission and the development of organic EL devices having prolonged lifetime.

For tackling these issues, many compounds have been developed so far, including imidazophenanthroline derivatives substituted with specific aryl groups or heteroaryl groups (for example, see Patent document 1), imidazophenanthrene derivatives having benzoacridine skeletons (for example, see Patent document 2), and imidazole derivatives substituted with specific aryl groups and heteroaryl groups (for example, see Patent documents 3 and 4).

### PRIOR ART DOCUMENTS

### PATENT DOCUMENTS

Patent document 1: Chinese Patent Publication No. 101024765
Patent document 2: International Publication WO 2015/083948
Patent document 3: Korean Patent Application Publication No. 2009/0079134
Patent document 4: Korean Patent Application Publication No. 2020/0076818

### SUMMARY OF INVENTION

### PROBLEMS TO BE SOLVED BY THE INVENTION

The techniques described in Patent documents 1 to 4 serve to produce organic EL elements having high luminance efficiency, low voltage drive capability, and high durability. However, demand for organic EL devices with high luminance efficiency and durability is escalating in recent years, and technologies that simultaneously achieve even higher luminance efficiency and longer service life are being demanded.

In view of such issues in the conventional technologies, the main object of the present invention is to provide an organic EL element that exhibits both high luminance efficiency and long service life.

### MEANS OF SOLVING THE PROBLEMS

To solve the above problems, the present invention adopts the configuration described below.
[1] A compound as represented by the general formula (1) given below.

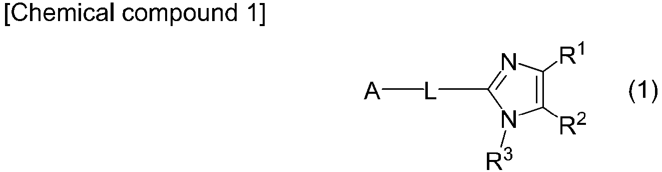

In the general formula (1), R¹ and R² are each independently one selected from the group consisting of substituted or unsubstituted aryl groups and substituted or unsubstituted heteroaryl groups. R¹ and R² may be linked to each other to form a ring structure. R³ is one selected from the group consisting of substituted or unsubstituted alkyl groups, substituted or unsubstituted aryl groups, and substituted or unsubstituted heteroaryl groups. L denotes a substituted or unsubstituted arylene group or a substituted or unsubstituted heteroarylene group. A denotes a structure as represented by the general formula (2) or (3) given below.

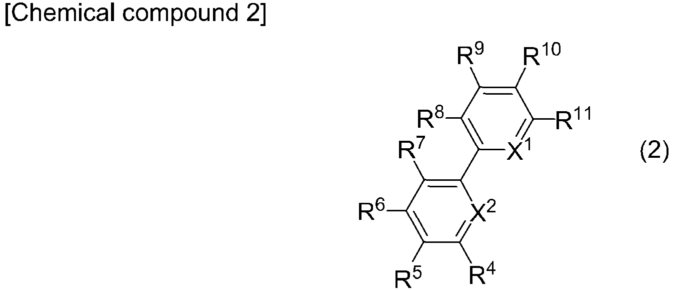

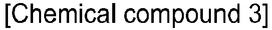

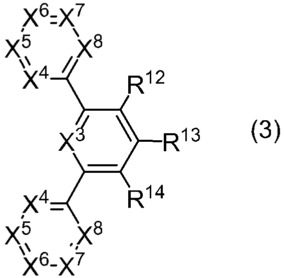

In the general formulae (2) and (3), R⁴ to R¹⁴ are each independently one selected from the group consisting of a hydrogen atom, substituted or unsubstituted alkyl groups, substituted or unsubstituted alkoxy groups, substituted or unsubstituted aryl groups, and substituted or unsubstituted heteroaryl groups. Here, one of R⁴ to R¹¹ is directly bonded to L, and one of R¹² to R¹⁴ is directly bonded to L. R⁷ and R⁸ may be linked to each other to form a ring structure. X¹ to X⁸ are each independently a carbon atom or a nitrogen atom. Here, at least either of X¹ and X² is a nitrogen atom, and at least one of X³ to X⁸ is a nitrogen atom.
[2] A compound as set forth in paragraph [1], wherein the total number of carbon atoms present in R¹ and R² in the general formula (1) is 8 to 20.
[3] A compound as set forth in either paragraph [1] or [2], wherein the number of carbon atoms present in R³ in the general formula (1) is 1 to 12.
[4] A compound as set forth in any one of paragraphs [1] to [3], wherein L in the general formula (1) is a phenylene group, a naphthylene group, or a biphenylene group.
[5] A compound as set forth in any one of paragraphs [1] to [4], wherein A in the general formula (1) is represented by any one of the general formulae (4) to (6) given below.

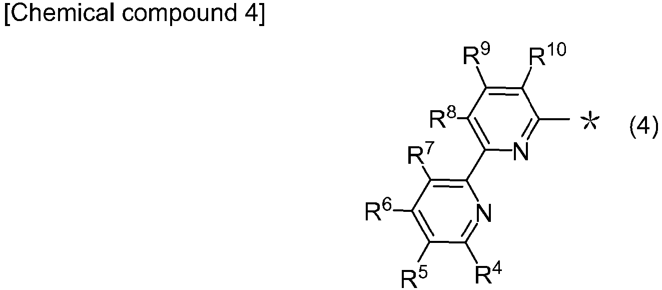

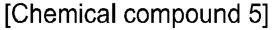

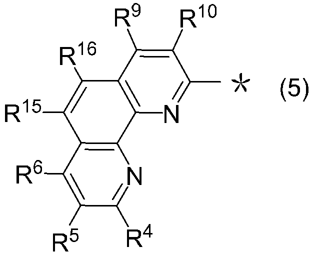

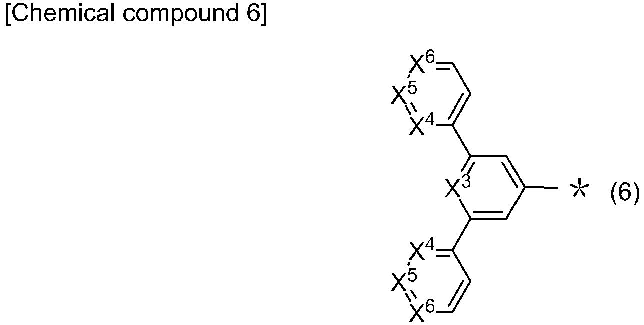

In the general formulas (4) to (6), R⁴ to R¹⁰, R¹⁵, and R¹⁶ are each independently one selected from the group consisting of a hydrogen atom, substituted or unsubstituted alkyl groups, substituted or unsubstituted alkoxy groups, substituted or unsubstituted aryl groups, and substituted or unsubstituted heteroaryl groups. X³ to X⁶ are each independently a carbon atom or a nitrogen atom. Here, at least one of X³ to X⁶ is a nitrogen atom. The symbol * represents a site for bonding to L.
[6] A compound as set forth in paragraph [5], wherein R⁵ to R¹⁰, R¹⁵, and R¹⁶ in the general formulae (4) and (5) are hydrogen atoms.
[7] A compound as set forth in either paragraph [5] or [6], wherein, in the general formula (6), X³ is a nitrogen atom and any one of X⁴ to X⁶ is a nitrogen atom.
[8] A light emitting element comprising at least an electron transporting layer and a light emitting layer located between an anode and a cathode to emit light by using electrical energy, wherein the electron transporting layer contains a compound as set forth in any one of paragraphs [1] to [7].
[9] A light emitting element as set forth in paragraph [8], wherein the electron transporting layer further contains an alkali metal atom or a rare earth metal atom.
[10] A light emitting element comprising at least a charge generation layer and a light emitting layer located between an anode and a cathode to emit light by using electrical energy, wherein the charge generation layer contains a compound as set forth in any one of paragraphs [1] to [7].
[11] A light emitting element as set forth in paragraph [10], wherein the charge generation layer further contains a phenanthroline derivative.
[12] A light emitting element as set forth in either paragraph [10] or [11], wherein the charge generation layer further contains an alkali metal atom, a copper group element atom, or a rare earth metal atom.
[13] A light emitting element as set forth in any one of paragraphs [10] to [12], wherein the charge generation layer further contains an alkali metal atom, with the alkali metal atom being Li.
[14] A light emitting element as set forth in paragraph [11], wherein the charge generation layer further contains a rare earth metal atom, with the rare earth metal atom being Yb.
[15] A light emitting element comprising at least an electron injection layer and a light emitting layer located between an anode and a cathode to emit light by using electrical energy, wherein the electron injection layer contains a compound as set forth in any one of paragraphs [1] to [7].
[16] A light emitting element as set forth in paragraph [15], wherein the electron injection layer further contains an alkali metal atom or a rare earth metal atom.
[17] A display device comprising a light emitting element as set forth in any one of paragraphs [8] to [16].
[18] An illumination device comprising a light emitting element as set forth in any one of paragraphs [8] to [16].
[19] A photosensitizer comprising a light emitting element as set forth in any one of paragraphs [8] to [16].

### ADVANTAGEOUS EFFECTS OF THE INVENTION

The present invention can provide an organic EL element that has high luminance efficiency and long service life.

### DESCRIPTION OF PREFERRED EMBODIMENTS

Preferred embodiments of the compound, light emitting element, display device, illumination device, and photosensitizer according to the present invention will be described in detail below. It should be noted, however, that the present invention is not limited to the embodiment described below and may be modified appropriately to suit particular objectives and purposes.

### (Compound as represented by the general formula (1))

The compound according to the embodiment of the present invention is a compound as represented by the general formula (1).

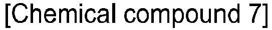

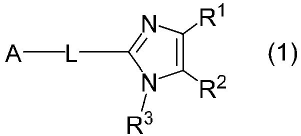

In the general formula (1), R¹ and R² are each independently one selected from the group consisting of substituted or unsubstituted aryl groups and substituted or unsubstituted heteroaryl groups. R¹ and R² may be linked to each other to form a ring structure. R³ is one selected from the group consisting of substituted or unsubstituted alkyl groups, substituted or unsubstituted aryl groups, and substituted or unsubstituted heteroaryl groups. L denotes a substituted or unsubstituted arylene group or a substituted or unsubstituted heteroarylene group. A denotes a structure as represented by the general formula (2) or (3) given below.

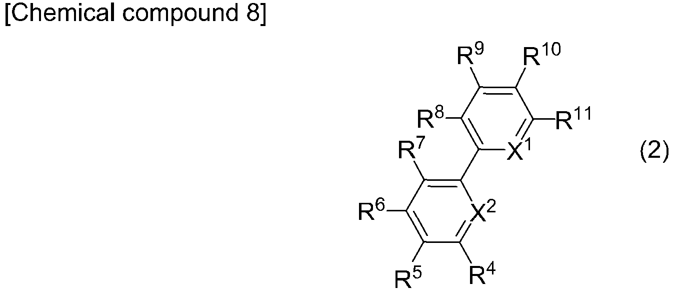

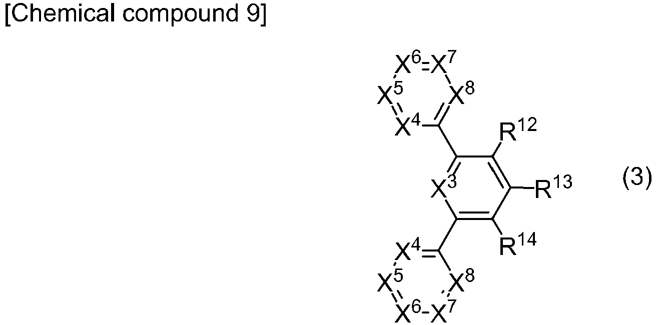

In the general formulae (2) and (3), R⁴ to R¹⁴ are each independently one selected from the group consisting of a hydrogen atom, substituted or unsubstituted alkyl groups, substituted or unsubstituted alkoxy groups, substituted or unsubstituted aryl groups, and substituted or unsubstituted heteroaryl groups. Here, one of R⁴ to R¹¹ is directly bonded to L, and one of R¹² to R¹⁴ is directly bonded to L. R⁷ and R⁸ may be linked to each other to form a ring structure. X¹ to X⁸ are each independently a carbon atom or a nitrogen atom. Here, at least either of X¹ and X² is a nitrogen atom, and at least one of X³ to X⁸ is a nitrogen atom.

In all the groups mentioned above, each hydrogen atom may be a deuterium atom. The same applies to the substituents, compounds, and partial structures thereof described below.

When the expression "substituted or unsubstituted" is used, "unsubstituted" means that a hydrogen atom or a deuterium atom is bonded. The same interpretation also applies where the expression "substituted or unsubstituted" is used for compounds or partial structures thereof that are described below.

An aryl group refers to an aromatic hydrocarbon group such as, for example, phenyl group, biphenyl group, terphenyl group, naphthyl group, fluorenyl group, benzofluorenyl group, dibenzofluorenyl group, phenanthryl group, anthracenyl group, benzophenanthryl group, benzoanthracenyl group, chrysenyl group, pyrenyl group, fluoranthenyl group, triphenylenyl group, benzofluoranthenyl group, dibenzoanthracenyl group, perylenyl group, and helicenyl group. There are no specific limitations on the number of carbon atoms present in a ring, but it is preferably 6 or more and 40 or less, and more preferably 6 or more and 30 or less. In particular, the use of a phenyl group is preferable.

An arylene group refers to an aromatic hydrocarbon group such as, for example, phenylene group, biphenylene group, terphenylene group, naphthylene group, fluorenylene group, benzofluorenylene group, dibenzofluorenylene group, phenanthrylene group, anthracenylene group, benzophenanthrylene group, benzoanthracenylene group, chrysenylene group, pyrenylene group, fluoranthenylene group, triphenylenylene group, benzofluoranthenylene group, dibenzoanthracenylene group, perylenylene group, or helicenylene group. Here, the two bonds of the arylene group are attached to the same conjugated system. There are no specific limitations on the number of carbon atoms present in a ring, but it is preferably 6 or more and 40 or less, and more preferably 6 or more and 30 or less. In particular, the use of a phenylene group is preferable.
A heteroaryl group refers to a cyclic aromatic group containing one or more non-carbon atoms in the ring such as, for example, pyridyl group, furanyl group, thiophenyl group, quinolinyl group, isoquinolinyl group, pyrazinyl group, pyrimidyl group, pyridazinyl group, triazinyl group, naphthyridinyl group, cinnolinyl group, phthalazinyl group, quinoxalinyl group, quinazolinyl group, benzofuranyl group, benzothiophenyl group, indolyl group, dibenzofuranyl group, dibenzothiophenyl group, carbazolyl group, benzocarbazolyl group, carborynyl group, indolocarbazolyl group, benzofluorocarbazolyl group, benzothienocarbazolyl group, dihydroindenocarbazolyl group, benzoquinolinyl group, acridinyl group, dibenzoacridinyl group, benzoimidazolyl group, imidazopyridyl group, benzoxazolyl group, benzothiazolyl group, and phenanthrolinyl group. Here, a naphthyridinyl group refers to any one of 1,5-naphthyridinyl group, 1,6-naphthyridinyl group, 1,7-naphthyridinyl group, 1,8-naphthyridinyl group, 2,6-naphthyridinyl group, or 2,7-naphthyridinyl group. There are no specific limitations on the number of carbon atoms present in a ring, but it is preferably 5 or more and 40 or less, and more preferably 5 or more and 30 or less.

A heteroarylene group refers to a cyclic aromatic group containing one or more non-carbon atoms in the ring such as, for example, pyridylene group, furanylene group, thiophenylene group, quinolynylene group, isoquinolynylene group, pyrazinylene group, pyrimidilene group, pyridazinylene group, triazinylene group, naphthyridinylene group, cinnolynylene group, phthalazinylene group, quinoxalinylene group, quinazolinylene group, benzofuranylene group, benzothiophenylene group, indolylene group, dibenzofuranylene group, dibenzothiophenylene group, carbazolylene group, benzocarbazolylene group, carborynylene group, indolocarbazolylene group, benzofluorocarbazolylene group, benzothienocarbazolylene group, dihydroindenocarbazolylene group, benzoquinolynylene group, acridinylene group, dibenzoacridinylene group, benzoimidazolylene group, imidazopyridylene group, benzoxazolylene group, benzothiazolylene group, and phenanthrolinylene group. Here, a naphthyridinylene group refers to any one of 1,5-naphthyridinylene group, 1,6-naphthyridinylene group, 1,7-naphthyridinylene group, 1,8-naphthyridinylene group, 2,6-naphthyridinylene group, and 2,7-naphthyridinylene group. Here, the two bonds of the heteroarylene group are attached to the same conjugated system. There are no specific limitations on the number of carbon atoms present in a ring, but it is preferably 5 or more and 40 or less, and more preferably 5 or more and 30 or less. In particular, the use of a phenylene group or a biphenylene group is preferable.
An alkyl group is a saturated aliphatic hydrocarbon group such as, for example, methyl group, ethyl group, n-propyl group, isopropyl group, n-butyl group, sec-butyl group, and tert-butyl group. There are no specific limitations on the number of carbon atoms present in an alkyl group, but from the perspective of availability and cost, it is preferably 1 or more and 20 or less, and more preferably 1 or more and 8 or less. The number of carbon atoms mentioned herein includes the number of carbon atoms contained in the substituents attached to the alkyl group, and this also applies to other substituents for which the number of carbon atoms is specified.

An alkoxy group refers to a group that contains an alkyl group bonded to an oxygen atom such as, for example, methoxy group, ethoxy group, n-propoxy group, isopropoxy group, n-butoxy group, sec-butoxy group, and tert-butoxy group. There are no specific limitations on the number of carbon atoms present in an alkoxy group, but from the perspective of availability and cost, it is preferably 1 or more and 20 or less, and more preferably 1 or more and 8 or less.

As conventionally used compounds that include nitrogen-containing aromatic heterocycles and polycyclic aromatic hydrocarbons, Patent documents 1 to 4 disclose, for example, the compounds W, X, Y, and Z that are represented by the formulae given below.

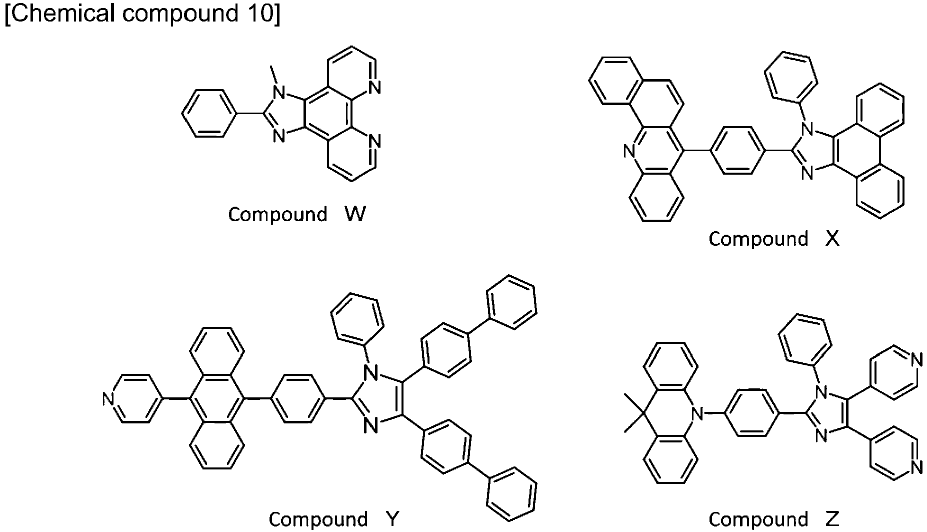

However, it has been found that even when these compounds are used to form electron injection layers, electron transporting layers, or charge generation layers, they cannot serve to provide organic EL elements that have sufficient characteristics to exhibit performance required in recent years. Accordingly, there is a stronger demand for compounds that can serve to achieve improved performance in terms of luminance efficiency and service life.

For example, compounds having imidazophenanthroline skeletons, such as the compound W, have molecular weights of 310 and have problems in terms of thermal stability and service life.

Compounds having benzoacridine skeletons, such as the compound X, tend to have excessively high degrees of crystallinity due to their high flatness attributed to the benzoacridine skeletons, and this leads to problems such as low film quality stability, high driving voltage, low luminance efficiency, and short service life.

Compounds having anthracene skeletons and para-biphenyl skeletons, such as the compound Y, tend to not only have excessively high degrees of crystallinity due to their high flatness attributed to the anthracene skeletons but also have molecular weights of 700 or more, which leads to problems such as poor handling properties and decreased film quality stability.

Compounds having arylamine skeletons, such as the compound Z, have electron donating substituents within the molecules, which work to reduce the electron transport ability of the compounds and lead to a problem of increased driving voltage.

The present inventors, in the course of studying improvements, focused on the effect of the imidazole skeleton. The imidazole skeleton exhibits high electron transport ability and acts as a substituent that tend to coordinate strongly to metal atoms. A compound as represented by the general formula (1) has an aryl group or a heteroaryl group as R¹ or R², and accordingly undergoes conjugation more easily to exhibit enhanced electron transport ability. Furthermore, if R¹ and R² are linked to each other, the ring structure will be immobilized, thereby acting to further improve the durability of the compound.

From the perspective of availability and handleability of the compound, it is preferable that the total number of carbon atoms present in R¹ and R² be 8 to 20. In particular, from the perspective of further enhancing the film quality stability and increasing the service life of the light emitting element, it is more preferable that R¹ and R² be each independently one selected from the group consisting of phenyl group and pyridyl group.

The presence of R³ as described above serves to improve the durability of the compound and the stability of the light emitting element. In order to impart an appropriate degree of crystallinity to the compound, it is preferable that the number of carbon atoms present in R³ be 1 to 12. Furthermore, from the perspective of availability of the compound, it is more preferable that R³ be either a methyl group or a phenyl group.

The presence of L as a group as described above serves to allow the groups located on both sides of L to conjugate easily. Accordingly, this allows the compound to have increased electron transport ability. From the perspective of imparting an appropriate degree of crystallinity to the compound, improving the film quality stability, and further improving the service life of the light emitting element, it is preferable that L be a phenylene group, a naphthylene group, or a biphenylene group.

In the case where A has a structure as represented by the general formula (2), the electron transport ability of the compound can be higher when at least either of X¹ and X² is a nitrogen atom. Similarly, in the case where A has a structure as represented by the general formula (3), the electron transport ability of the compound can be higher when at least one of X³ to X⁸ is a nitrogen atom. In particular, if A has a structure as represented by any one of the general formulae (4) to (6), it is more preferable because the resulting layers exhibit higher stability and superior performance.

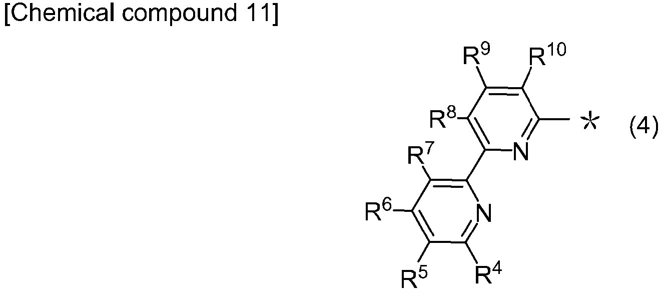

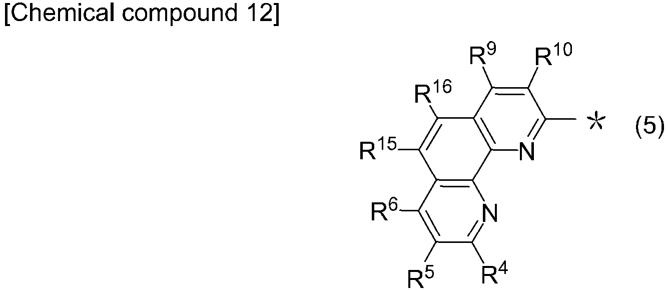

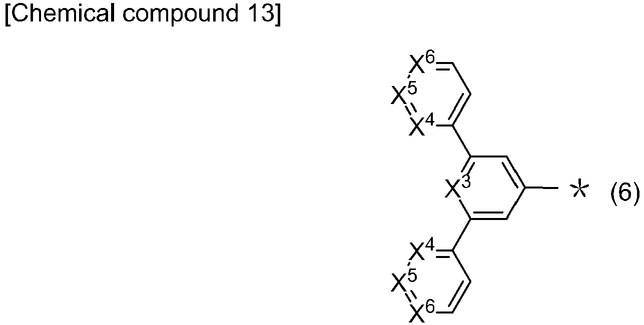

In the case where A has a structure as represented by any one of the general formulae (4) to (6), R⁴ to R¹⁰, R¹⁵, and R¹⁶ are each independently one selected from the group consisting of a hydrogen atom, substituted or unsubstituted alkyl groups, substituted or unsubstituted alkoxy groups, substituted or unsubstituted aryl groups, and substituted or unsubstituted heteroaryl groups. X³ to X⁶ are each independently a carbon atom or a nitrogen atom. Here, at least one of X³ to X⁶ is a nitrogen atom. The symbol * represents a site for bonding to L.

The general formulae (4) and (5) represent a bipyridine skeleton and a phenanthroline skeleton, respectively. Since these skeletons possess both strong coordination tendency to metals and high electron transport ability, they can contribute to lowering the driving voltage and improving the efficiency in a light emitting element. In particular, the presence of a phenanthroline skeleton, in which the ring structure is immobilized, is more preferable because it can serve to further increase the coordination tendency to metal atoms and act to further decrease the driving voltage and increase the luminance efficiency.

It is preferable from the perspective of availability of the compound that R⁵ to R¹⁰, R¹⁵, and R¹⁶ in the general formulae (4) and (5) be each independently one selected from the group consisting of a hydrogen atom, a methyl group, and a phenyl group. Furthermore, from the perspective of improving the film quality stability, it is more preferable that R⁵ to R¹⁰, R¹⁵, and R¹⁶ be hydrogen atoms.

In the general formulae (4) and (5), it is preferable that R⁴ be a substituted or unsubstituted aryl group or a substituted or unsubstituted heteroaryl group. In this case, R⁴ is bonded at the highly reactive 2-position in the bipyridine skeleton or the phenanthroline skeleton, and this works to increase the stability of the compound and the element. It is more preferable that R⁴ be a phenyl group, a pyridyl group, a fluorophenyl group, or a tolyl group. These substituents impart an appropriate degree of crystallinity to the compound, thereby enabling the formation of stable layers. As a result, this serves to further decrease the driving voltage and increase the service life. From the perspective of providing an element with higher stability and longer service life, it is preferable that R⁴ be a phenyl group.

The structure represented by the general formula (6) contains at least one six-membered ring skeleton having a nitrogen atom. Therefore, if it is adopted as A, it exhibits high coordination tendency to metals and high electron transport ability, thereby serving to decrease the driving voltage and improve the efficiency of a light emitting element. In particular, in the case where X³ is a nitrogen atom while at least one of X⁴ to X⁶ is a nitrogen atom, the structure represented by the general formula (6) is a terpyridine skeleton, which works to ensure even higher coordination tendency to metal atoms. This is more preferable because it serves to further decrease the driving voltage and increase the luminance efficiency.

From the perspective of suppressing crystallization and improving film quality stability, it is preferable that the compound represented by the general formula (1) have a molecular weight of 380 or more. On the other hand, from the perspective of improving processability during sublimation purification or vapor evaporation, it is preferable that the molecular weight of the compound represented by the general formula (1) be 660 or less.

In the case where a substituent is present on any of the above groups, preferable examples thereof include alkyl groups, cycloalkyl groups, heteroalicyclic groups, alkenyl groups, cycloalkenyl groups, alkynyl groups, aryl groups, heteroaryl groups, hydroxyl groups, thiol groups, alkoxy groups, alkylthio groups, aryl ether groups, arylthioether groups, halogens, cyano groups, aldehyde groups, acyl groups, carboxyl groups, ester groups, amide groups, acyl groups, sulfonyl groups, sulfonic acid ester groups, sulfonamide groups, amino groups, nitro groups, silyl groups, siloxanyl groups, boryl groups, and oxo groups. These groups may be further substituted with groups given above.

A cycloalkyl group is a saturated alicyclic hydrocarbon group such as, for example, cyclopropyl group, cyclohexyl group, norbornyl group, and adamantyl group, which may or may not contain a substituent group. There are no specific limitations on the number of carbon atoms present in a ring, but it is preferably in the range of 3 or more and 20 or less.

A heteroalicyclic group is an aliphatic ring having a non-carbon atom in the ring such as, for example, pyran ring, piperidine ring, and cyclic amides, which may or may not contain a substituent group. There are no specific limitations on the number of atoms present in a ring, but it is preferably in the range of 3 or more and 20 or less.

An alkenyl group is an unsaturated aliphatic hydrocarbon group containing a double bond such as, for example, vinyl group, allyl group, and butadienyl group, which may or may not contain a substituent group. There are no specific limitations on the number of carbon atoms present in an alkenyl group, but it is preferably in the range of 2 or more and 20 or less.

A cycloalkenyl group is an unsaturated alicyclic hydrocarbon group containing a double bond such as, for example, cyclopentenyl group, cyclopentadienyl group, and cyclohexenyl group, which may or may not contain a substituent group.

An alkynyl group is an unsaturated aliphatic hydrocarbon group containing a triple bond such as, for example, ethynyl group, which may or may not contain a substituent group. There are no specific limitations on the number of carbon atoms present in an alkynyl group, but it is preferably in the range of 2 or more and 20 or less.

Examples of compounds as represented by the general formula (1) include, for example, the compounds shown below. It should be noted that these are only examples and compounds other than those listed here may also be used favorably if they have structures as represented by the general formula (1).

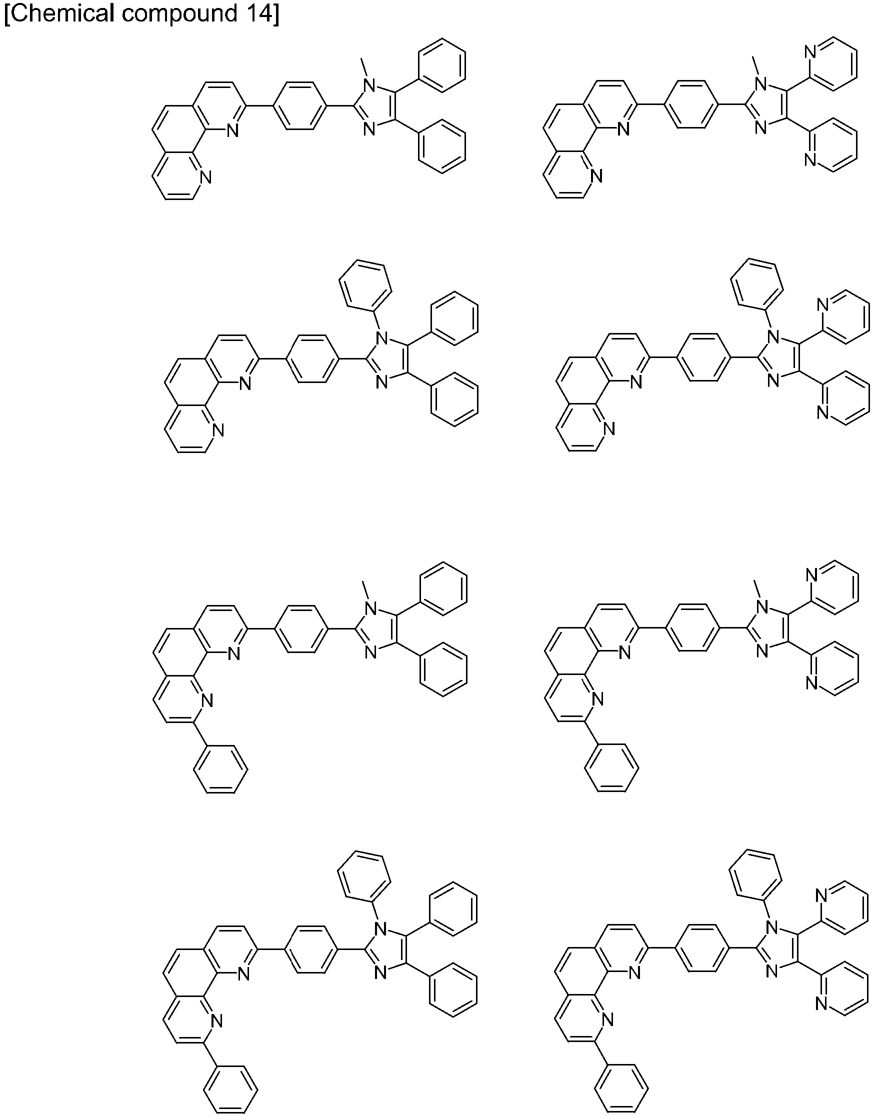

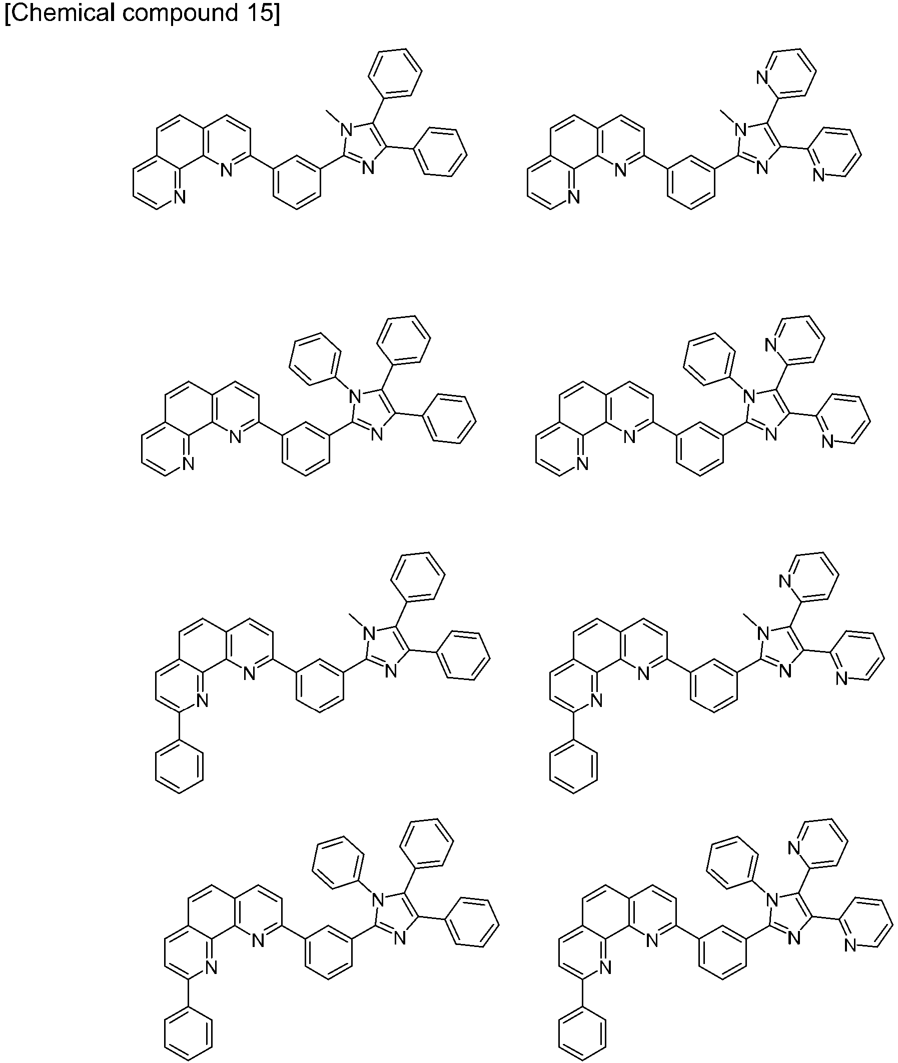

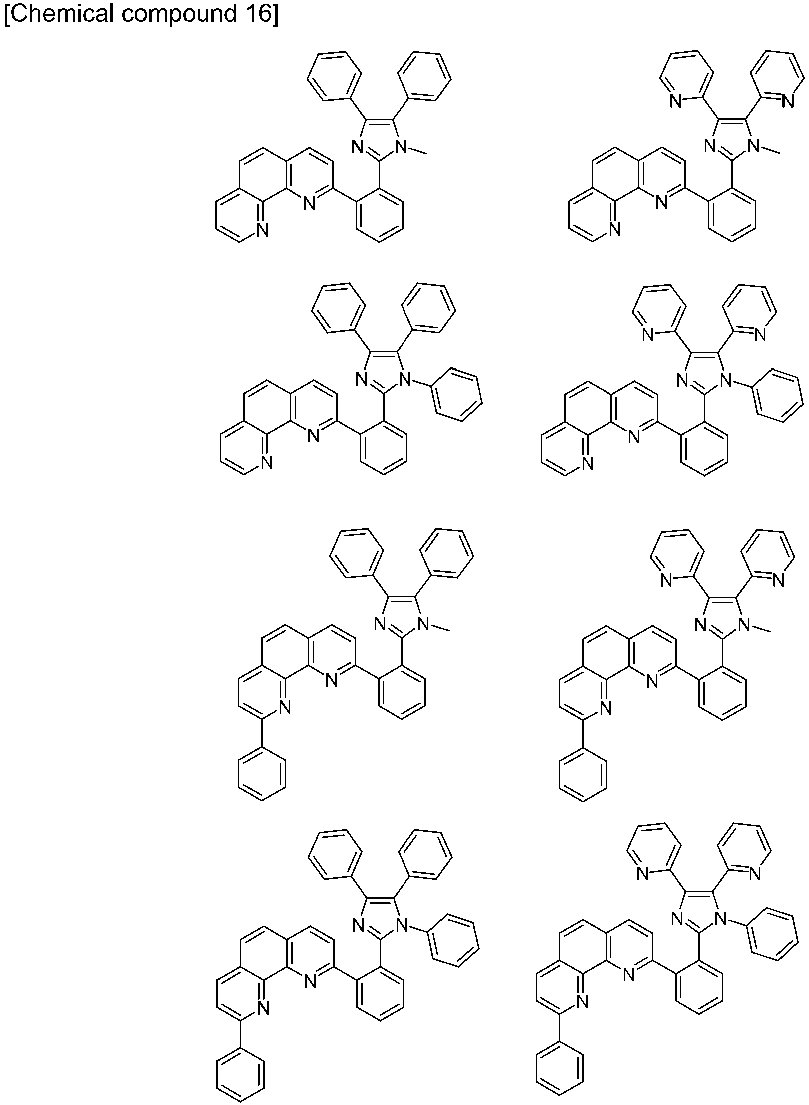

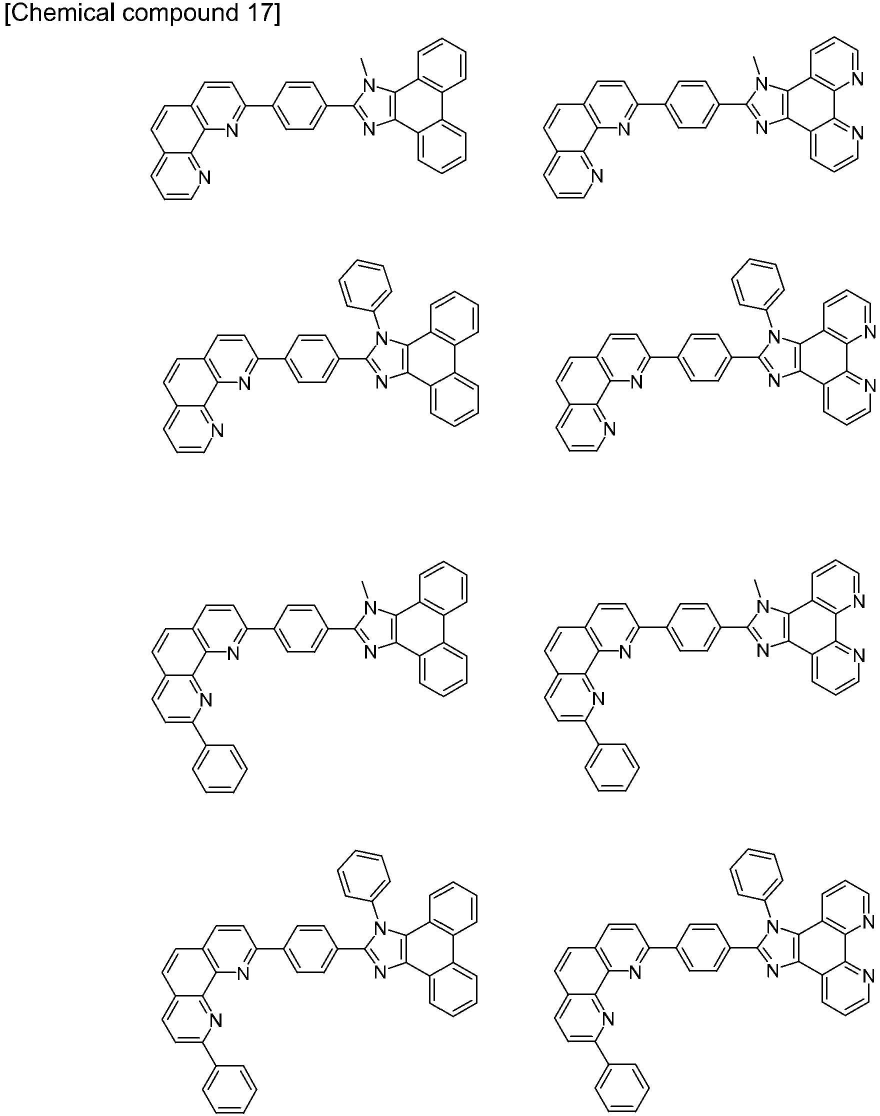

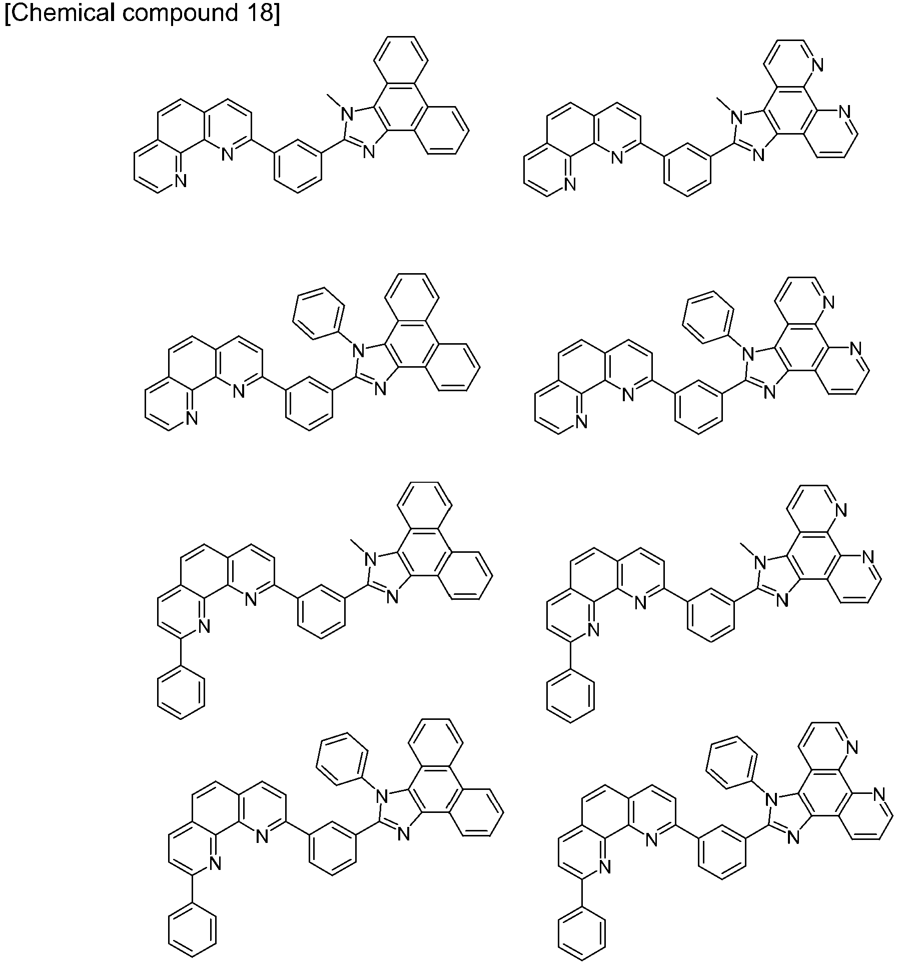

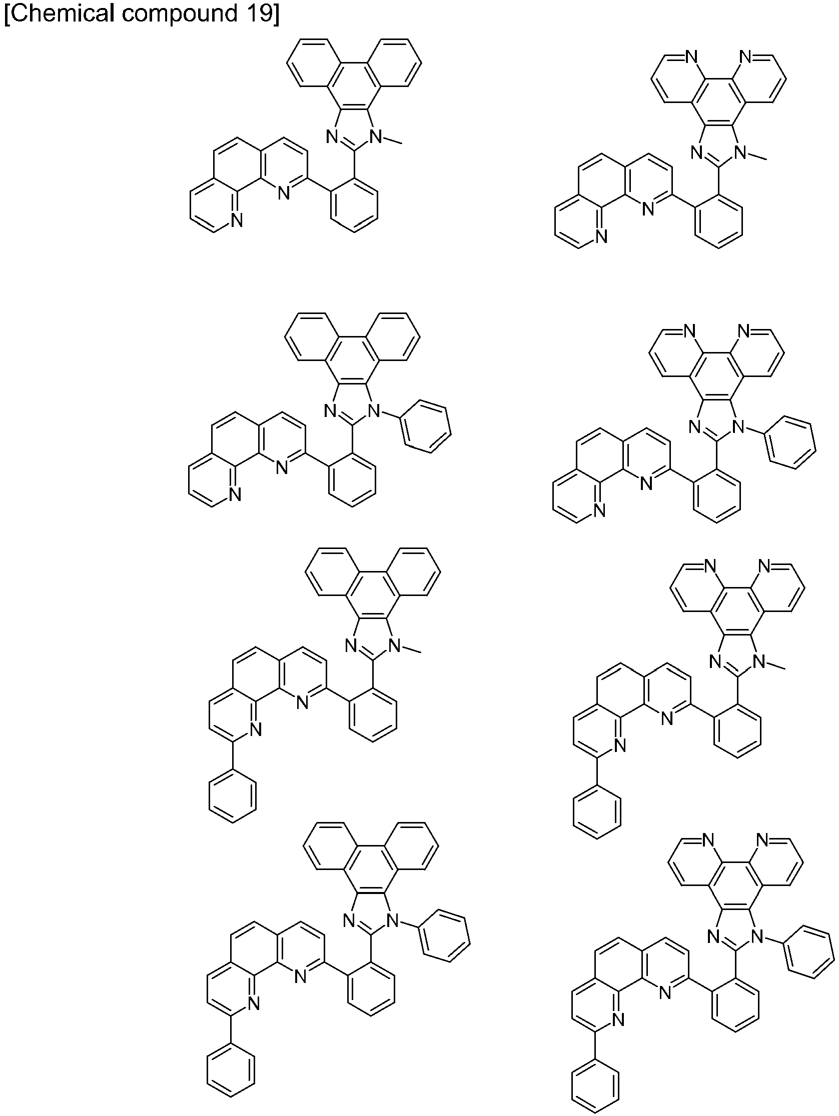

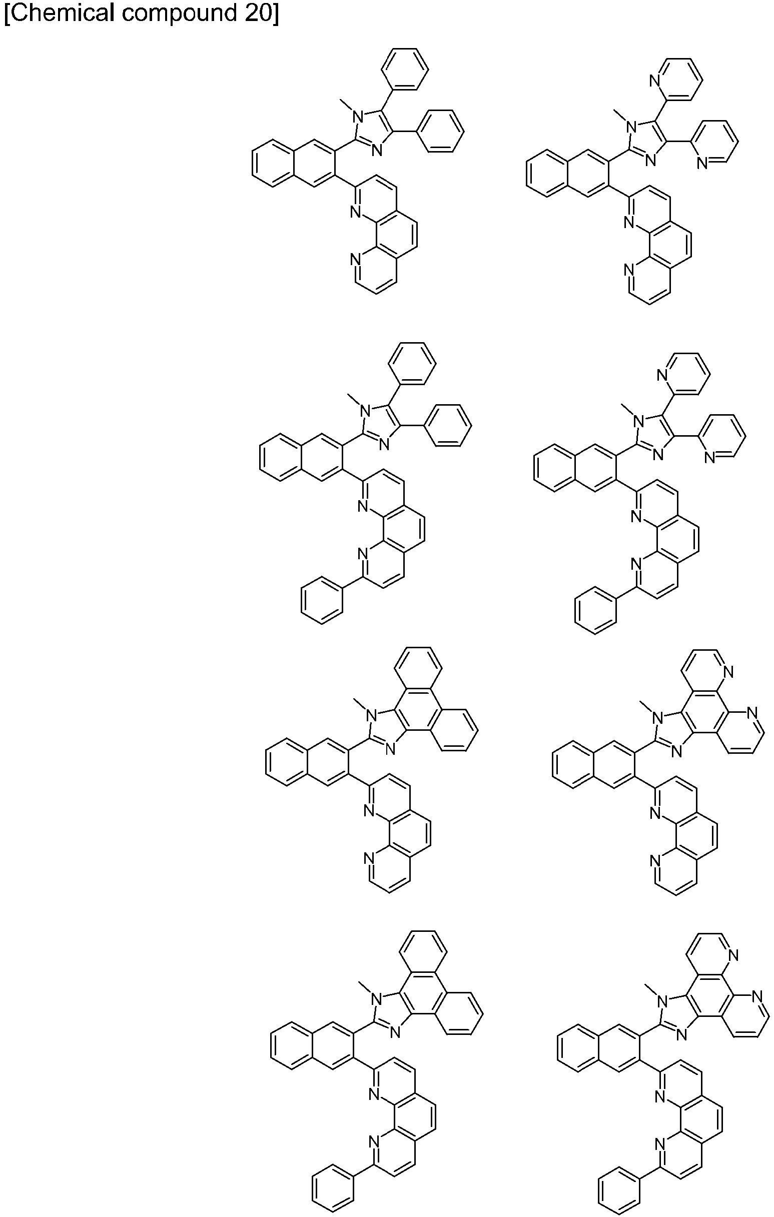

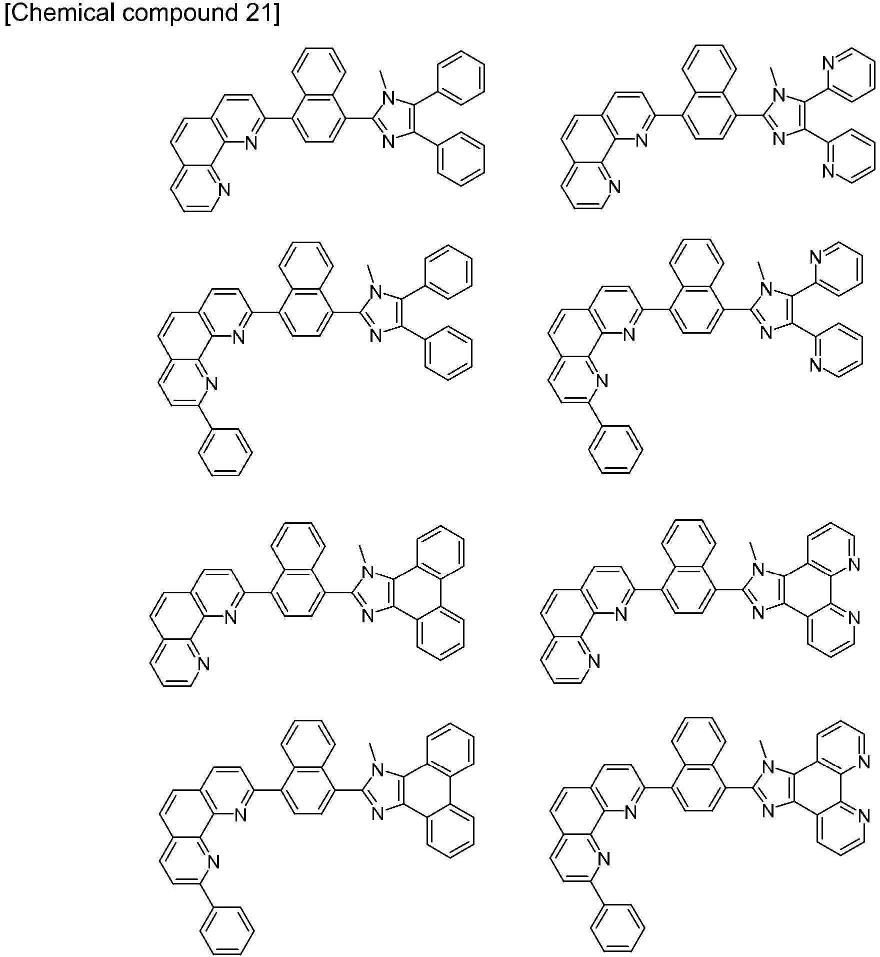

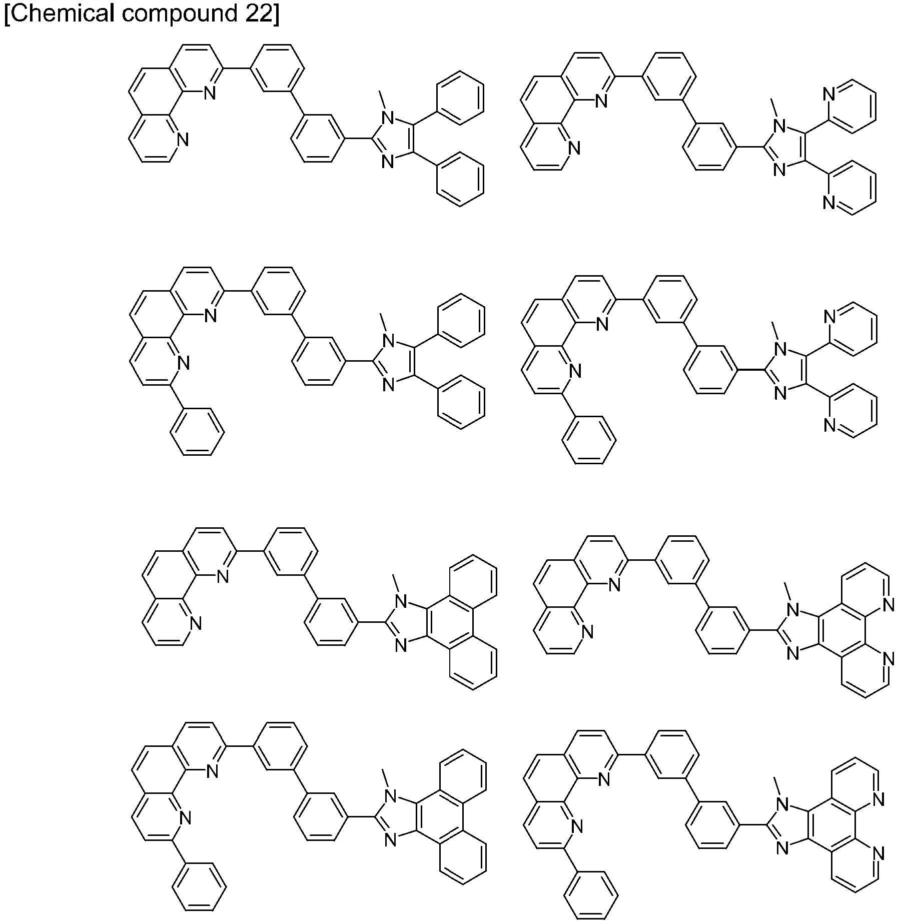

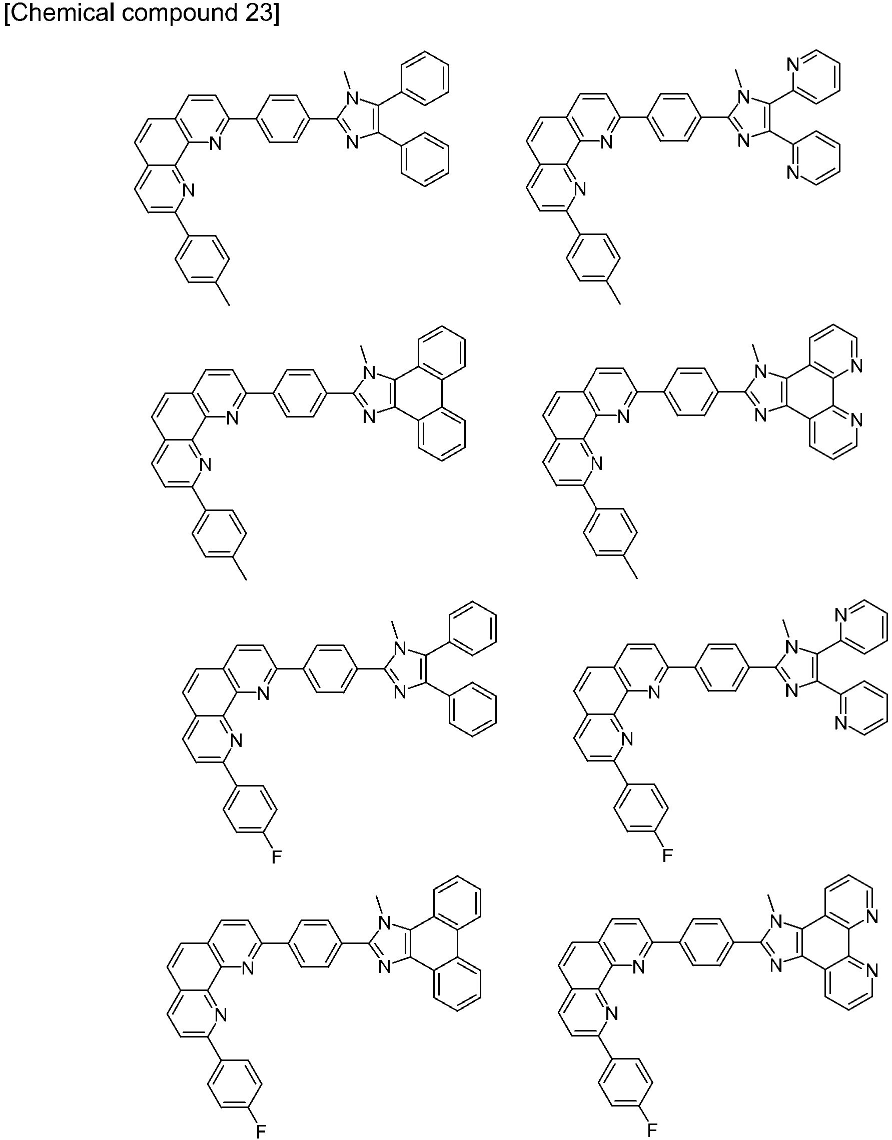

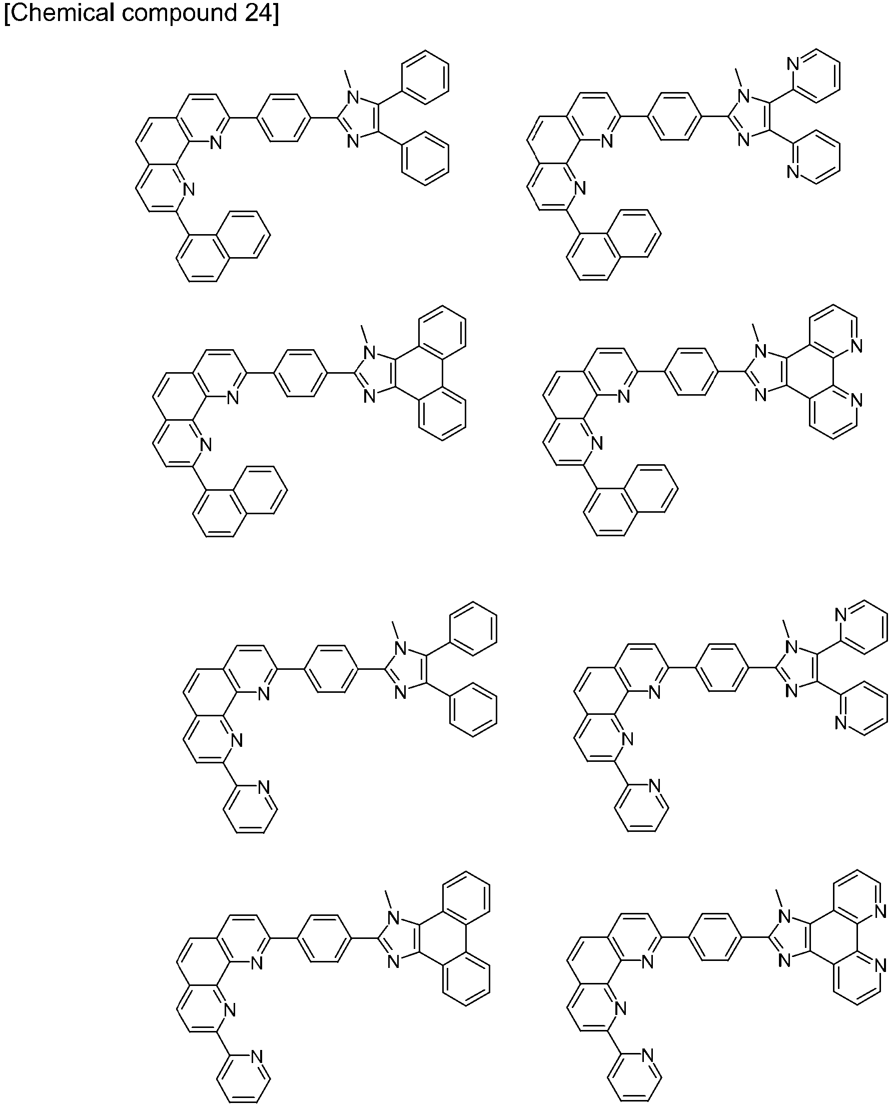

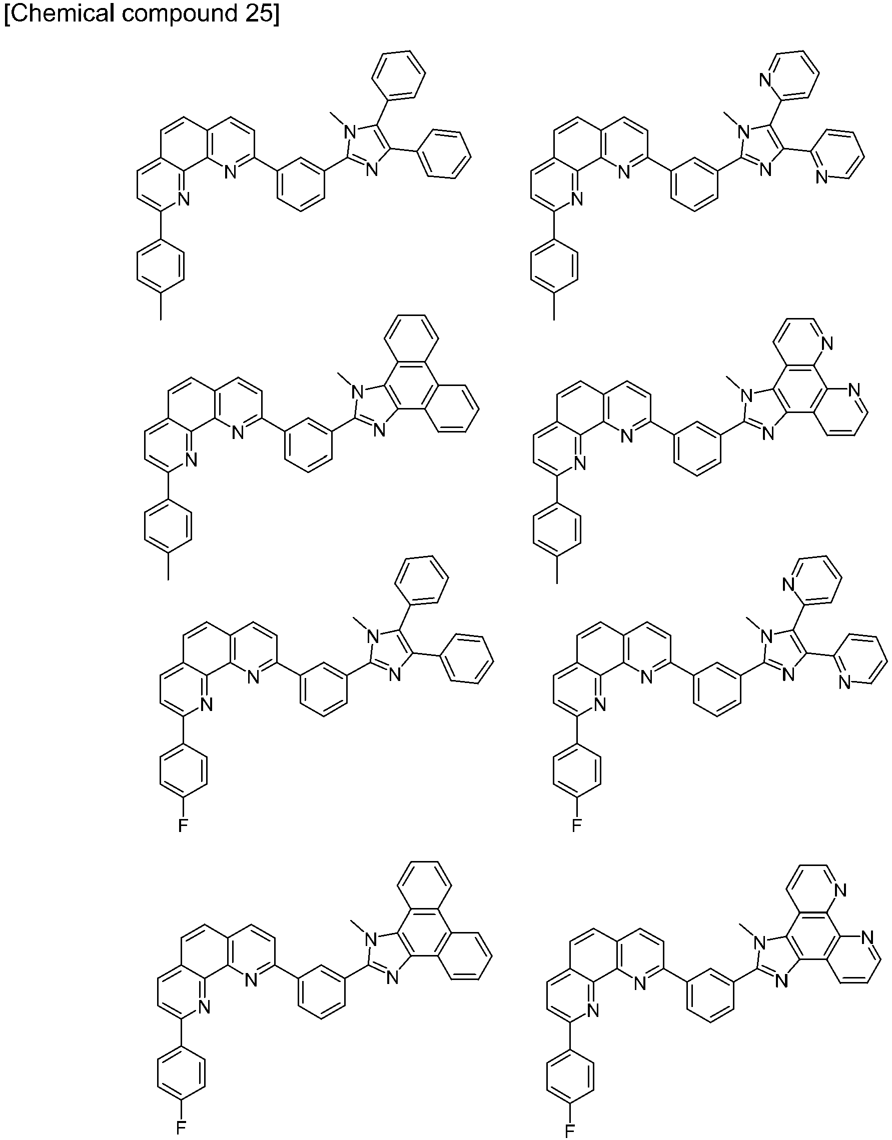

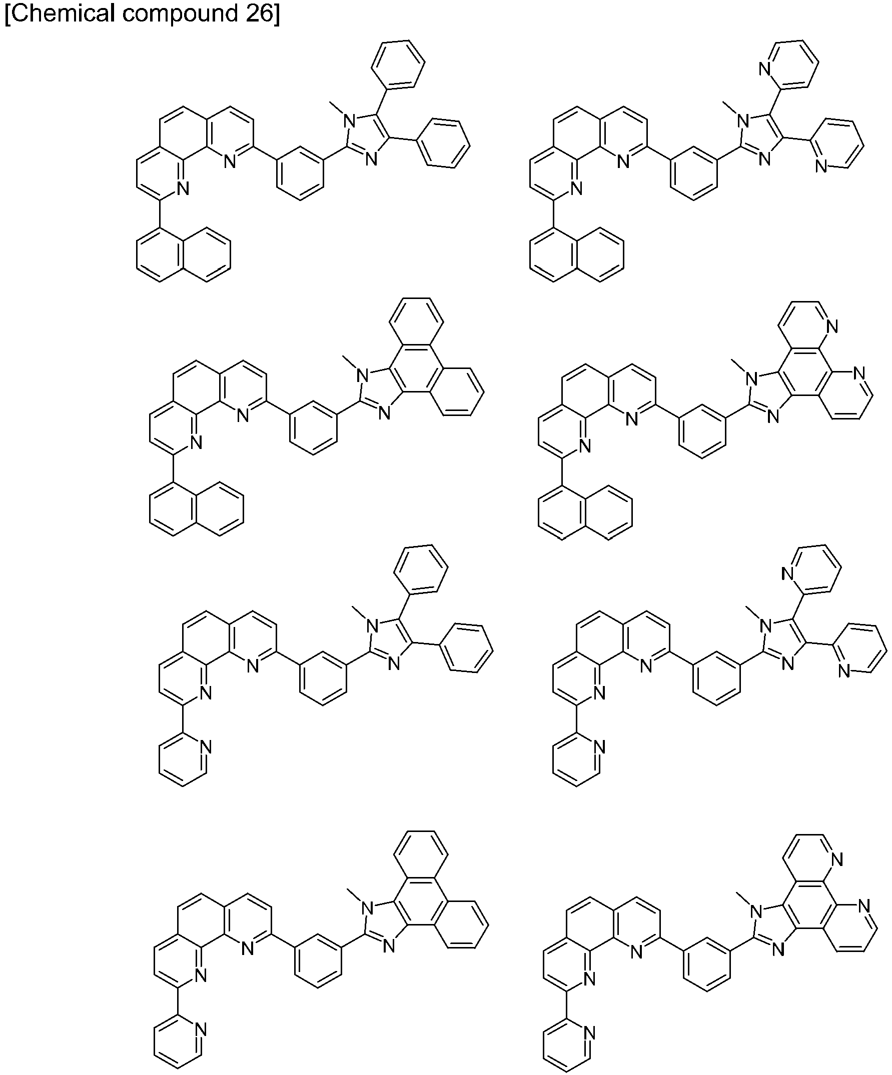

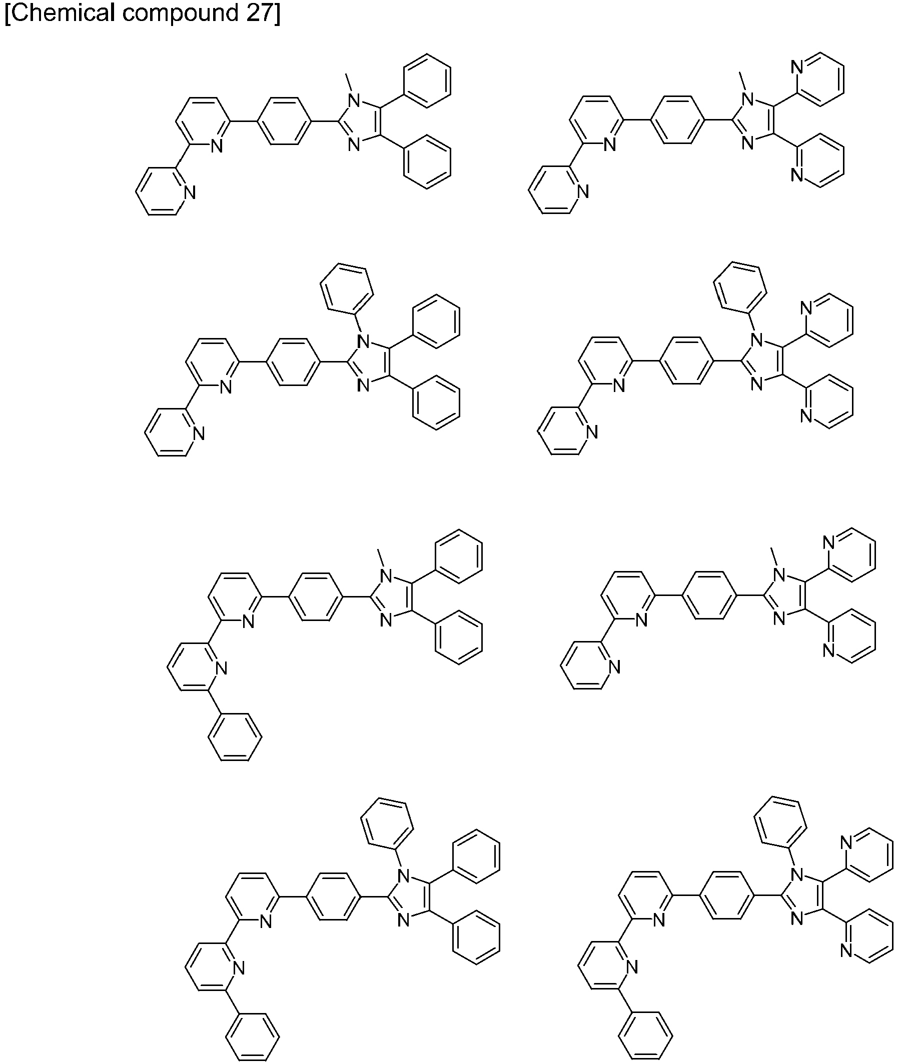

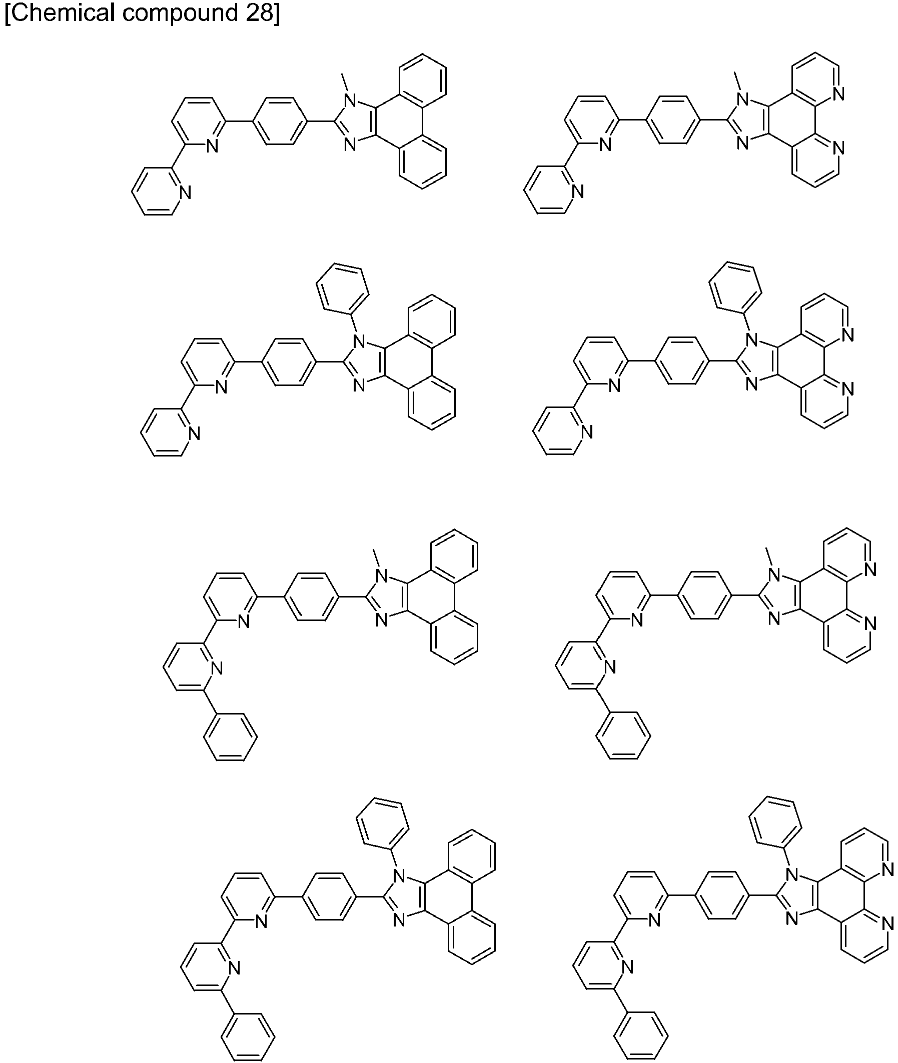

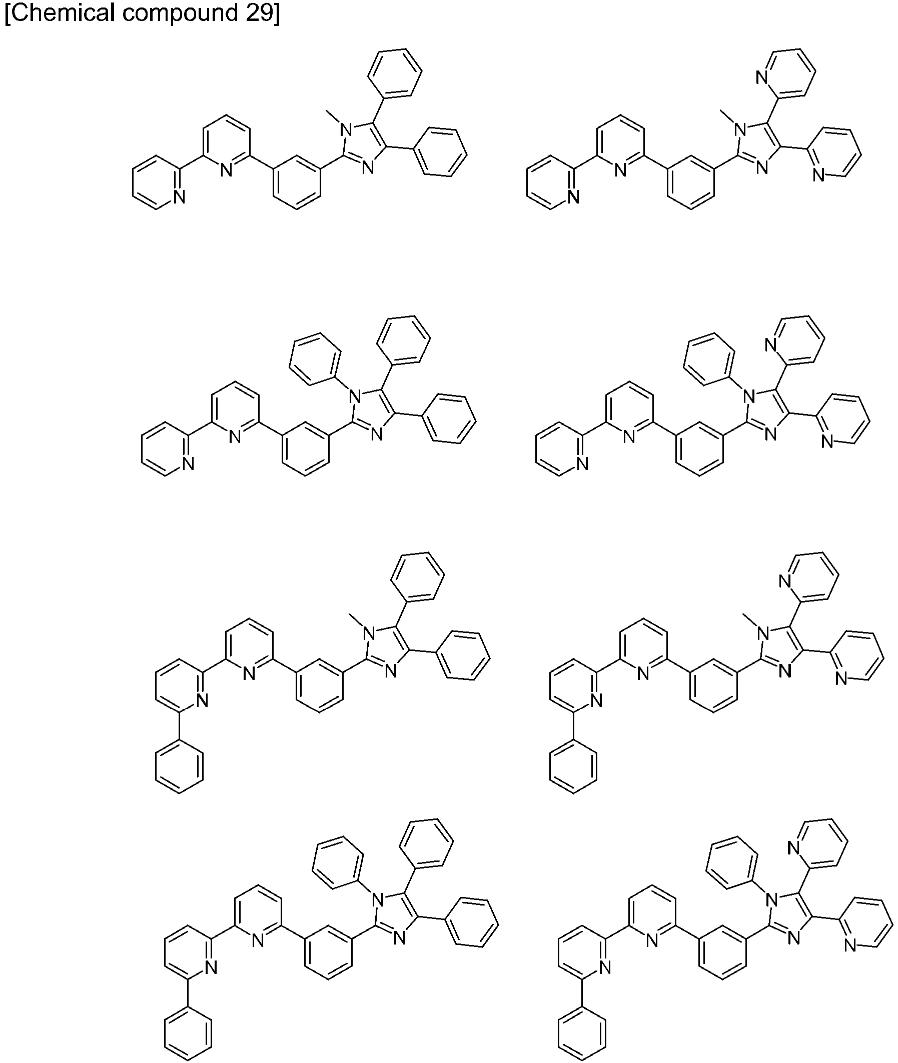

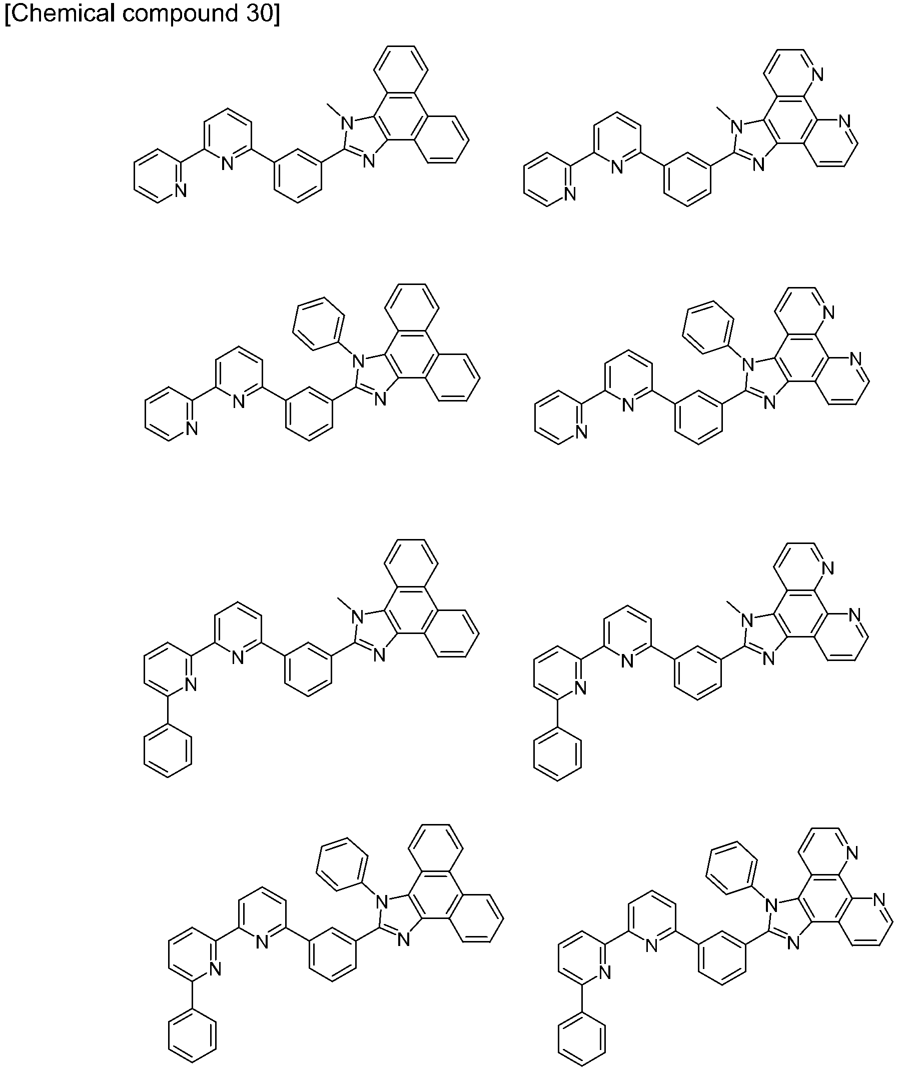

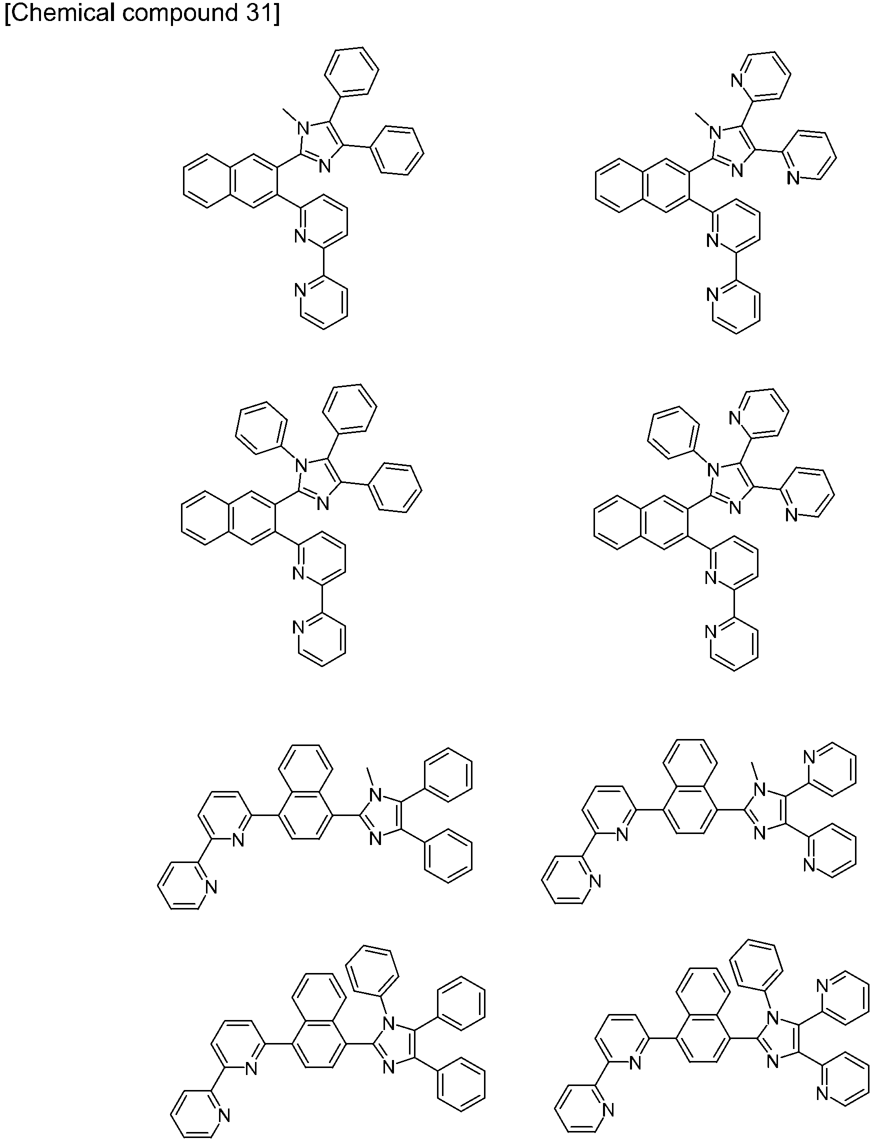

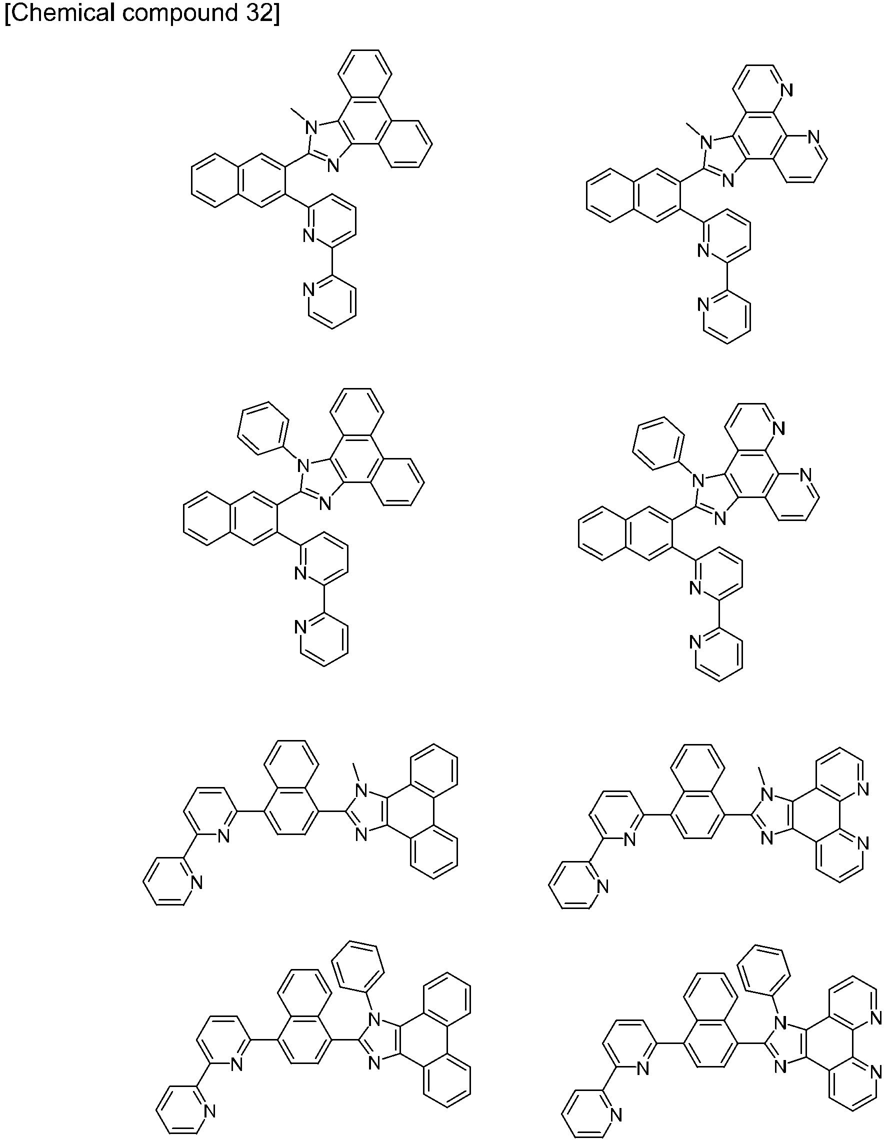

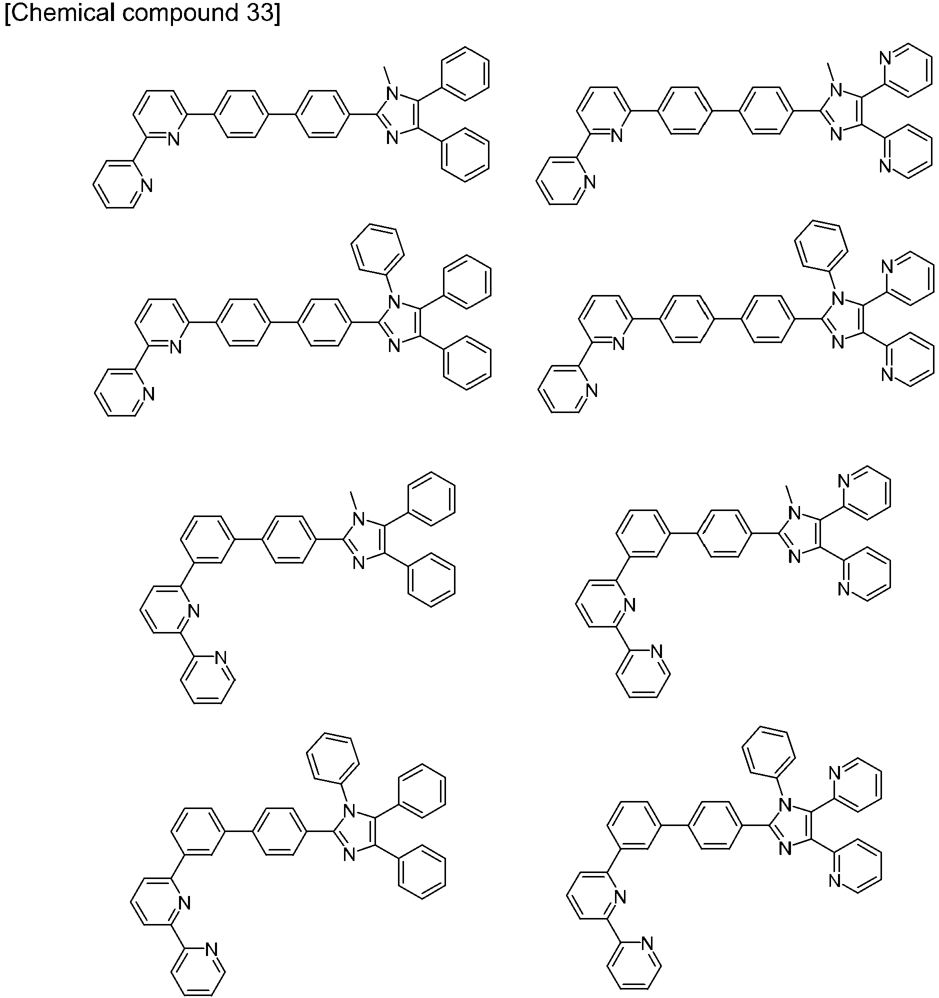

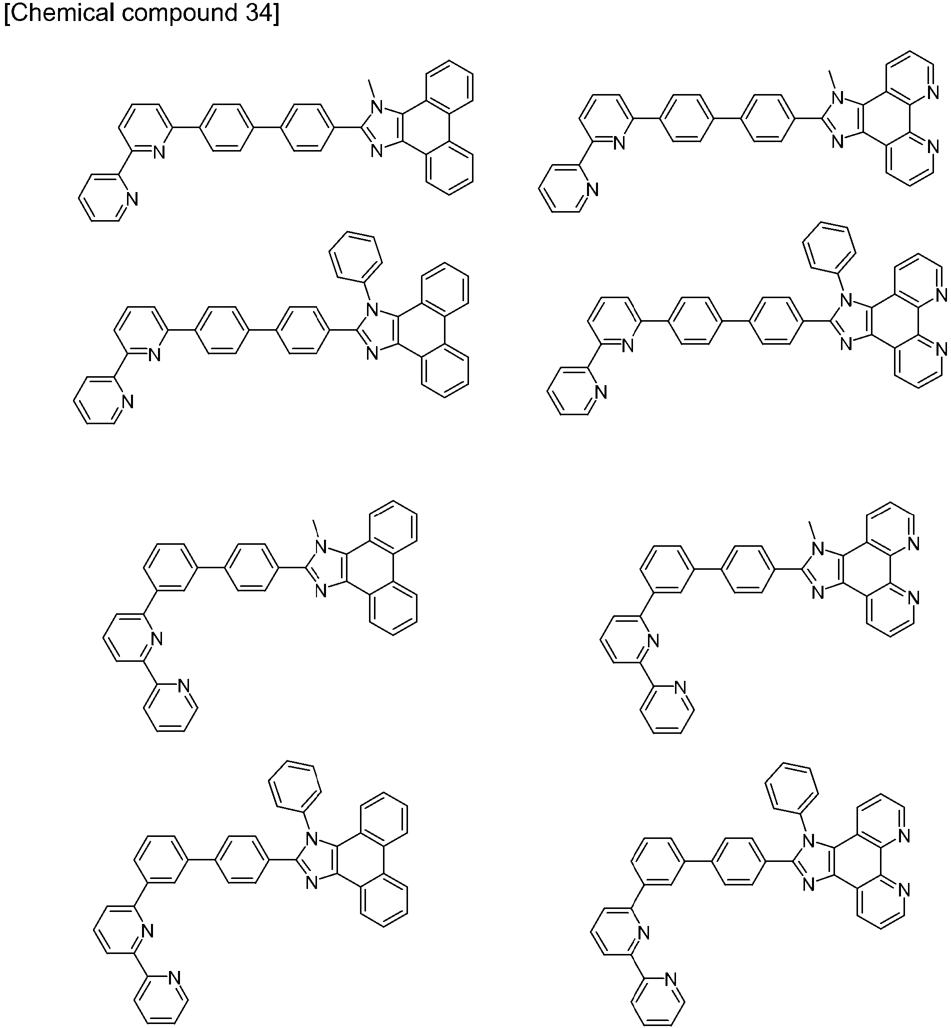

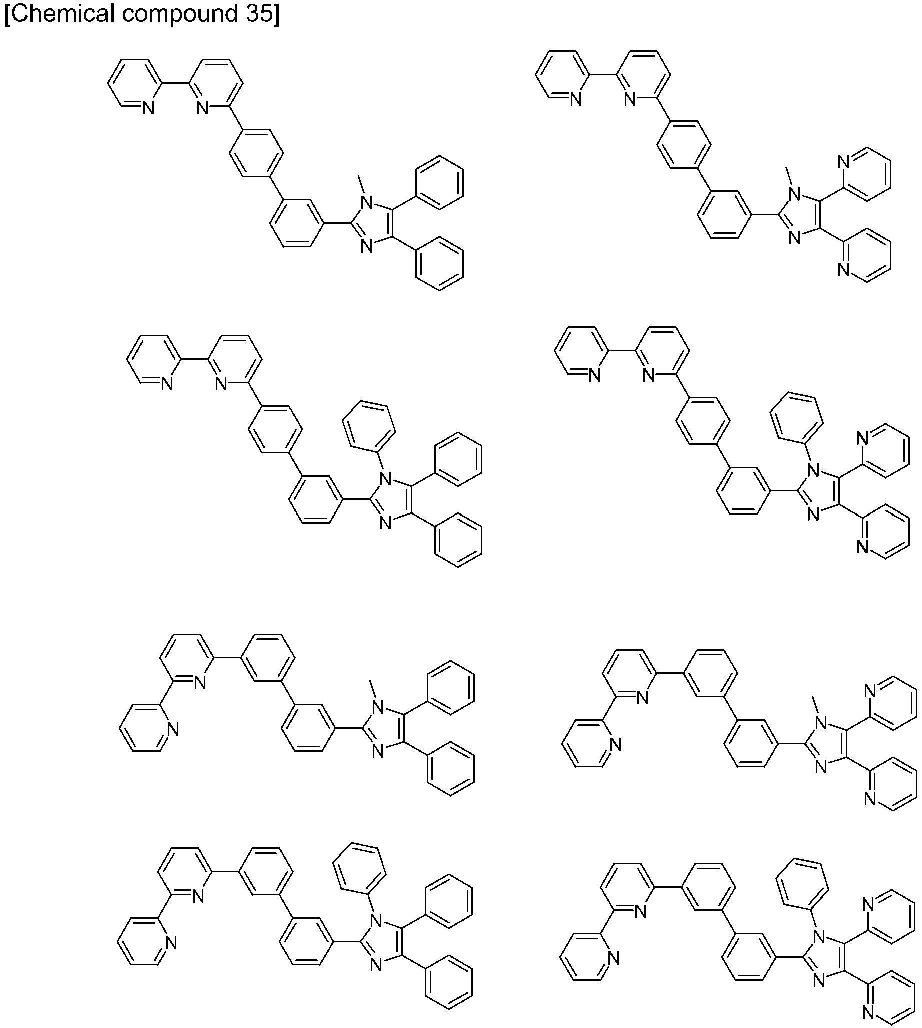

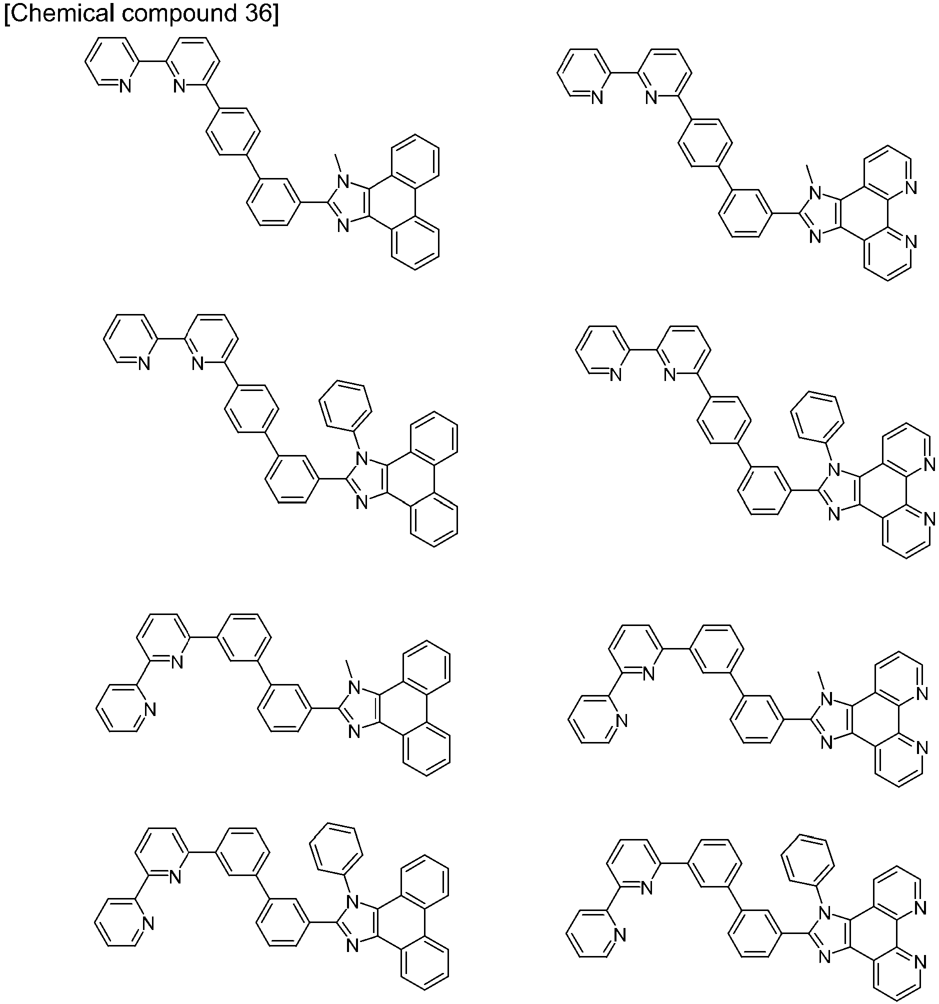

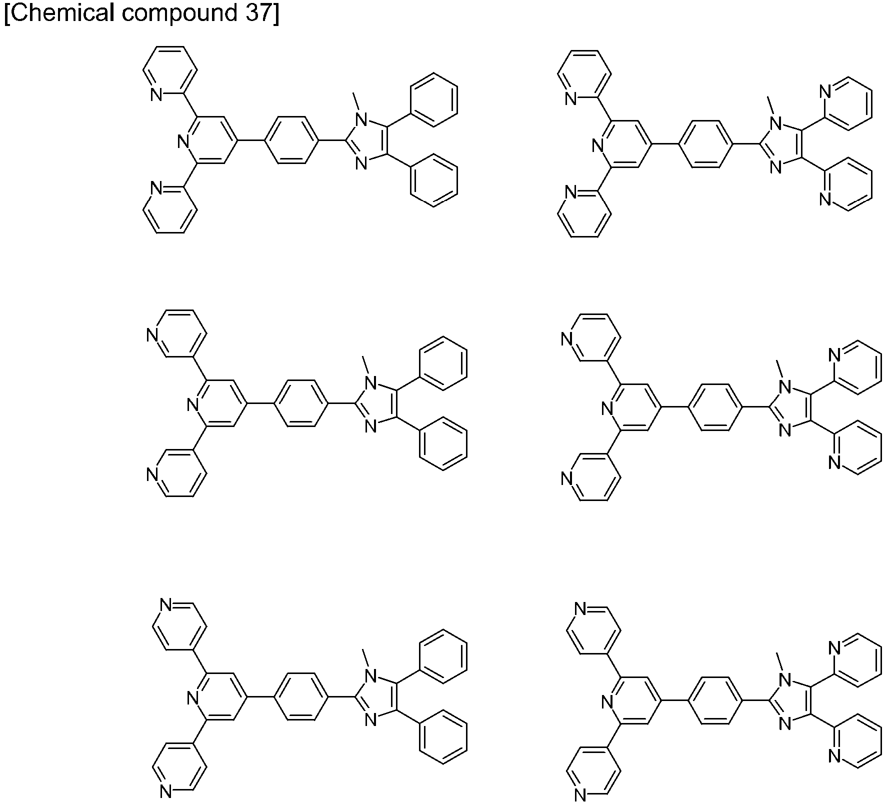

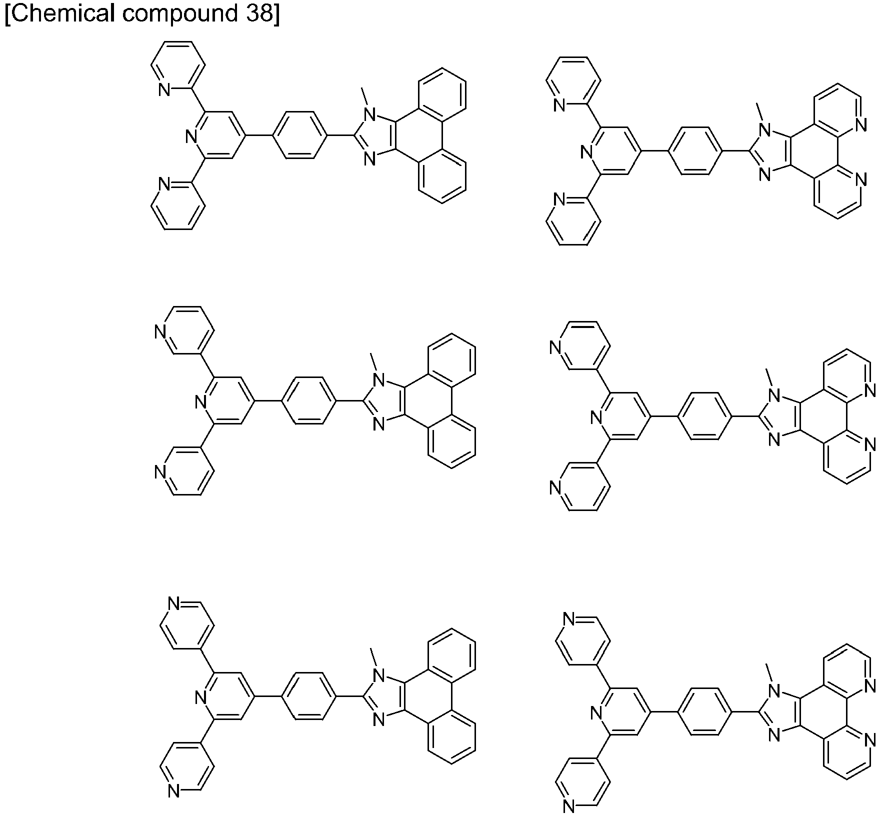

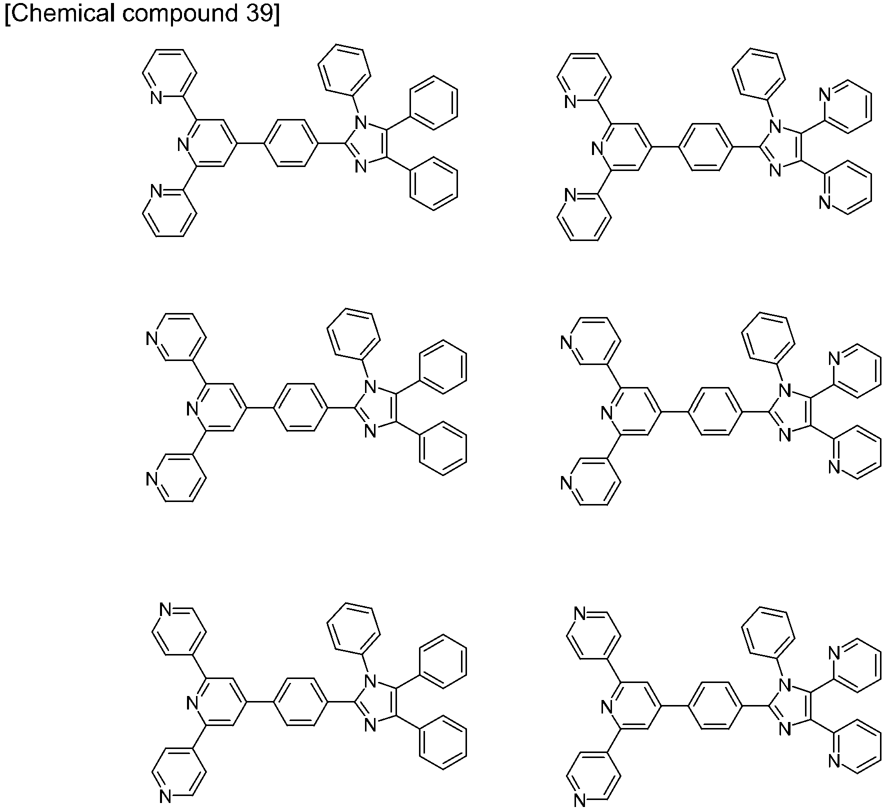

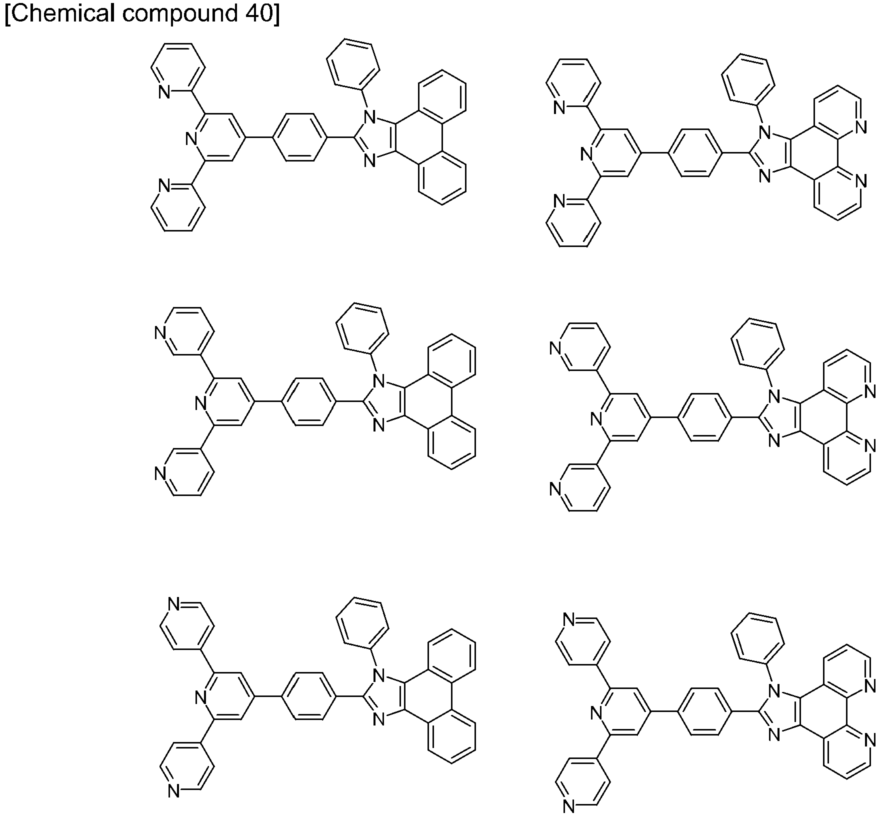

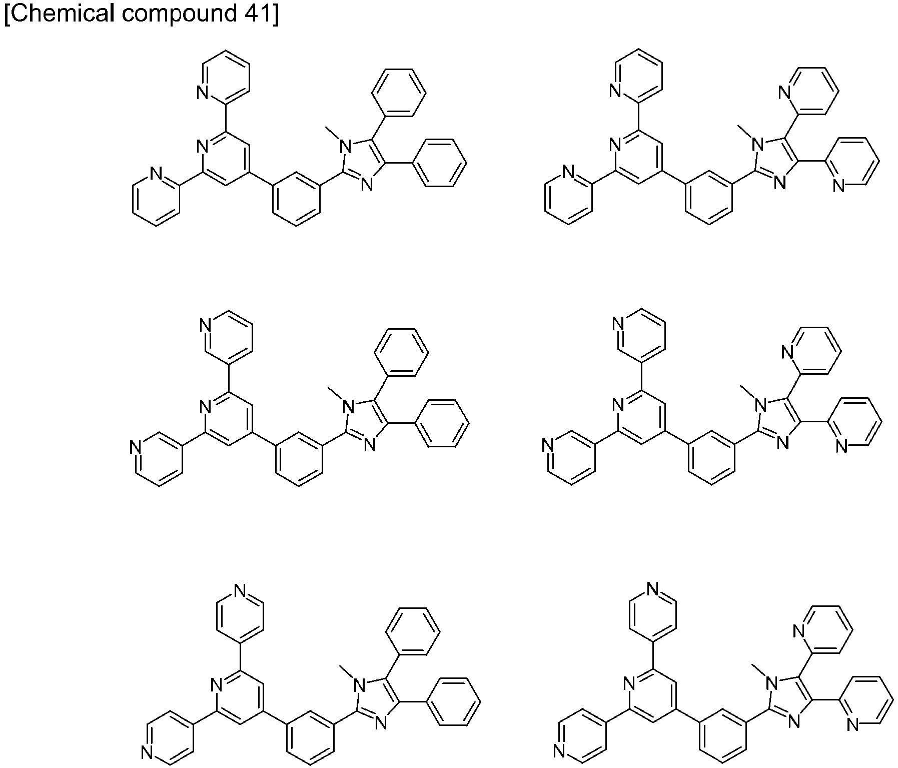

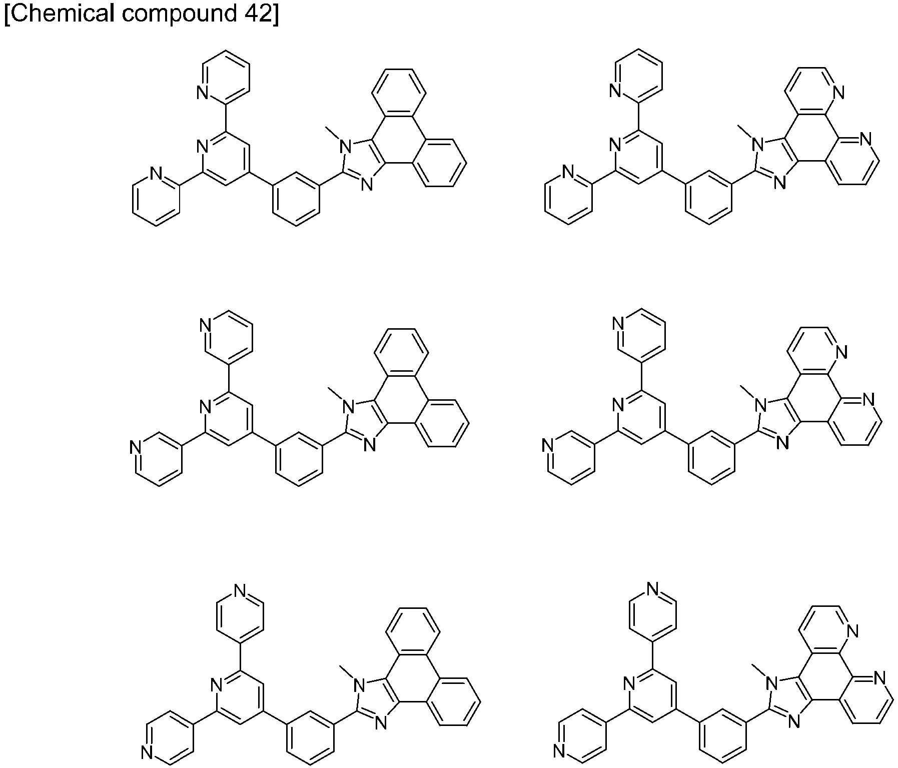

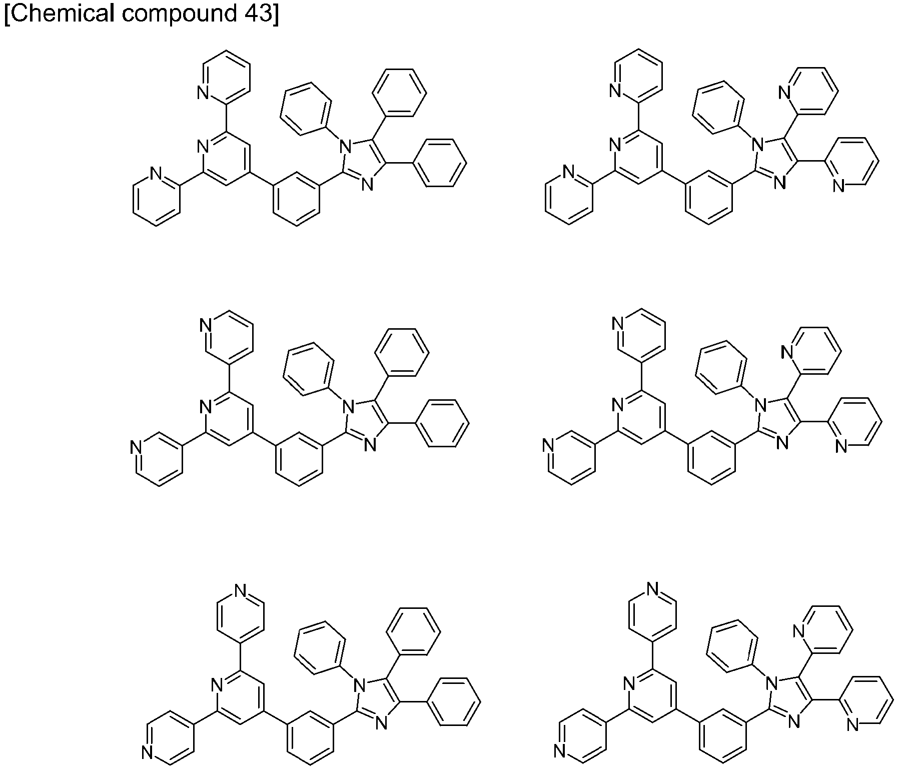

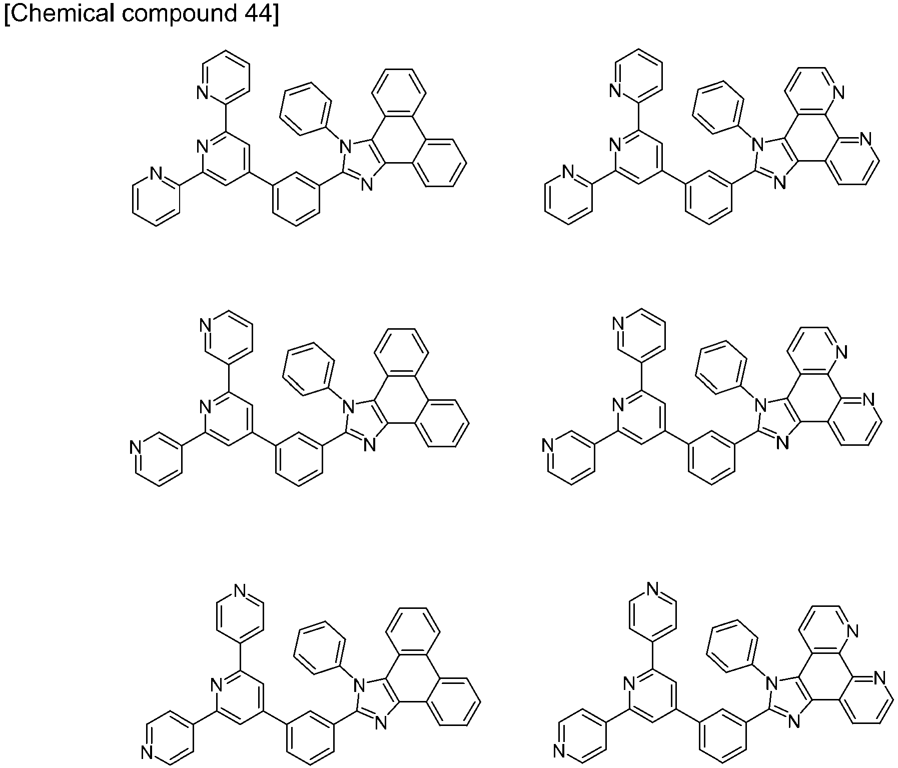

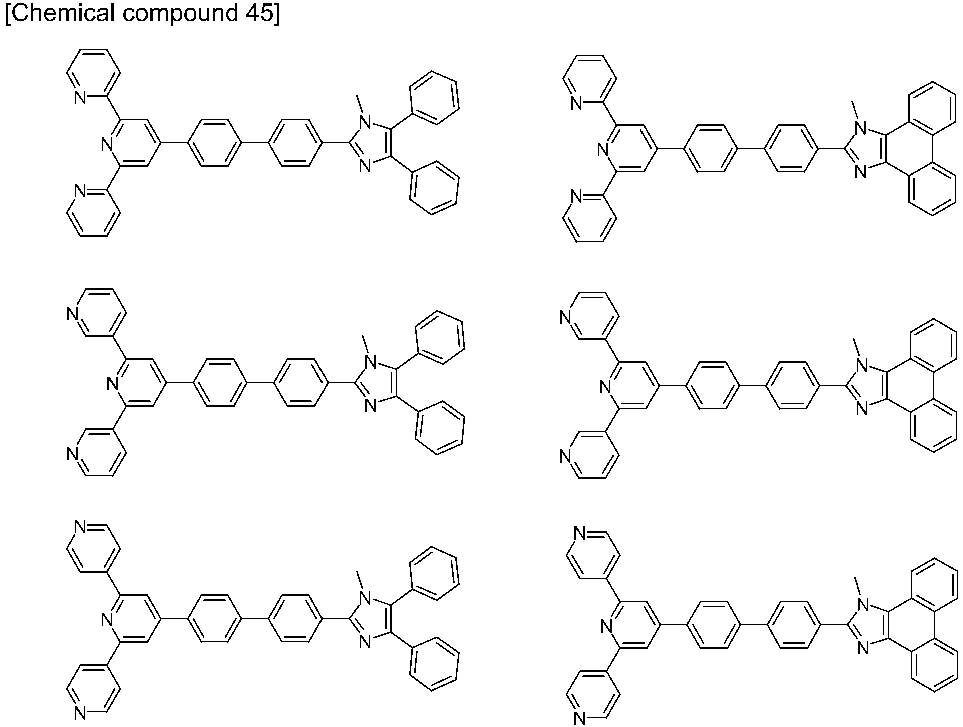

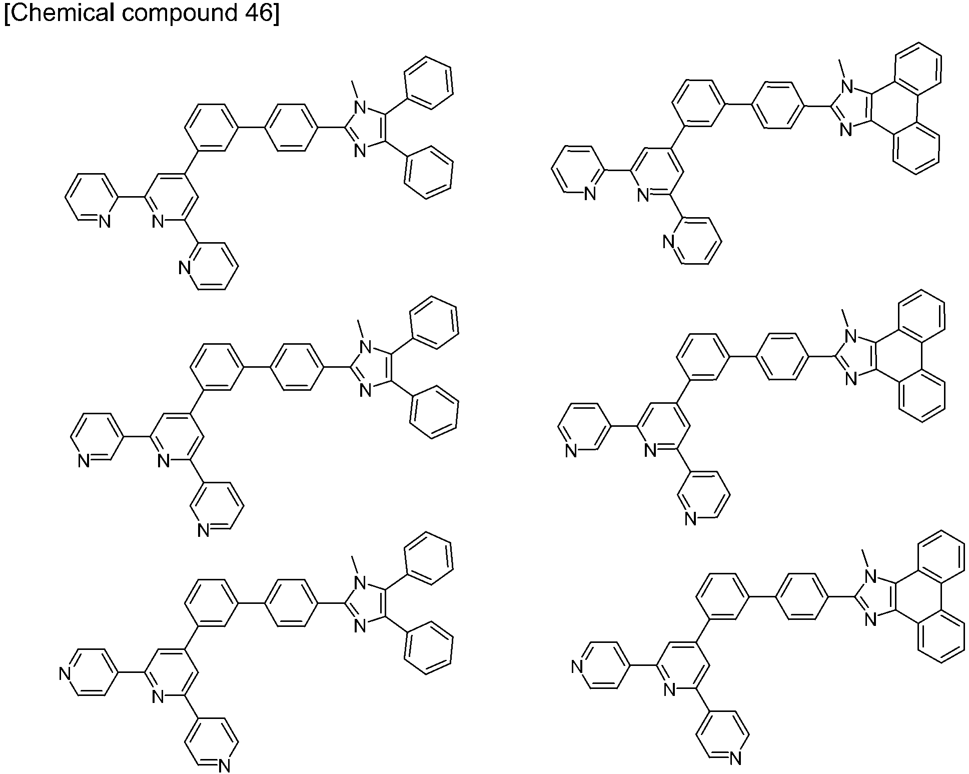

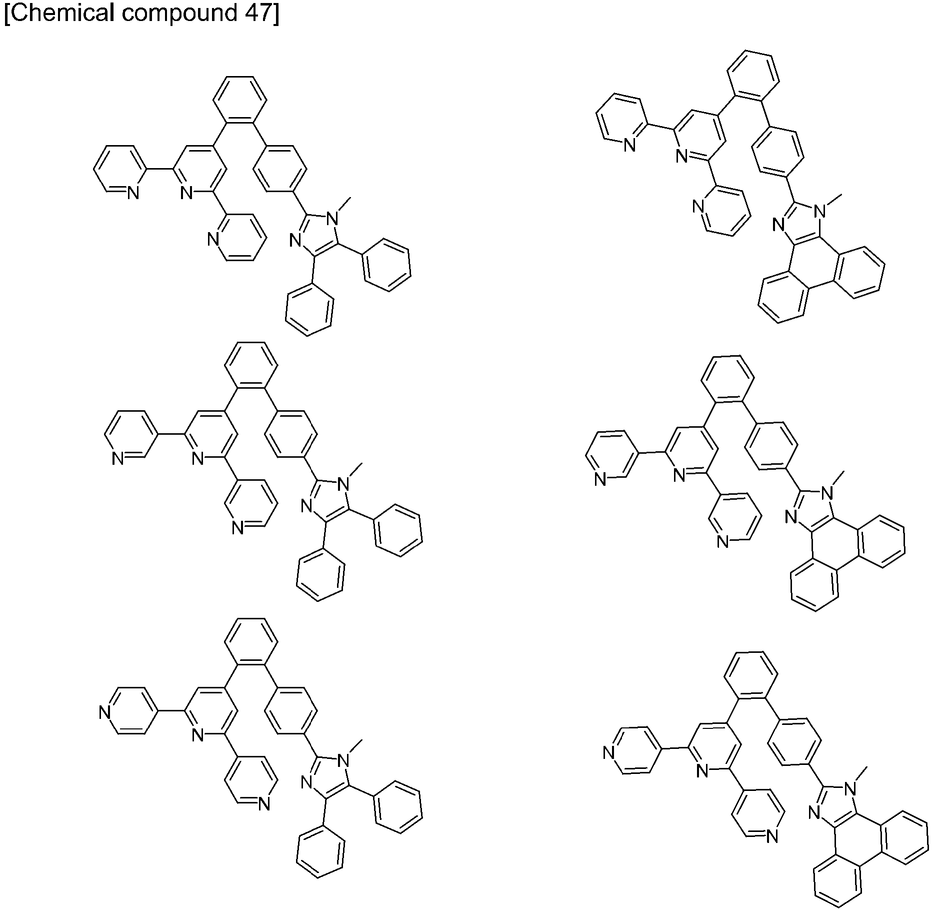

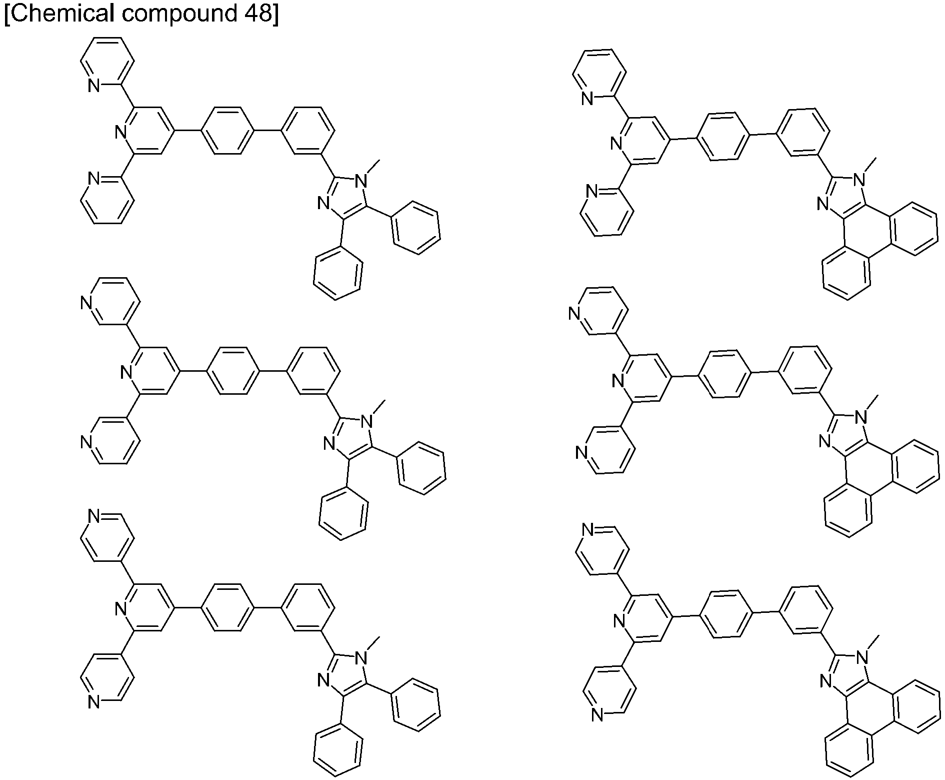

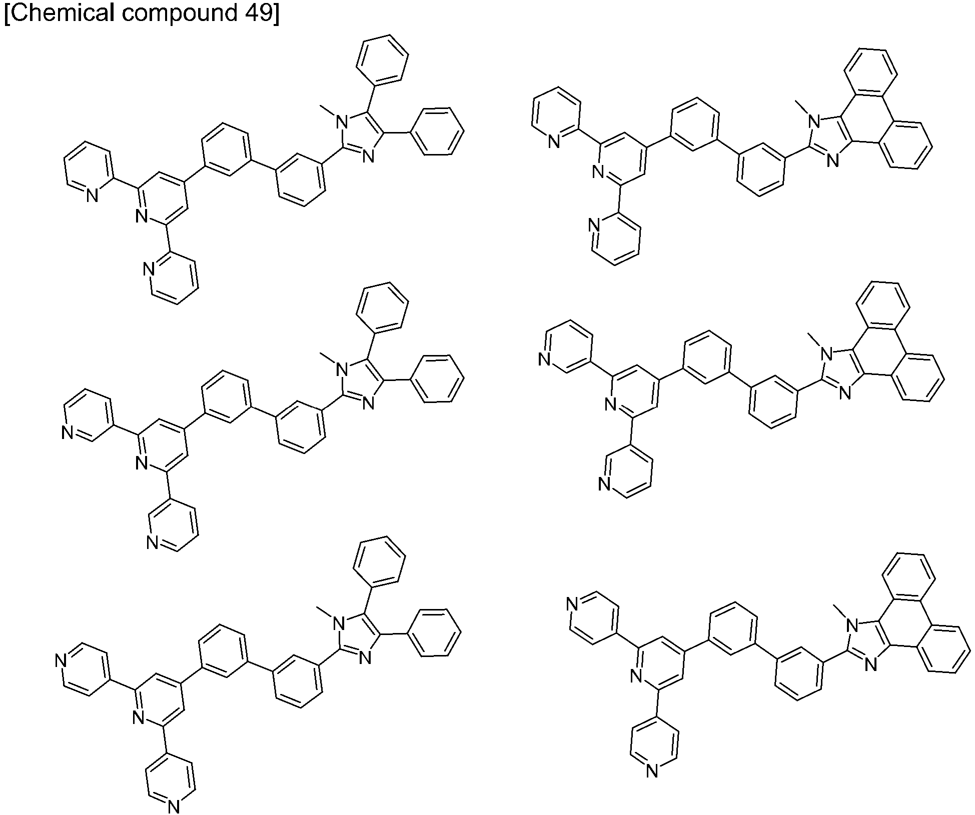

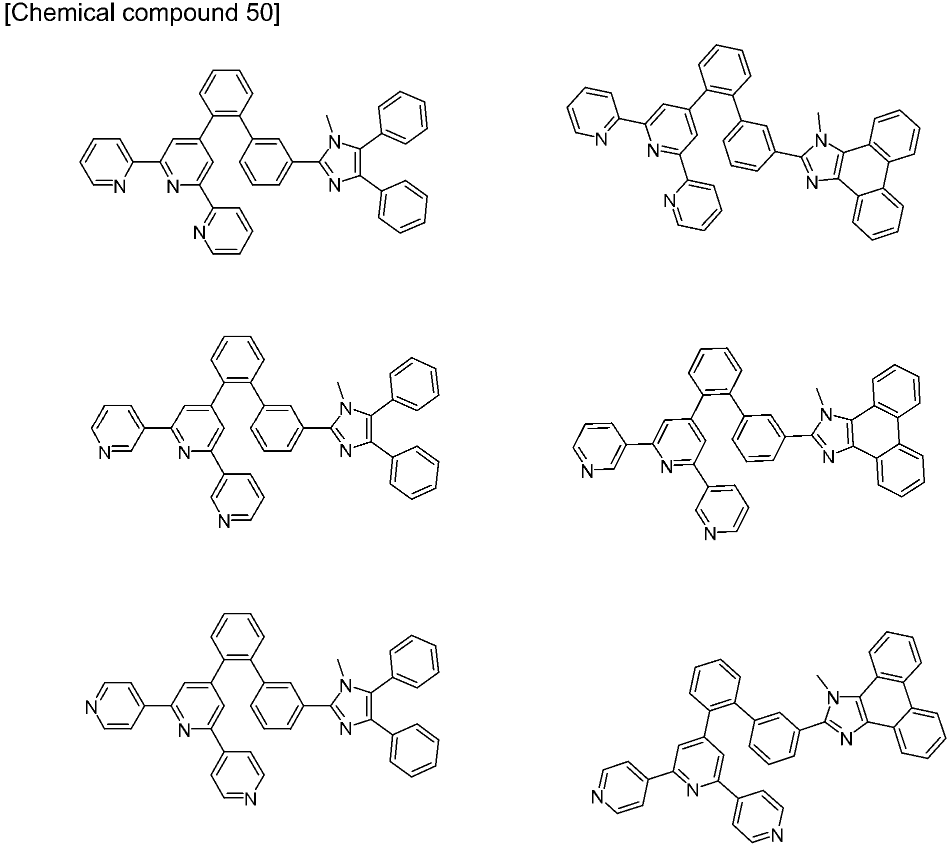

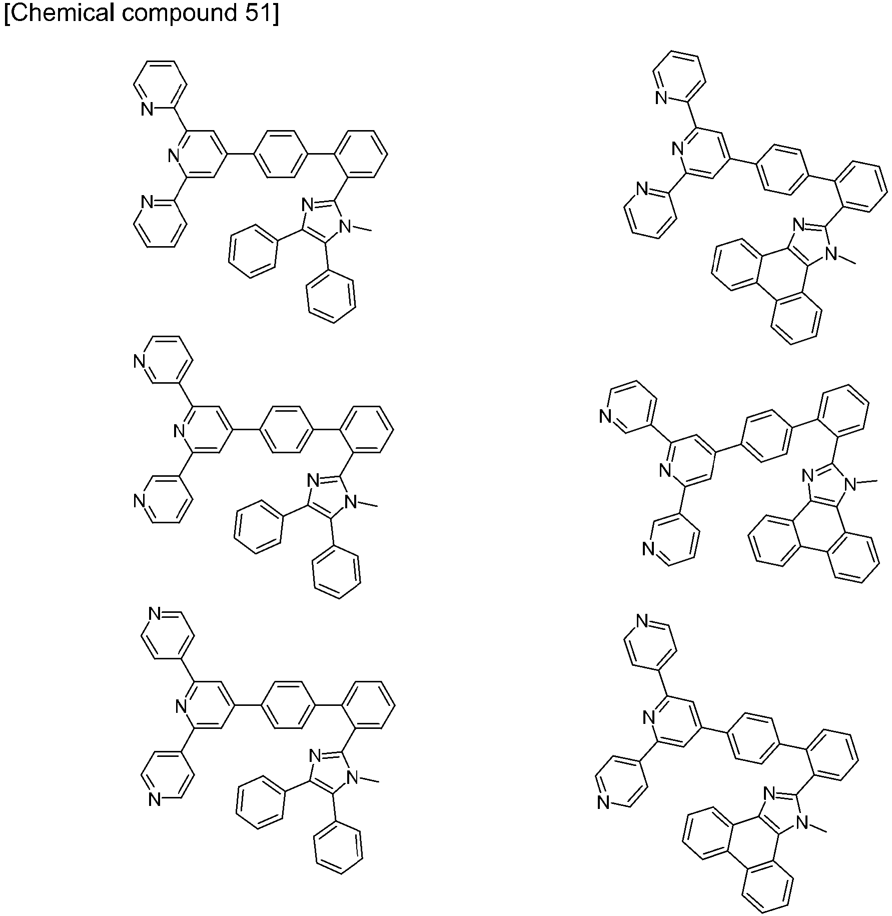

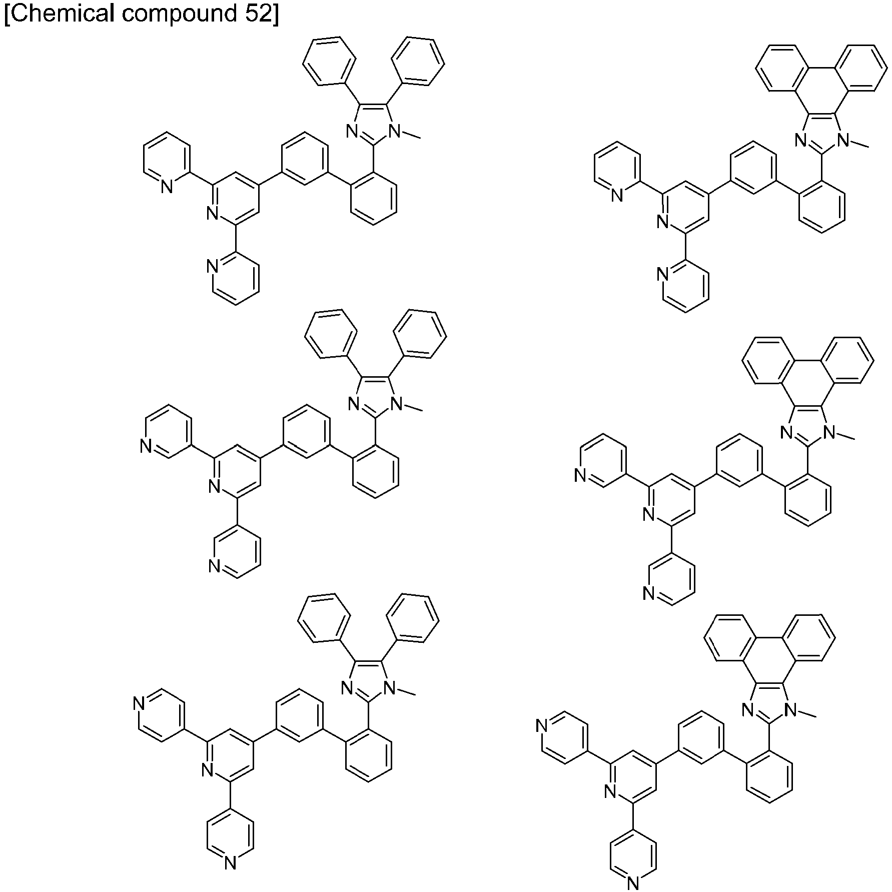

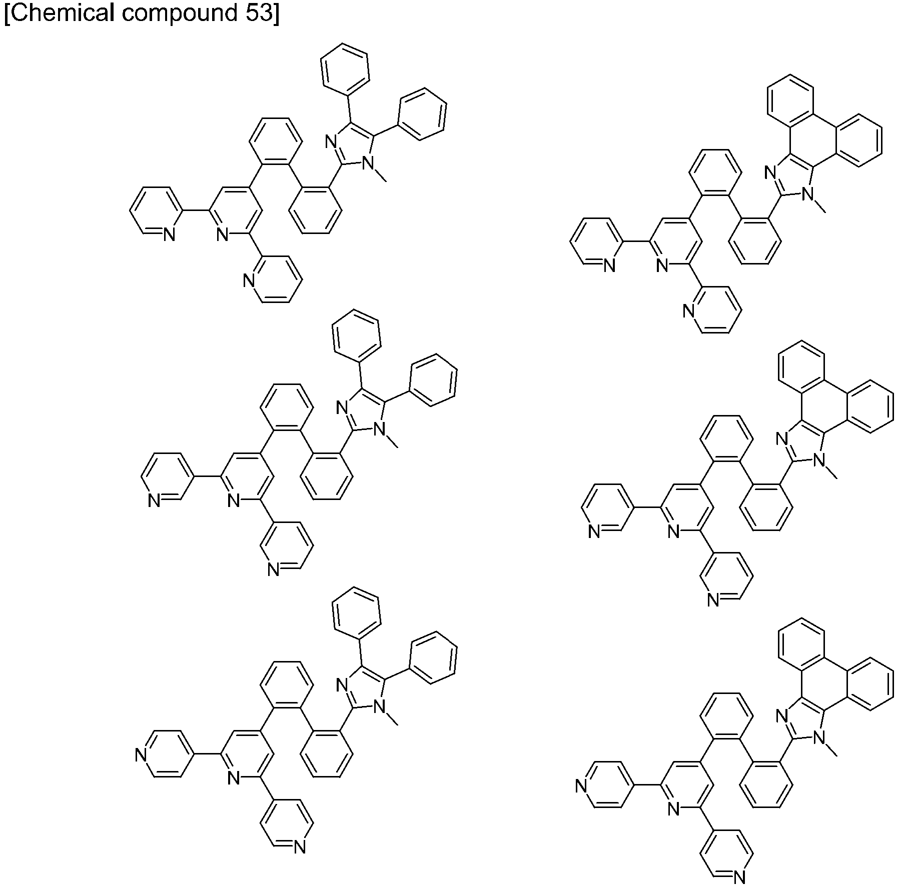

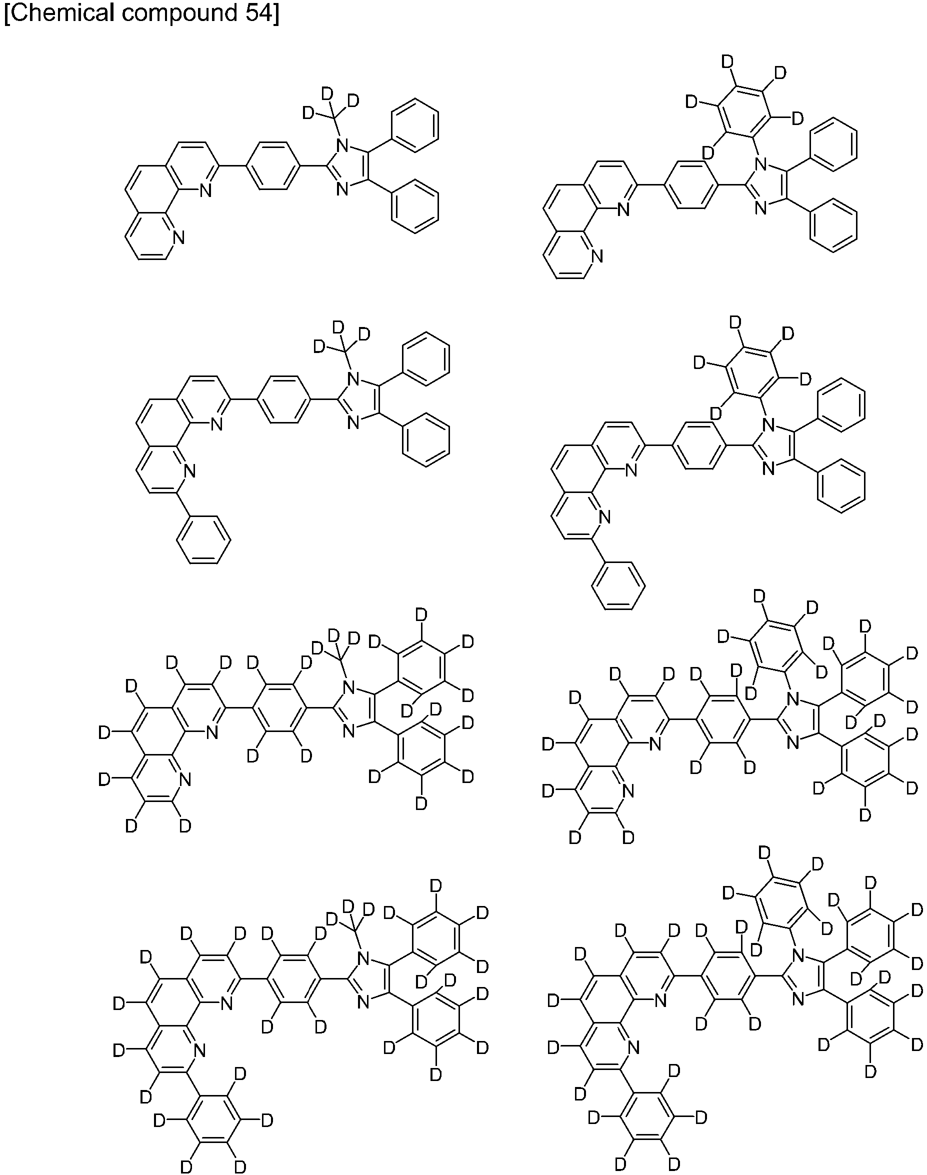

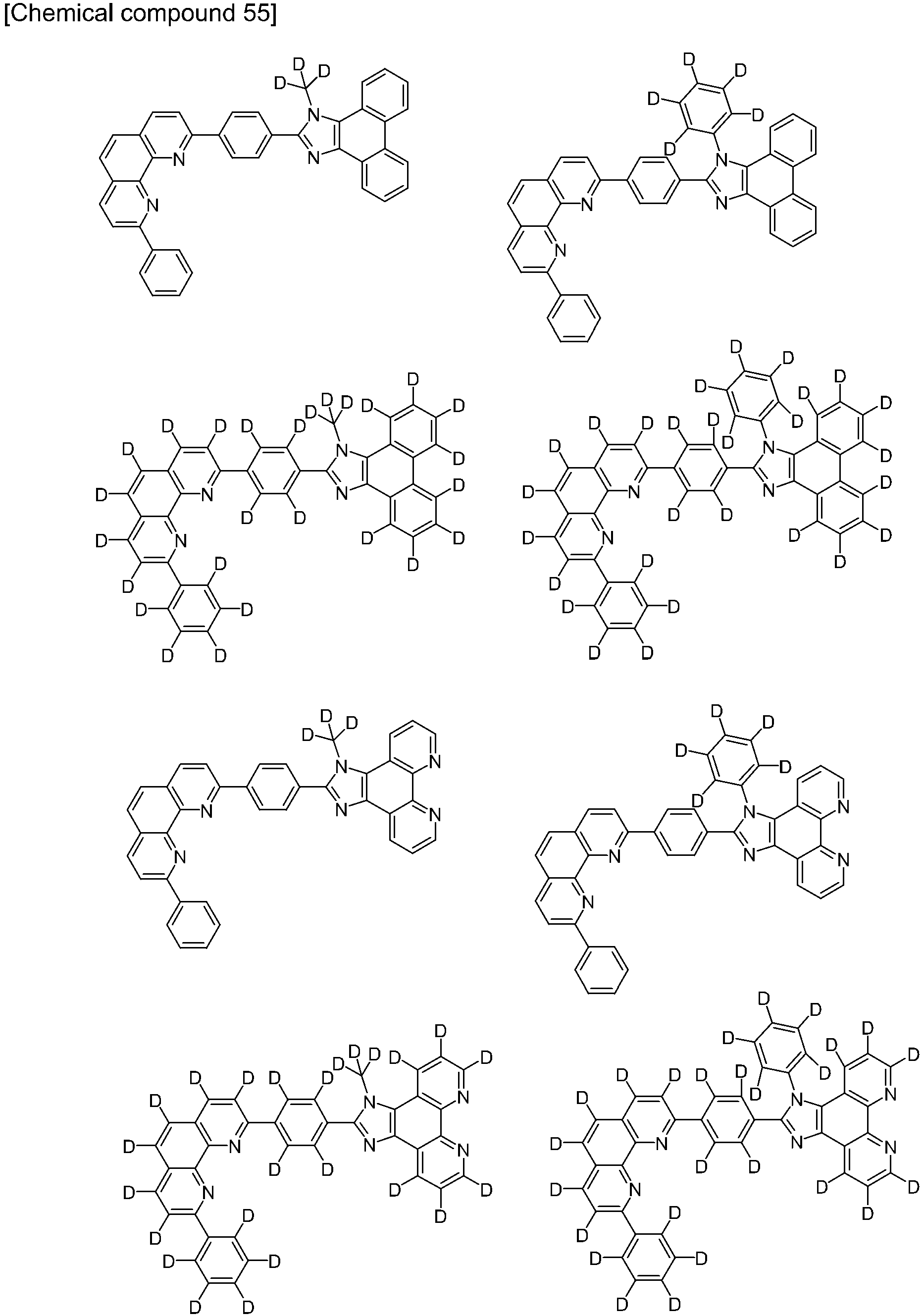

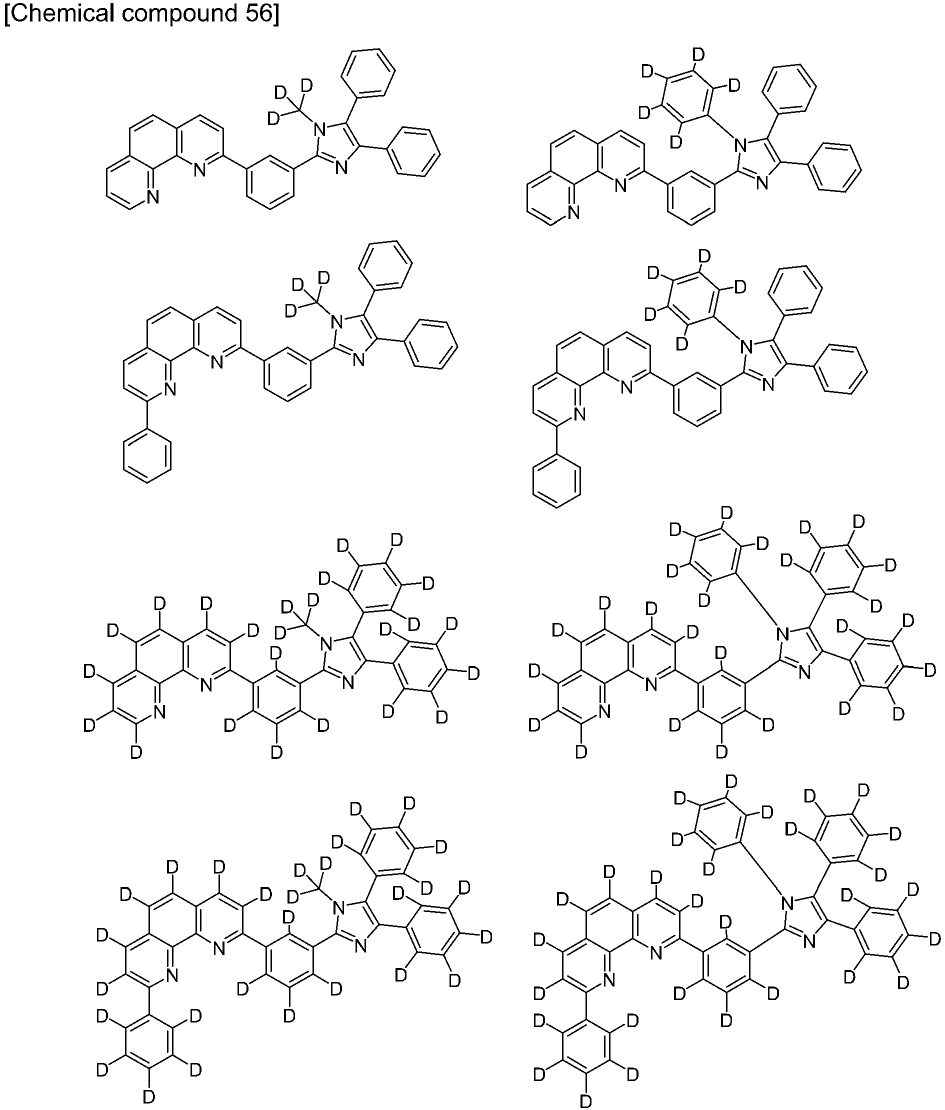

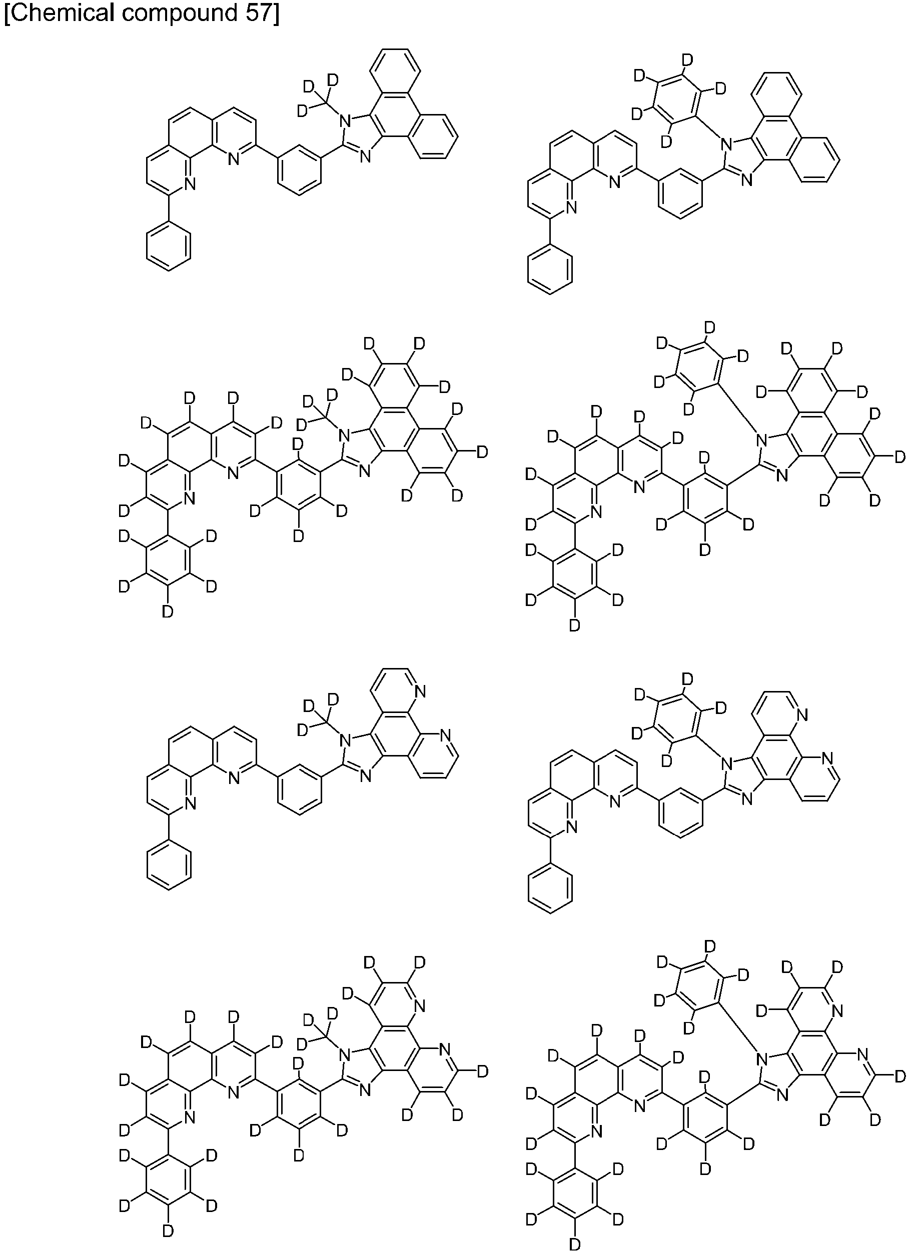

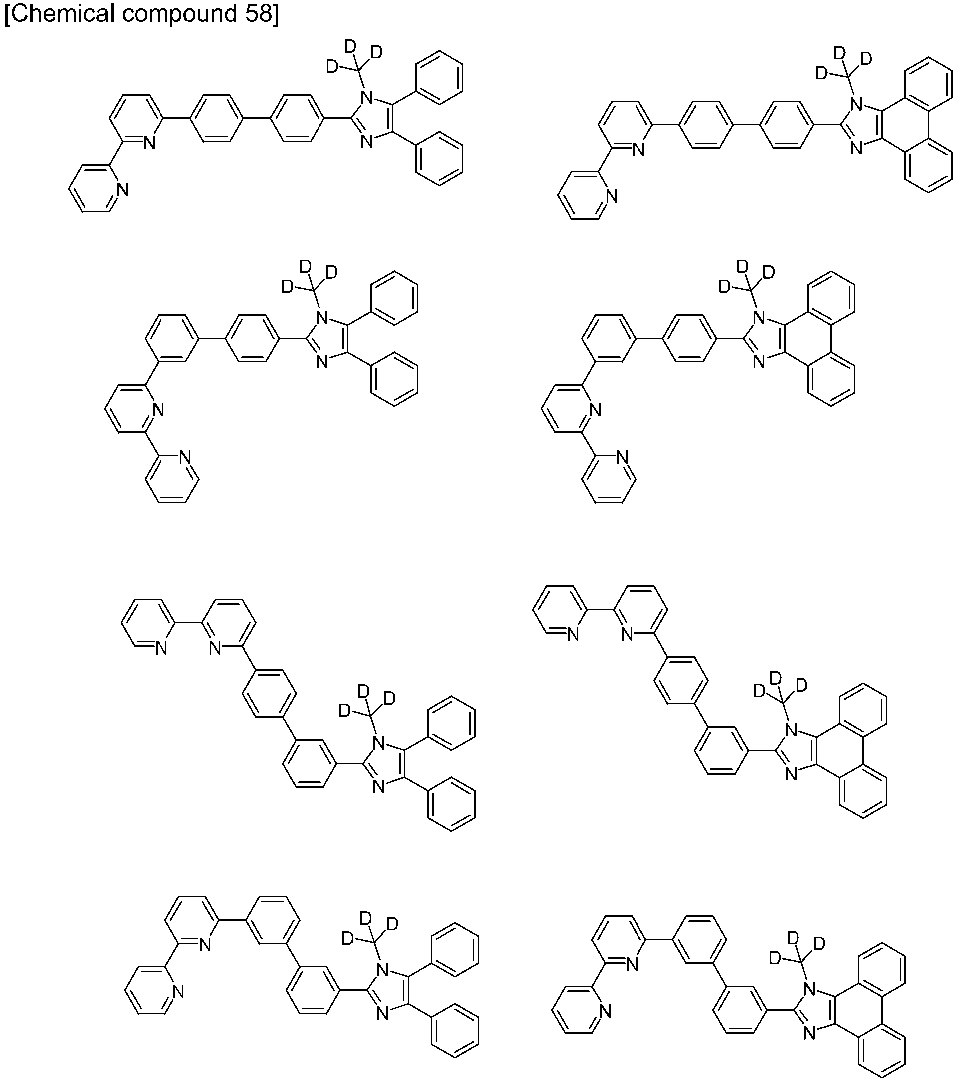

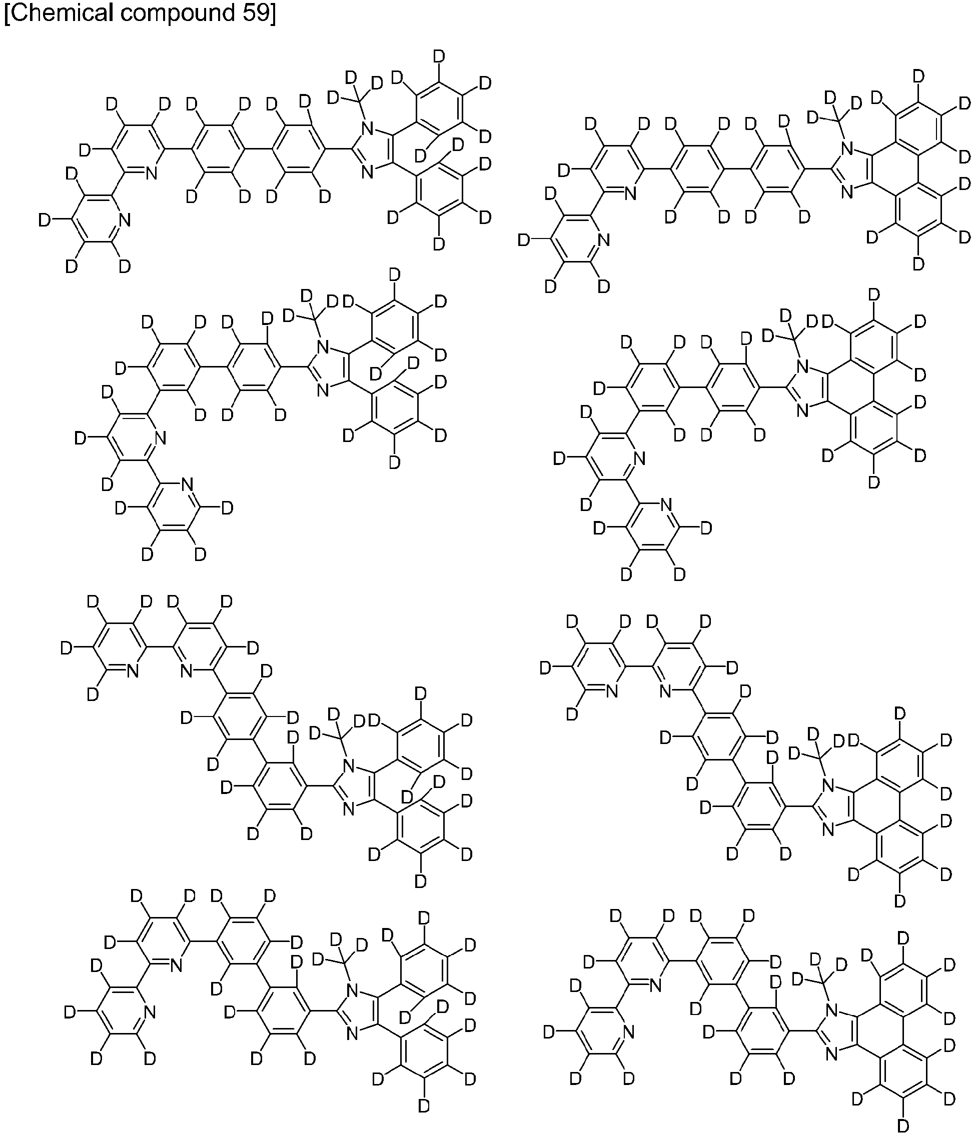

Compounds as represented by the general formula (1) can be synthesized by generally known synthesis methods. Examples of such synthesis methods include the reaction between an arylaldehyde and 1,2-dione using ammonium acetate, although useful methods are not limited thereto.

It is preferable that a compound as represented by the general formula (1) is used in any of the layers of a light emitting element. As described later, compounds as represented by the general formula (1) can be suitably used in a hole injection layer, hole transporting layer, light emitting layer, electron transporting layer, or electrode capping film (capping layer) of a light emitting element. The presence of a material as represented by the general formula (1) in any of the layers of a light emitting element serves to produce a light emitting element having high luminance efficiency and long service life.

### (Light emitting element)

A light emitting element contains an anode, a cathode, and an organic layer interposed between the anode and the cathode, and the organic layer works for light emission by using electrical energy. In the following description, such a light emitting element will be occasionally referred to as organic EL element.

Examples of the layer structure located between the anode and the cathode in an organic EL element include, in addition to the use of a light emitting layer alone, the following: 1) light emitting layer / electron transporting layer, 2) hole transporting layer / light emitting layer, 3) hole transporting layer / light emitting layer / electron transporting layer, 4) hole injection layer / hole transporting layer / light emitting layer / electron transporting layer, 5) hole transporting layer / light emitting layer / electron transporting layer / electron injection layer, 6) hole injection layer / hole transporting layer / light emitting layer / electron transporting layer / electron injection layer, and 7) hole injection layer / hole transporting layer / light emitting layer / hole blocking layer / electron transporting layer / electron injection layer.

Furthermore, a tandem type structure in which two or more of the aforementioned layered structures are stacked with interlayers interposed in between may also be used. Examples of such interlayers are also referred to as interlaminar electrode, interlaminar conductive layer, charge generation layer, electron extraction layer, connection layer, and interlaminar insulating layer, and generally known material configurations can be used. Examples of tandem type structures include: 8) hole transporting layer / light emitting layer / electron transporting layer / charge generation layer / hole transporting layer / light emitting layer / electron transporting layer, and 9) hole injection layer / hole transporting layer / light emitting layer / electron transporting layer / electron injection layer / charge generation layer / hole injection layer / hole transporting layer / light emitting layer / electron transporting layer / electron injection layer, where a charge generation layer is located as an interlayer between the anode and the cathode.

Each of the above layers may be a single layer or composed of a plurality of layers and they may be doped. In particular, it is preferable for the electron injection layer and the charge generation layer to be metal-doped layers because doping with a metal can serve to improve electron transport ability and ability for electron injection into adjacent layers. Furthermore, it is preferable to add a capping layer in addition to the above layers because its optical interference effect can realize further enhancement in luminance efficiency.

Compounds as represented by the general formula (1) may be used in any of the aforementioned layers in an organic EL element, but they are used particularly favorably in an electron transporting layer, charge generation layer, or electron injection layer. According to preferred embodiments of the present invention, organic EL elements preferably have structures as follows: a structure including at least an electron transporting layer and a light emitting layer between an anode and a cathode, wherein the electron transporting layer contains a compound as represented by the general formula (1); a structure including at least a charge generation layer and a light emitting layer between an anode and a cathode, wherein the charge generation layer contains a compound as represented by the general formula (1); and a structure including at least an electron injection layer and a light emitting layer between an anode and a cathode, wherein the electron injection layer contains a compound as represented by the general formula (1). Materials for organic EL elements as represented by the general formula (1) may be present in two or more of the above layers.

In the organic EL element according to an embodiment of the present invention, the anode and the cathode play a role in supplying a sufficient electric current to allow the element to emit light and it is desirable for at least either of them to be transparent or translucent to extract light. Commonly, the anode formed on the substrate is a transparent electrode.

### (Substrate)

In order to ensure mechanical strength of an organic EL element, it is preferable that the organic EL element is formed on a substrate. Examples of the substrate include glass substrates of soda glass, alkali-free glass, or the like, and plastic substrates. When using a glass substrate, it suffices that it is thick enough to ensure a required mechanical strength, and a thickness of 0.5 mm or more is sufficient. In regard to material of the glass, the use of alkali-free glass is preferable because it does not release significant ion elution. In addition, soda lime glass with a barrier coat of SiO₂ or the like is now commercially available and this may be used.

### (Anode)

An anode is formed on the substrate. The material to use for the anode is preferably one that can enable efficient hole injection into an organic layer. It is also preferable that the material is transparent or translucent to extract light. Examples of the material to use to form the anode include electrically conductive metal oxides such as zinc oxide, tin oxide, indium oxide, indium tin oxide (ITO), and indium zinc oxide (IZO); metals such as gold, silver, and chromium; inorganic electrically conductive substances such as copper iodide and copper sulfide; and electrically conductive polymers such as polythiophene, polypyrrole, and polyaniline. In particular, the use of ITO glass or NESA glass is preferable. Each of these electrode materials may be used singly or a plurality of these materials may be used in the form of a stack of layers or a mixture.

### (Cathode)

There are no specific limitations on the material of the cathode as long as the material can work efficiently to inject electrons into the light emitting layer. Useful materials for the cathode include metals such as platinum, gold, silver, copper, iron, tin, aluminum, and indium as well as alloys and multilayer laminates formed of these metals with other metals having low work functions such as lithium, sodium, potassium, calcium, and magnesium. Among these, from the perspective of electrical resistance, ease of film formation, film stability, and luminance efficiency, it is preferable that the cathode contain, as main component, a metal selected from the group consisting of aluminum, silver, and magnesium, and from the perspective of easy electron injection into the electron transporting layer and electron injection layer, it is more preferably composed mainly of magnesium and silver.

### (Capping layer)

In order to protect the cathode, it is preferable to add a capping layer on the cathode. There are no specific limitations on the material (capping material) to use for the capping layer, but good examples include metals such as platinum, gold, silver, copper, iron, tin, aluminum, and indium; alloys containing these metals; inorganic substances such as silica, titania, and silicon nitride; and organic polymer compounds such as polyvinyl alcohol, polyvinyl chloride, and hydrocarbon based polymers. In addition, compounds as represented by the general formula (1) can also be used as capping materials. However, in the case where the intended organic EL element has a structure designed to extract light from the cathode side (top emission structure), it is preferable that the capping material to use be optically transparent in the visible light region.

### (Hole injection layer)

The hole injection layer is a layer interposed between the anode and the hole transporting layer. The hole injection layer may be a single layer or a stack of a plurality of layers. The presence of a hole injection layer between the hole transporting layer and the anode is preferable because this allows driving at a lower voltage and an increase in service life and furthermore, it serves to produce an element having an improved carrier balance, leading to an increased luminance efficiency.

Generally known materials can be used to form a hole injection layer. Examples thereof include heterocyclic compounds such as benzidine derivatives, materials known as starburst arylamine compounds, triarylamine derivatives, biscarbazole derivatives, pyrazoline derivatives, stilbene based compounds, fluorene based compounds, hydrazone based compounds, benzofuran derivatives, thiophene derivatives, oxadiazole derivatives, phthalocyanine derivatives, and porphyrin derivatives; polycarbonates and styrene derivatives having aforementioned derivatives or compounds as side chains; and other polymeric materials such as polythiophene, polyaniline, polyfluorene, polyvinylcarbazole, and polysilane. From the perspective of smooth injection and transport of holes from the anode into the hole transporting layer, benzidine derivatives, starburst arylamine based materials, and fluorene based compounds are used preferably.

These materials may be used singly or a plurality of these materials may be used as a mixture. Alternatively, a plurality of the materials may be stacked to form a hole injection layer. It is preferable, furthermore, that this hole injection layer be formed only of an acceptor type compound or formed of a hole injection material as given above that is doped with an acceptor type compound because the effect described above will be further enhanced. An acceptor type compound as referred to herein is one that forms a charge-transfer complex by working with the adjacent hole transporting layer in the case where the compound is in the form of a monolayer film or working with the material present in the hole injection layer in the case where it is used as a dopant. The use of such a material serves to improve the electric conductivity of the hole injection layer and contributes to decreasing the driving voltage of the element, leading to a higher luminance efficiency and longer service life.

The acceptor type compound to use may be a generally known substance. Examples thereof include metal chlorides, metal oxides such as molybdenum oxide, charge-transfer complexes, and organic compounds having a nitro group, cyano group, halogen atom, or trifluoromethyl group within the molecules, as well as quinone based compounds, acid anhydride based compounds, and fullerene. Of these, metal oxides and cyano group-containing compounds are preferable because they can be handled easily and evaporated easily, allowing the effects described above to be realized easily. Both in the case where the hole injection layer is formed only of an acceptor type compound and in the case where the hole injection layer is doped with an acceptor type compound, the hole injection layer may be in the form of a monolayer or a stack of a plurality of layers.

### (Hole transporting layer)

The hole transporting layer acts to transport holes, which are injected from the anode, to the light emitting layer. The hole transporting layer may be in the form of a monolayer or a stack of a plurality of layers.

The materials listed above as being suitable for the hole injection layer may also be used as materials for the hole transporting layer. From the perspective of smoothly injecting and transporting holes into the light emitting layer, the use of a triarylamine derivative or a benzidine derivative is more preferable.

### (Light emitting layer)

The light emitting layer may be in the form of a single layer or a stack of a plurality of layers. A light emitting layer is formed mainly of a light emitting material, and the light emitting material may be any of a mixture of a host material and a dopant material, a single host material, and a mixture of two host materials and one dopant material. This means that for the organic EL element according to an embodiment of the present invention, either only the host material or only the dopant material may emit light from the light emitting layer, or both the host material and the dopant material may emit light. From the perspective of efficient use of electrical energy for realizing light emission with high color purity, it is preferable for the light emitting layer to be formed of a mixture of a host material and a dopant material. The host material and the dopant material may each be either a single material or a combination of a plurality of materials.

In the case where the light emitting layer includes a mixture of a host material and a dopant material, the dopant material may be completely contained in the host material or partially contained therein. The dopant material may be either laminated or dispersed. The dopant material can serve to control the luminescent color. From the perspective of suppressing concentration quenching, it is preferable that the dopant material account for 30 wt% or less, more preferably 20 wt% or less, relative to the total amount, which accounts for 100 wt%, of the host material and the dopant material. Its doping may be achieved by means of coevaporation with a host material or by means of evaporation of a mixture with a host material prepared in advance.

The light emitting material to use may be a generally known substance. Examples thereof include fused ring derivatives such as anthracene and pyrene, which are conventionally known as illuminants; metal-chelated oxynoid compounds such as tris-(8-quinolinolato)aluminum; bisstyryl derivatives such as bisstyryl anthracene derivatives and distyryl benzene derivatives; and others such as tetraphenyl butadiene derivatives, indene derivatives, coumarin derivatives, oxadiazole derivatives, pyrrolopyridine derivatives, perinone derivatives, cyclopentadiene derivatives, thiadiazolopyridine derivatives, dibenzofuran derivatives, carbazole derivatives, indolocarbazole derivatives; as well as polymer type ones such as polyphenylene vinylene derivatives, polyparaphenylene derivatives, and polythiophene derivatives.

The host material contained in the light emitting material is not necessarily a compound of a single species and it may be a mixture of a plurality of the compounds. Furthermore, they may be laminated. The host material to use may be a generally known substance. There are no specific limitations thereon and useful ones include compounds having fused aryl rings such as naphthalene, anthracene, phenanthrene, pyrene, chrysene, naphthacene, triphenylene, perylene, fluoranthene, fluorene, and indene, as well as derivatives thereof; aromatic amine derivatives such as N,N'-dinaphthyl-N,N'-diphenyl-4,4'-diphenyl-1,1'-diamine; metal-chelated oxynoid compounds such as tris-(8-quinolinato)aluminum (III); bisstyryl derivatives such as distyryl benzene derivatives; and others such as tetraphenyl butadiene derivatives, indene derivatives, coumarin derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, carbazole derivatives, indolocarbazole derivatives, triazine derivatives; as well as polymer type ones such as polyphenylene vinylene derivatives, polyparaphenylene derivatives, polyfluorene derivatives, polyvinyl carbazole derivatives, and polythiophene derivatives. In particular, in the case of a light emitting layer designed for triplet light emission (phosphorescene emission), host materials that can be used suitably include metal-chelated oxynoid compounds, dibenzofuran derivatives, dibenzothiophene derivatives, carbazole derivatives, indolocarbazole derivatives, triazine derivatives, and triphenylene derivatives.

Examples of the dopant material to be incorporated in the light emitting material include compounds having aryl rings and derivatives thereof, compounds having heteroaryl rings and derivatives thereof, distyrylbenzene derivatives, aminostyryl derivatives, aromatic acetylene derivatives, tetraphenylbutadiene derivatives, stilbene derivatives, aldazine derivatives, pyrromethene derivatives, diketopyrrolo[3,4-c]pyrrole derivatives, coumarin derivatives, azole derivatives and metal complexes thereof, aromatic amine derivatives, and compounds as represented by the general formula (7) given below. Among these, dopant materials containing diamine skeletons and dopant materials containing fluoranthene skeletons can serve to further improve the luminance efficiency, and compounds as represented by the general formula (7) can work to further enhance the luminance efficiency and service life.

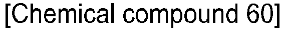

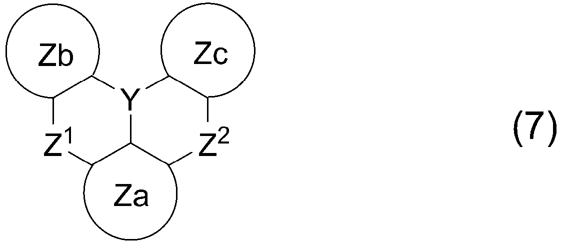

In the general formula (7), the Za ring, Zb ring, and Zc ring are each independently a substituted or unsubstituted aryl ring having 6 to 30 ring-forming carbon atoms or a substituted or unsubstituted heteroaryl ring having 5 to 30 ring-forming atoms. Z¹ and Z² are each independently an oxygen atom, NRa (a nitrogen atom having a substituent Ra), or a sulfur atom. When Z¹ is NRa, Ra may or may not form a ring by bonding with the Za ring or the Zb ring, while when Z² is NRa, Ra may or may not form a ring by bonding with the Zb ring or the Zc ring. In the case where both Z¹ and Z² are NRa's, each Ra is independently a substituted or unsubstituted aryl group having 6 to 30 ring-forming carbon atoms, a substituted or unsubstituted heteroaryl group having 5 to 30 ring-forming atoms, or a substituted or unsubstituted alkyl group having 1 to 30 carbon atoms. It is preferable that in the general formula (7), both Z¹ and Z² are NRa's wherein each Ra is a substituted or unsubstituted aryl group having 6 to 30 ring-forming carbon atoms. In the general formula (7), Y is a boron atom, phosphorus atom, SiRb (a silicon atom having a substituent Rb), P=O, or P=S. Each Rb is independently a substituted or unsubstituted aryl group having 6 to 30 ring-forming carbon atoms, a substituted or unsubstituted heteroaryl group having 5 to 30 ring-forming atoms, or a substituted or unsubstituted alkyl group having 1 to 30 carbon atoms. It is preferable that Y be a boron atom. In the case where a substituent is present on any of the above groups, preferable examples thereof include alkyl groups, cycloalkyl groups, heteroalicyclic groups, alkenyl groups, cycloalkenyl groups, alkynyl groups, aryl groups, heteroaryl groups, hydroxyl groups, thiol groups, alkoxy groups, alkylthio groups, aryl ether groups, arylthioether groups, halogens, cyano groups, aldehyde groups, acyl groups, carboxyl groups, ester groups, amide groups, acyl groups, sulfonyl groups, sulfonic acid ester groups, sulfonamide groups, amino groups, nitro groups, silyl groups, siloxanyl groups, boryl groups, and oxo groups. These substituents may be further substituted with groups as given above.

Examples of such alkyl groups, alkoxy groups, aryl group, and heteroaryl groups include those mentioned above as substituents present in the general formula (1).

A cycloalkyl group is a saturated alicyclic hydrocarbon group such as, for example, cyclopropyl group, cyclohexyl group, norbornyl group, and adamantyl group, which may or may not contain a substituent group. There are no specific limitations on the number of ring-forming carbon atoms, but it is preferably in the range of 3 or more and 20 or less.

A heterocyclic group is an aliphatic ring having a non-carbon atom in the ring such as, for example, pyran ring, piperidine ring, and cyclic amides, which may or may not contain a substituent group. There are no specific limitations on the number of ring-forming atoms, but it is preferably in the range of 3 or more and 20 or less.

An alkenyl group is an unsaturated aliphatic hydrocarbon group containing a double bond such as, for example, vinyl group, allyl group, and butadienyl group, which may or may not contain a substituent group. There are no specific limitations on the number of carbon atoms present in an alkenyl group, but it is preferably in the range of 2 or more and 20 or less.

A cycloalkenyl group is an unsaturated alicyclic hydrocarbon group containing a double bond such as, for example, cyclopentenyl group, cyclopentadienyl group, and cyclohexenyl group, which may or may not contain a substituent group.

An alkynyl group is an unsaturated aliphatic hydrocarbon group containing a triple bond such as, for example, ethynyl group, which may or may not contain a substituent group. There are no specific limitations on the number of carbon atoms present in an alkynyl group, but it is preferably in the range of 2 or more and 20 or less.

An alkylthio group has the same structure as the corresponding alkoxy group except that the oxygen atom in the ether bond is replaced with a sulfur atom. An alkylthio group may or may not contain a substituent group. There are no specific limitations on the number of carbon atoms in an alkylthio group, but it is preferably in the range of 1 or more and 20 or less.

An aryl ether group is a functional group in which an aromatic hydrocarbon group is bonded through an ether bond such as, for example, phenoxy group, which may or may not contain a substituent group. There are no specific limitations on the number of carbon atoms in an aryl ether group, but preferably it is in the range of 6 or more and 40 or less.

An arylthioether group is a functional group that has the same structure as the corresponding aryl ether group except that the oxygen atom in the ether bond is replaced with a sulfur atom, and it may or may not contain a substituent group. There are no specific limitations on the number of carbon atoms in an arylthioether group, but preferably it is in the range of 6 or more and 40 or less.

A halogen is fluorine, chlorine, bromine, or iodine.

An acyl group refers to a functional group in which, for example, an alkyl group, cycloalkyl group, alkenyl group, alkynyl group, aryl group, heteroaryl group, or the like is bonded through a carbonyl group such as acetyl group, propionyl group, benzoyl group, and acryloyl group, and it may or may not contain a substituent group. There are no specific limitations on the number of carbon atoms present in an acyl group, but it is preferably 2 or more and 40 or less, and more preferably 2 or more and 30 or less.

An ester group refers to a functional group in which, for example, an alkyl group, cycloalkyl group, aryl group, heteroaryl group, or the like is bonded through an ester bond, and it may or may not contain a substituent group. There are no specific limitations on the number of carbon atoms in an ester group, but it is preferably in the range of 1 or more and 20 or less. More specifically, examples thereof include methyl ester groups such as methoxycarbonyl group, ethyl ester groups such as ethoxycarbonyl group, propyl ester groups such as propoxycarbonyl group, butyl ester groups such as butoxycarbonyl group, isopropyl ester groups such as isopropoxymethoxycarbonyl group, hexyl ester groups such as hexyloxycarbonyl group, and phenyl ester groups such as phenoxycarbonyl group.

An amide group refers to a functional group in which, for example, an alkyl group, cycloalkyl group, aryl group, heteroaryl group, or the like is bonded through an amide bond, and it may or may not contain a substituent group. There are no specific limitations on the number of carbon atoms present in an amide group, but it is preferably in the range of 1 or more and 20 or less. More specifically, examples thereof include methylamide group, ethylamide group, propylamide group, butylamide group, isopropylamide group, hexylamide group, and phenylamide group.

A sulfonyl group refers to a functional group in which, for example, an alkyl group, cycloalkyl group, aryl group, heteroaryl group, or the like is bonded through a -S(=O)₂- bond, and it may or may not contain a substituent group. There are no specific limitations on the number of carbon atoms present in a sulfonyl group, but it is preferably in the range of 1 or more and 20 or less.

A sulfonic acid ester group refers to a functional group in which, for example, an alkyl group, cycloalkyl group, aryl group, heteroaryl group, or the like is bonded through a sulfonic acid ester bond, and it may or may not contain a substituent group. Here, a sulfonic acid ester bond refers to a structure in which the carbonyl part, i.e. -C(=O)-, of an ester bond is replaced with a sulfonyl part, i.e. -S(=O)₂-. There are no specific limitations on the number of carbon atoms in a sulfonic acid ester group, but it is preferably in the range of 1 or more and 20 or less.

A sulfonamide group refers to a functional group in which, for example, an alkyl group, cycloalkyl group, aryl group, heteroaryl group, or the like is bonded through a sulfonamide bond, and it may or may not contain a substituent group. Here, a sulfonic acid ester bond refers to a structure in which the carbonyl part, i.e. -C(=O)-, of an amide bond is replaced with a sulfonyl part, i.e. -S(=O)₂-. There are no specific limitations on the number of carbon atoms present in a sulfonamide group, but it is preferably in the range of 1 or more and 20 or less.

An amino group may or may not contain a substituent group. There are no specific limitations on the number of carbon atoms present in an amino group, but it is preferably 2 or more and 50 or less, more preferably 6 or more and 40 or less, and particularly preferably 6 or more and 30 or less.

A silyl group refers to a functional group that contains a bond through a substituted or unsubstituted silicon atom, and examples thereof include alkylsilyl groups such as trimethylsilyl group, triethylsilyl group, tert-butyldimethylsilyl group, propyldimethylsilyl group, and vinyldimethylsilyl group, as well as arylsilyl groups such as phenyldimethylsilyl group, tert-butyldiphenylsilyl group, triphenylsilyl group, and trinaphthylsilyl group. A silyl group may or may not contain a substituent group. There are no specific limitations on the number of carbon atoms in a silyl group, but it is preferably in the range of 1 or more and 30 or less.

A siloxanyl group refers to a silicon compound group that contains a bond through an ether bond, such as trimethylsiloxanyl group. The siloxanyl group may or may not contain a substituent group.

A boryl group may or may not contain a substituent group.

Examples of compounds as represented by the general formula (7) are shown below.

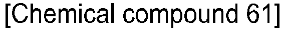

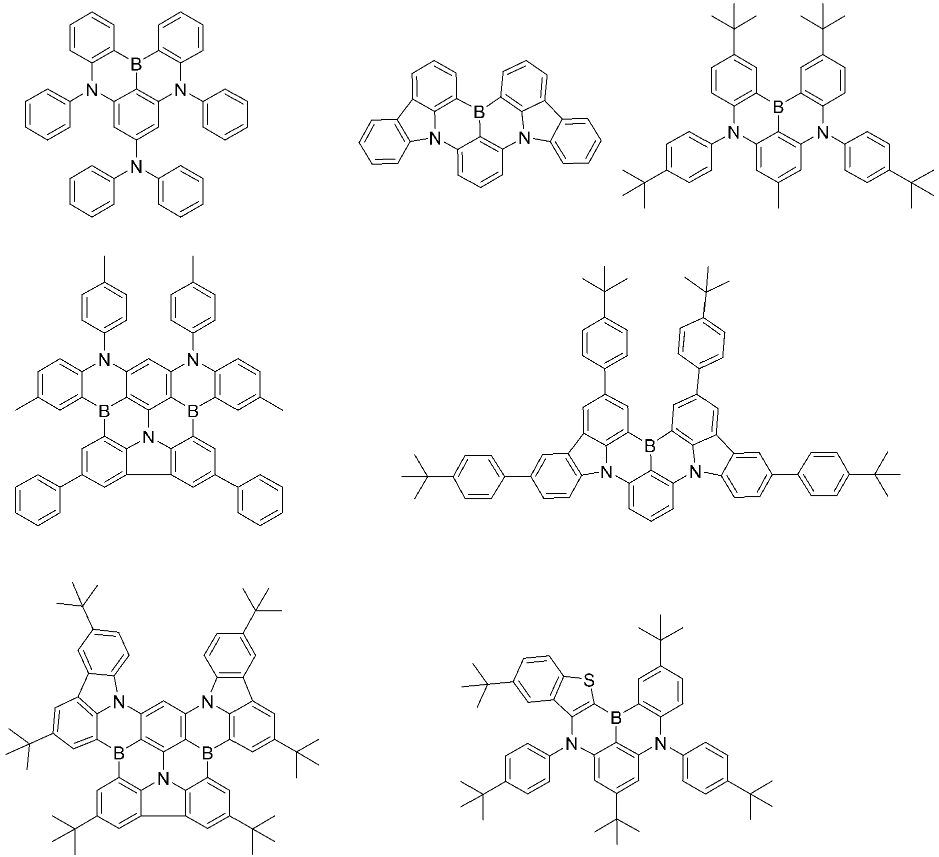

For the organic EL element according to an embodiment of the present invention, it is also preferable that the light emitting layer contain a triplet emission material.

In the case of a light emitting layer designed for triplet light emission (phosphorescene emission), the dopant material to use is preferably a metal complex compound containing at least one metal selected from the group consisting of iridium (Ir), ruthenium (Ru), palladium (Pd), platinum (Pt), osmium (Os), and rhenium (Re). It is preferable that the ligand incorporated in the metal complex compound contain a nitrogen-containing aromatic heterocyclic ring such as phenyl pyridine skeleton, phenyl quinoline skeleton, and carbene skeleton. However, the material to use is not limited thereto and an appropriate complex is selected based on the relations among the required luminescent color, element performance, and host material. Specific examples include tris-(2-phenylpyridyl) iridium complex, tris-{2-(2-thiophenyl)pyridyl} iridium complex, tris-{2-(2-benzothiophenyl)pyridyl} iridium complex, tris-(2-phenylbenzothiazole) iridium complex, tris-(2-phenylbenzoxazole) iridium complex, tris-benzoquinoline iridium complex, bis(2-phenylpyridyl)(acetylacetonate) iridium complex, bis{2-(2-thiophenyl)pyridyl} iridium complex, bis{2-(2-benzothiophenyl)pyridyl}(acetylacetonate) iridium complex, bis(2-phenylbenzothiazole)(acetylacetonate) iridium complex, bis(2-phenylbenzoxazole)(acetylacetonate) iridium complex, bisbenzoquinoline(acetylacetonate) iridium complex, bis{2-(2,4-difluorophenyl)pyridyl}(acetylacetonate) iridium complex, tetraethylporphyrin platinum complex, {tris-(thenoyltrifluoroacetone) mono(1,10-phenanthroline)} europium complex, {tris-(thenoyltrifluoroacetone) mono(4,7-diphenyl-1,10-phenanthroline)} europium complex, {tris-(1,3-diphenyl-1,3-propanedione) mono(1,10-phenanthroline)} europium complex, and tris-acetylacetone terbium complex. In addition, phosphorescene dopant materials as disclosed in Japanese Unexamined Patent Publication (Kokai) No. 2009-130141 are also used favorably. The use of an iridium complex or a platinum complex is preferable because they can serve to further enhance the luminance efficiency.

The aforementioned triplet emission materials used as dopant materials may be contained singly in the light emitting layer or used as a mixture of two or more thereof. When two or more triplet emission materials are used in combination, it is preferable that the total weight of the dopant materials account for 30 wt% or less, more preferably 20 wt% or less, relative to the amount of the host material.

For triplet emission type layers, there are no specific limitations on the host material and dopant material to use, but preferable ones include the following.

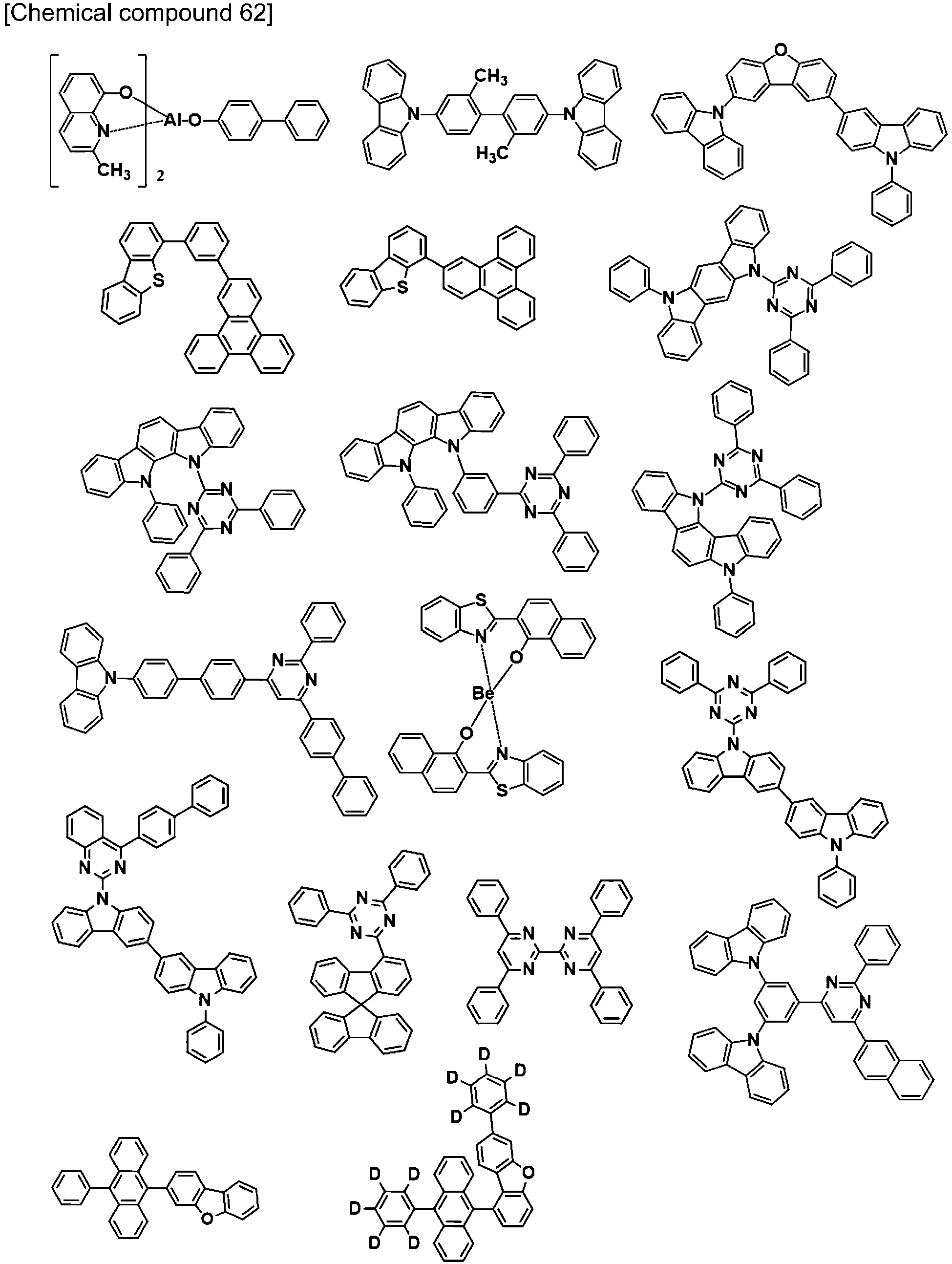

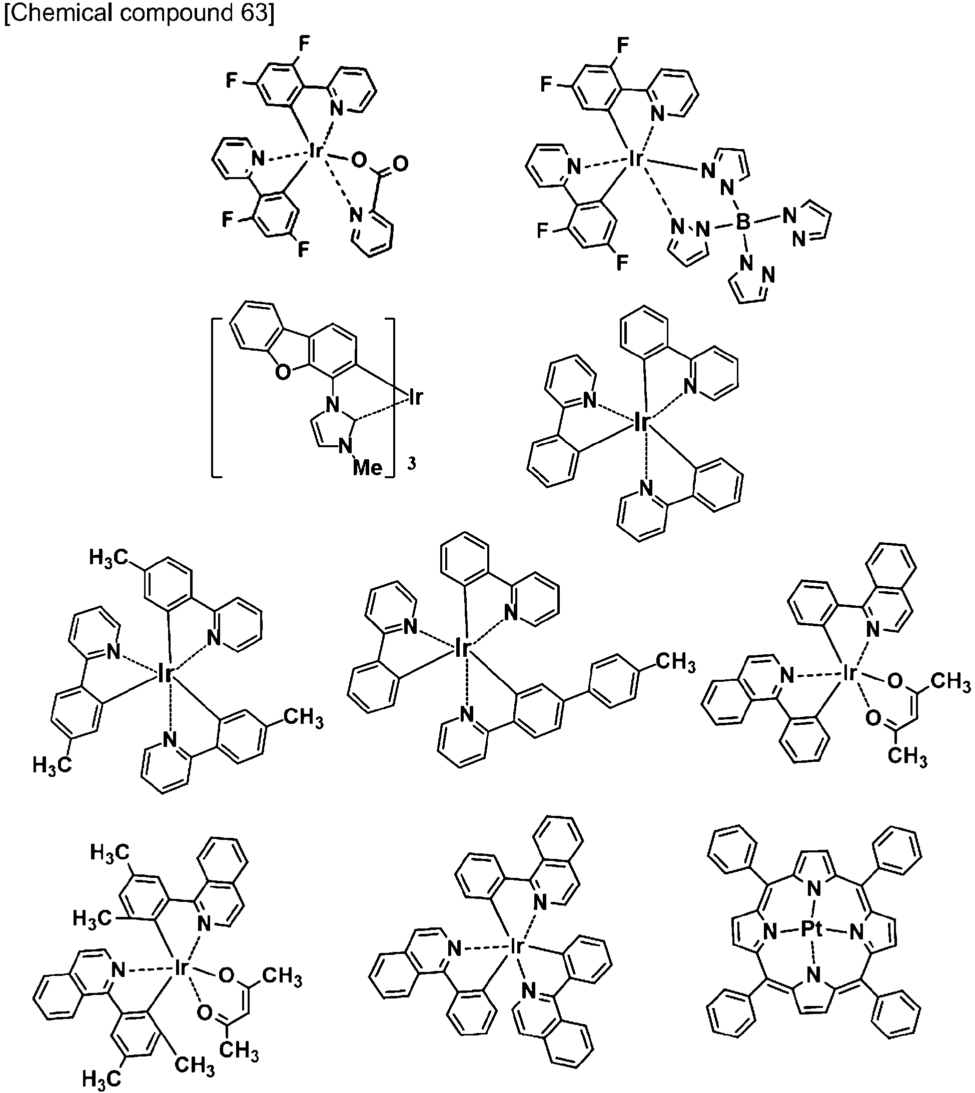

It is also preferable that the light emitting layer contain a thermally activated delayed fluorescence material. The thermally activated delayed fluorescence material is described in "Saisentan-no Yuki EL (Latest Organic EL)" (edited by Chihaya Adachi and Hiroshi Fujimoto, published by CMC Publishing) p.p. 87-103. In the above document, as the energy level of the excited singlet state and that of the excited triplet state in a fluorescent material are brought closer to each other, reverse energy transfer from the triplet excited state to the excited singlet state, which normally has a lower transition probability, can occur efficiently, thereby manifesting thermally activated delayed fluorescence (TADF). The generation mechanism of delayed fluorescence is also explained in Figure 5 of that document. The occurrence of delayed fluorescence can be confirmed by transient PL (photoluminescence) observation.

In general, thermally activated delayed fluorescence materials are also referred to as TADF materials. A thermally activated delayed fluorescence material to use here may be a single material that exhibits thermally activated delayed fluorescence or a combination of two or more materials that exhibits thermally activated delayed fluorescence. In the case of using a combination of two or more materials, those materials may be in the form of a mixture or a stack of layers containing different materials. The materials to use may be generally known thermally activated delayed fluorescence ones. Examples thereof include, but not limited to, benzonitrile derivatives, triazine derivatives, disulfoxide derivatives, carbazole derivatives, indolocarbazole derivatives, dihydrophenazine derivatives, thiazole derivatives, and oxadiazole derivatives.

In the case of an element that contains a TADF material in its light emitting layer, it is preferable that a fluorescent dopant material be also present in the light emitting layer. This is because higher luminance efficiency and longer service life can be achieved as the triplet excitons are converted into singlet excitons by the TADF material while the fluorescent dopant material receives the singlet excitons.

### (Electron transporting layer)

For the present invention, the electron transporting layer is a layer that works to receive electrons injected from the cathode and then transport those electrons. The electron transporting layer is required to ensure a high electron injection efficiency and transport the injected electrons efficiently. Accordingly, it is preferable that the material that forms the electron transporting layer be of a substance that is high in electron affinity, high in electron mobility, high in stability, and little susceptible to generation of impurities that work as traps during production or use. In the case where it has a thick laminated structure, it is preferable that compounds with molecular weights of 380 or more be used to maintain stable film quality because low molecular weight compounds tend to suffer degradation, for example, as a result of crystallization. In regard to the balance between transport of holes and transport of electrons, however, if the electron transporting layer mainly plays the role of efficiently preventing the holes coming from the anode from flowing to the cathode without undergoing recombination, it can serve, even when it is formed of a material that is not significantly high in electron transport ability, to achieve a luminance efficiency that is as high as achievable by a layer formed of a material with a high electron transport ability. Accordingly, a hole blocking layer that works efficiently to block the movement of holes can be regarded as being equivalent to the electron transporting layer according to the present invention. The hole blocking layer and the electron transporting layer may each be in the form of a monolayer or a stack of a plurality of layers.

The electron transporting layer to use may be a generally known electron transporting material. Examples thereof include condensed polycyclic aromatic derivatives, styryl based aromatic ring derivatives, quinone derivatives, phosphorus oxide derivatives, quinolinol complexes, benzquinolinol complexes, hydroxyazole complexes, azomethine complexes, tropolone metal complexes, flavonol metal complexes, and other various metal complexes. From the perspective of further lowering the driving voltage and achieving more efficient light emission, it is preferable to use a compound that includes an element selected from the group consisting of carbon, hydrogen, nitrogen, oxygen, silicon, and phosphorus and has a heteroaryl ring structure containing an electron-accepting nitrogen atom.

An electron-accepting nitrogen atom as referred to herein is a nitrogen atom that is bonded with an adjacent atom through a multiple bond. Such a nitrogen atom is so high in electronegativity that the multiple bond shows electron-accepting property. Thus, an aromatic heterocyclic ring containing an electron-accepting nitrogen atom has a high electron affinity. An electron transporting material containing an electron-accepting nitrogen atom makes it easy to receive electrons from a cathode with a high electron affinity and acts to enable driving at a low voltage. In addition, an increased number of electrons will be supplied to the light emitting layer and the recombination probability will increase, leading to an increase in the luminance efficiency.

Heteroaryl rings that contain electron-accepting nitrogen atoms include, for example, triazine ring, pyridine ring, pyrazine ring, pyrimidine ring, quinoline ring, quinoxaline ring, quinazoline ring, naphthyridine ring, pyrimidopyrimidine ring, benzoquinoline ring, phenanthroline ring, imidazole ring, oxazole ring, oxadiazole ring, triazole ring, thiazole ring, thiadiazole ring, benzoxazole ring, benzothiazole ring, benzimidazole ring, and phenanthroimidazole ring.

Compounds that have any of these heteroaryl ring structures include, for example, pyridine derivatives, triazine derivatives, quinazoline derivatives, pyrimidine derivatives, benzimidazole derivatives, benzoxazole derivatives, benzthiazole derivatives, oxadiazole derivatives, thiadiazole derivatives, triazole derivatives, pyrazine derivatives, phenanthroline derivatives, quinoxaline derivatives, quinoline derivatives, benzoquinoline derivatives, oligopyridine derivatives, quinoxaline derivatives, and naphthyridine derivatives. In particular, from the perspective of electron transport ability, preferable ones include imidazole derivatives, oxadiazole derivatives, triazole derivatives, triazine derivatives, pyrimidine derivatives, phenanthroline derivatives, benzoquinoline derivatives, bipyridine derivatives, terpyridine derivatives, and naphthyridine derivatives.

It is also preferable for these derivatives to have fused polycyclic aromatic skeletons because it worsks to raise the glass transition temperature and increase the electron mobility, thereby serving to produce an organic EL element with a lower driving voltage. Furthermore, in view of improvement in service life of the element as well as easiness of synthesis and availability of materials, it is more preferable that the fused polycyclic aromatic backbone be a fluoranthene skeleton, anthracene skeleton, pyrene skeleton, or phenanthroline skeleton.

There are no specific limitations on the preferable electron transporting material to use, but specific examples include the following.

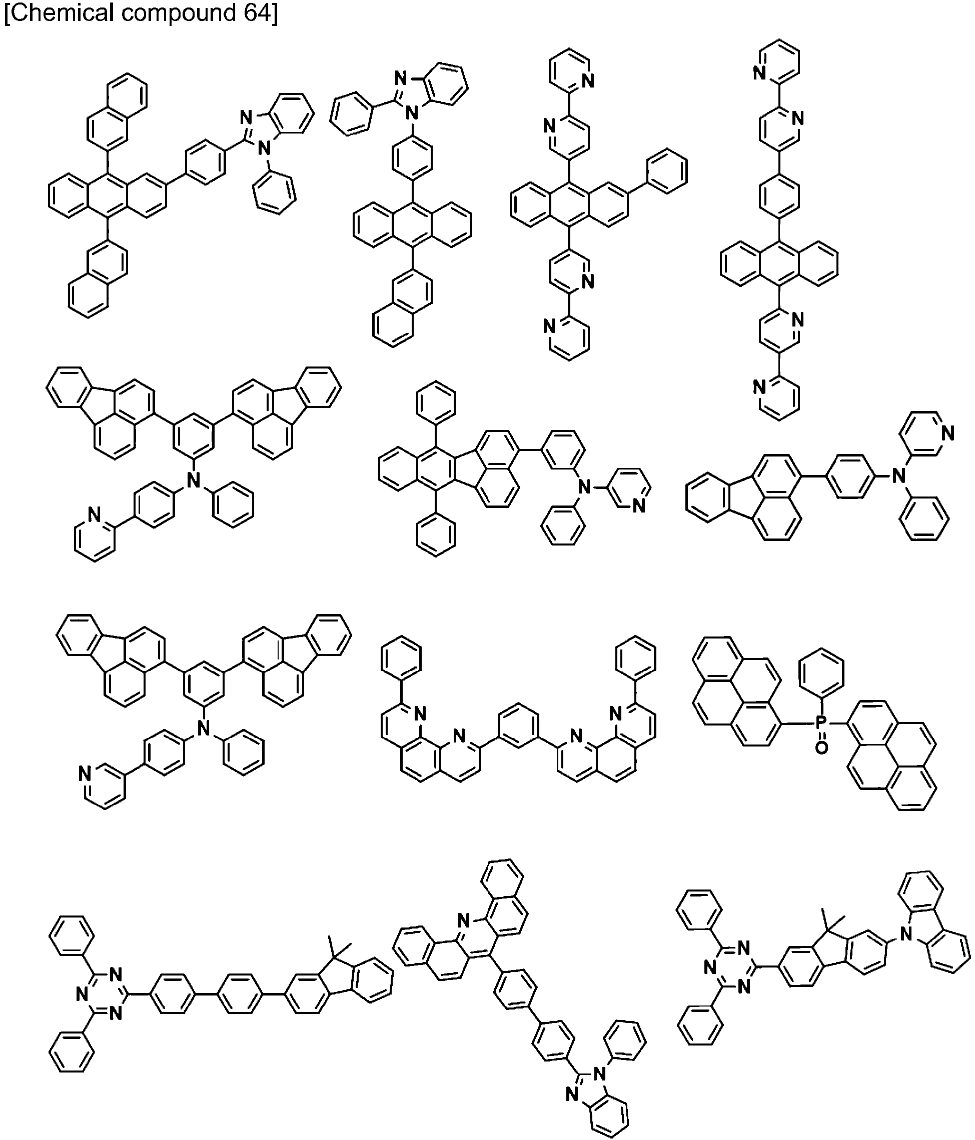

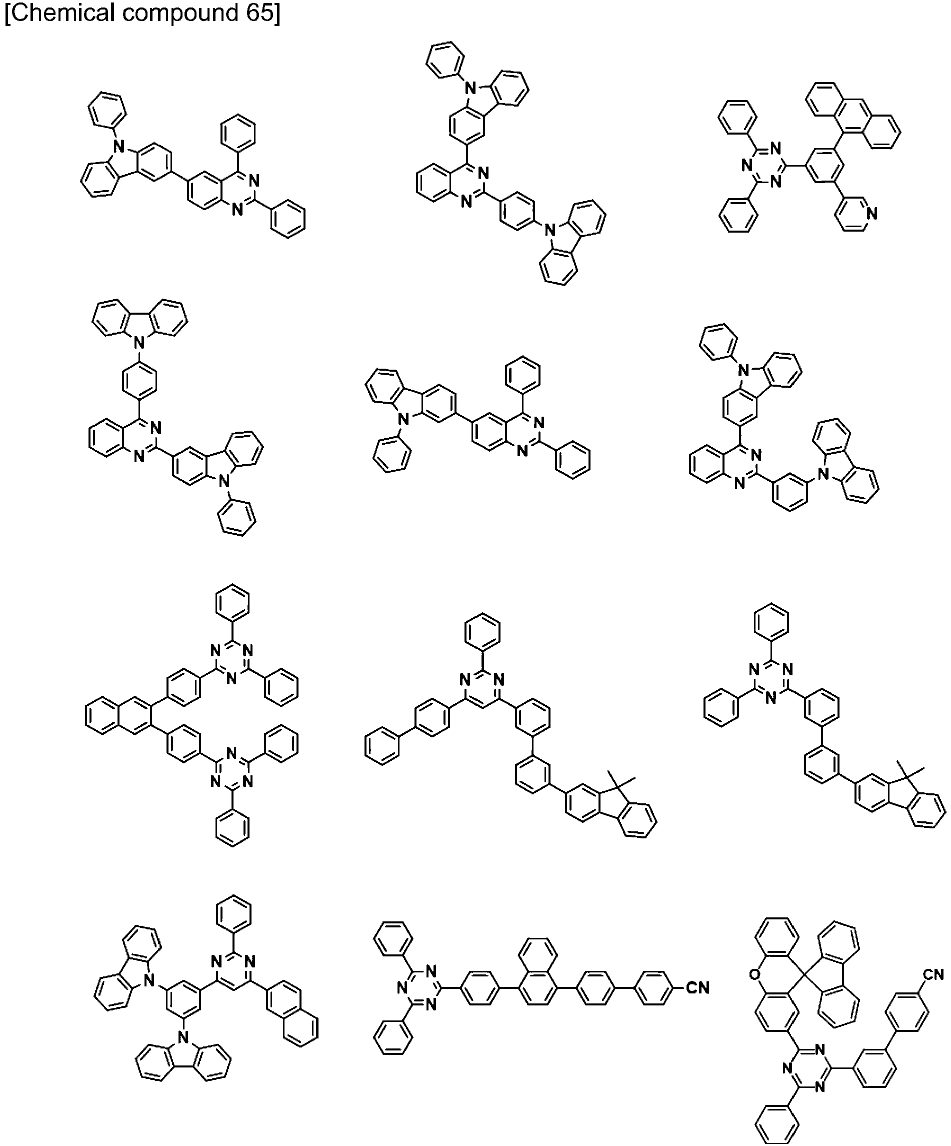

Furthermore, compounds as represented by the general formula (1) are also preferable because they exhibit high electron transport ability and provide electron transporting layers with excellent properties.

The electron transporting materials listed above may be used singly or as mixtures of two or more of the electron transporting materials listed above, and it may also be good to use a mixture of one or more of other electron transporting materials with an electron transporting material as listed above.

The electron transporting layer may also contain a donor type material. A donor type material as referred to herein is a compound that can reduce the electron injection barrier to facilitate the electron injection from the cathode or electron injection layer into the electron transporting layer and, in addition, serves to increase the electric conductivity of the electron transporting layer.

From the perspective of low work function and improved electron transport ability, the donor type material preferably contains an alkali metal atom, alkaline earth metal atom, or rare earth metal atom. In particular, it is more preferable to contain an alkali metal atom or a rare earth metal atom because they can achieve a further decrease in the driving voltage of an organic EL device.

### (Electron injection layer)

For the present invention, an electron injection layer may be present between the cathode and the electron transporting layer. In general, an electron injection layer is formed for the purpose of assisting the injection of electrons from the cathode into the electron transporting layer. When such a layer is provided, it may include a compound having a heteroaryl ring structure containing an electron-accepting nitrogen atom or it may be a layer containing a donor type material as described above.

An inorganic material such as insulator or semiconductor may also be present in the electron injection layer. When an inorganic material such as insulator or semiconductor is added in the electron injection layer, it may be selected from generally known materials. The addition of such a material serves to suppress short-circuiting in the organic EL element and improve the electron injection ability.

As such an insulator, it is preferable to use at least one metal compound selected from the group consisting of alkali metal chalcogenides, alkaline earth metal chalcogenides, alkali metal halides, and alkaline earth metal halides.

In addition, complexes of organic substances and metals can also be suitably used. If a complex of an organic substance and a metal is added in the electron injection layer, it serves to facilitate the adjustment of the film thickness. Preferable examples of useful organic substances to form such organometallic complexes include quinolinol, benzoquinolinol, pyridyl phenol, flavonol, hydroxyimidazopyridine, hydroxybenzazole, and hydroxytriazole.

Furthermore, the use of a layer that contains a compound as represented by the general formula (1) is also preferable because such a layer exhibits high electron injection ability and works as an electron injection layer with excellent properties. Furthermore, it is preferable that the electron injection layer contain both a compound as represented by the general formula (1) and either an alkali metal atom or a rare earth metal atom. In such a case, it is possible to further reduce the driving voltage and improve the service life.

### (Charge generation layer)

In general, a charge generation layer used for the present invention has a double layer structure, and specifically, it is preferable to use a pn-junction type charge generation layer that includes an n-type charge generation layer and a p-type charge generation layer. When voltage is applied to an organic EL element, the pn-junction type charge generation layer functions to generate charges or to separate charges into holes and electrons, and then works to inject these holes and electrons into the light emitting layer through the hole transporting layer and the electron transporting layer, respectively. In regard to specific functions of layers, in the case of an organic EL element that includes a stack of two or more light emitting layers, charge generation layers are inserted between these multiple light emitting layers to perform the role of generating charges. The n-type charge generation layer supplies electrons to the first light emitting layer positioned near the anode while the p-type charge generation layer supplies holes to the second light emitting layer positioned near the cathode. Thus, they can work to improve the luminance efficiency of an organic EL element that contains a stack of multiple light emitting layers, reduce the driving voltage, and increase the service life of the element.

The n-type charge generation layer includes an n-type dopant material and a host material, and conventional materials may be used as them. For example, alkali metals, alkaline earth metals, or rare earth metals can be used as the n-type dopant material. As host materials, on the other hand, compounds having nitrogen-containing aromatic heterocycles such as phenanthroline derivatives and oligopyridine derivatives can be used. In particular, compounds as represented by the general formula (1) and phenanthroline dimers are preferable because they exhibit excellent properties as host materials for the n-type charge generation layer.

As a configuration of the charge generation layer, it is preferable to further contain a phenanthroline derivative in addition to a compound as represented by the general formula (1). Examples of the phenanthroline derivative include the compounds given below.

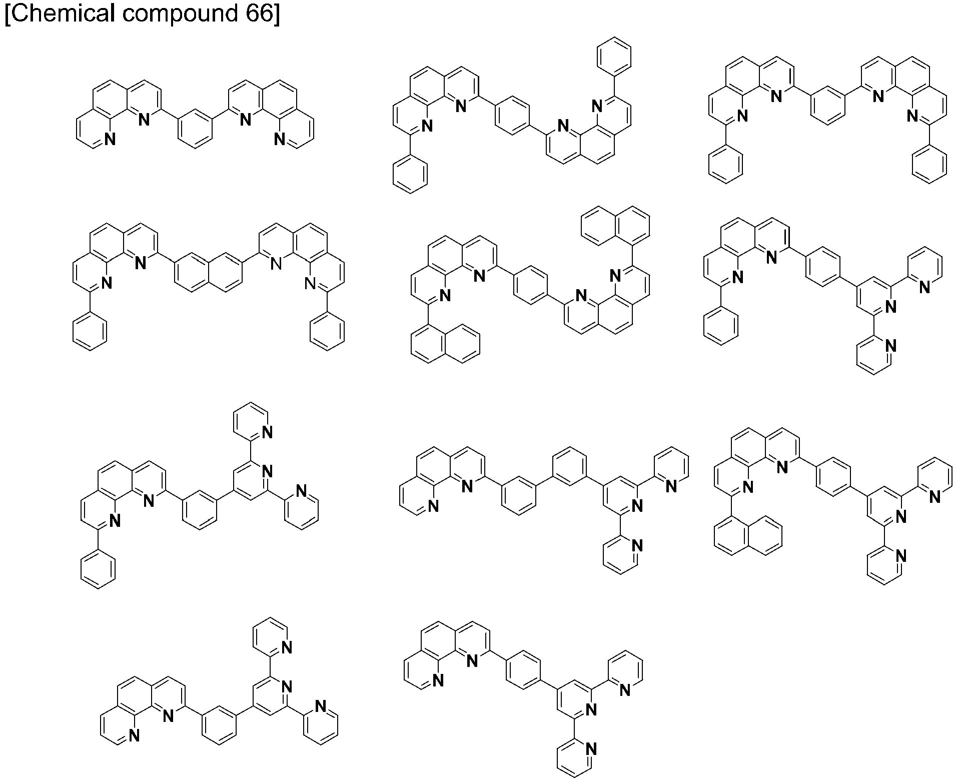

As a configuration of the charge generation layer, it is preferable to further contain an alkali metal atom, a copper group element atom, or a rare earth metal atom in addition to a compound as represented by the general formula (1). It is particularly preferable that the alkali metal atom be Li. It is particularly preferable that the copper group element atom be Ag. It is particularly preferable that the rare earth metal atom be Yb.

As a configuration of the charge generation layer, it is also preferable to contain a phenanthroline derivative, as well as an alkali metal atom, copper group atom, or rare earth metal atom, in addition to a compound as represented by the general formula (1).

The p-type charge generation layer includes a p-type dopant material and a host material, and conventional materials may be used as them. For example, useful p-type dopant materials include tetrafluoro-7,7,8,8-tetracyanoquinodimethane (F4-TCNQ), tetracyanoquinodimethane derivatives, radialene derivatives, iodine, FeCl₃, FeF₃, and SbCl₅. It is preferable to use a radialene derivative as a p-type dopant material. It is preferable to use an arylamine derivative as a host material.

There are no specific limitations on the technique to use for producing each of the layers that constitute an organic EL element and useful ones include resistance heating evaporation, electron beam evaporation, sputtering, molecular lamination, and coating, of which resistance heating evaporation and electron beam evaporation are commonly preferable from the perspective of element characteristics.

There are no specific limitations on the total thickness of the organic layers located between the anode and the cathode, but it is preferably in the range of 1 to 1,000 nm, although the optimum value depends on the resistance of the light emitting substance. The thicknesses of the light emitting layer, electron transporting layer, and hole transporting layer are each preferably 1 nm or more and 200 nm or less, and more preferably 5 nm or more and 100 nm or less.

The organic EL element according to an embodiment of the present invention has a function of converting electrical energy into light. Here, as the electrical energy, a direct current is commonly used, but it is also possible to use a pulse current or alternating current. There are no specific limitations on the electric current and voltage, but in view of the electric power consumption and life of the element, they should be such that a maximum luminance is realized with minimum required energy.

The organic EL element according to an embodiment of the present invention is suitably used, for example, in display devices such as matrix and/or segment type displays.

The organic EL element according to an embodiment of the present invention can also be used favorably in backlight components in various instruments. Backlight components are intended mainly to improve the visibility of display devices such as display components that do not emit light spontaneously and they are incorporated in instruments such as liquid crystal display devices, clocks, audio instruments, automobile panels, display boards, and signs. In particular, the organic EL element according to the present invention can be used favorably for backlight components of liquid crystal display devices, particularly for backlight components of personal computers, for which active studies are now performed for developing thin panels, thus serving to provide backlight components that are thin and lightweight compared to conventional ones.

The organic EL element according to an embodiment of the present invention can also be used favorably in various lighting instruments. The organic EL element according to an embodiment of the present invention can achieve both high luminance efficiency and high color purity, and furthermore, it can provide thin and lightweight devices. Accordingly, it serves to producing lighting instruments that combines low power consumption, vivid emission color, and excellent designability.

The organic EL element according to an embodiment of the present invention can also be used favorably as a photosensitizing device. A photosensitizing device is used as a device for photodynamic therapy, which utilizes the modification of a target substance in biological tissue caused by reactive oxygen species generated from a specific photosensitizing agent. An organic EL element can work to cause efficient light emission from electrodes implanted in a living body, thus enabling efficient generation of reactive oxygen species even in deep parts of a living body.

### EXAMPLES

The present invention will be illustrated below in greater detail with reference to examples, but it should be understood that the invention is not construed as being limited to these examples.

### Synthesis example 1: synthesis of compound 1

A compound 1 was synthesized according to the synthesis procedure described below.

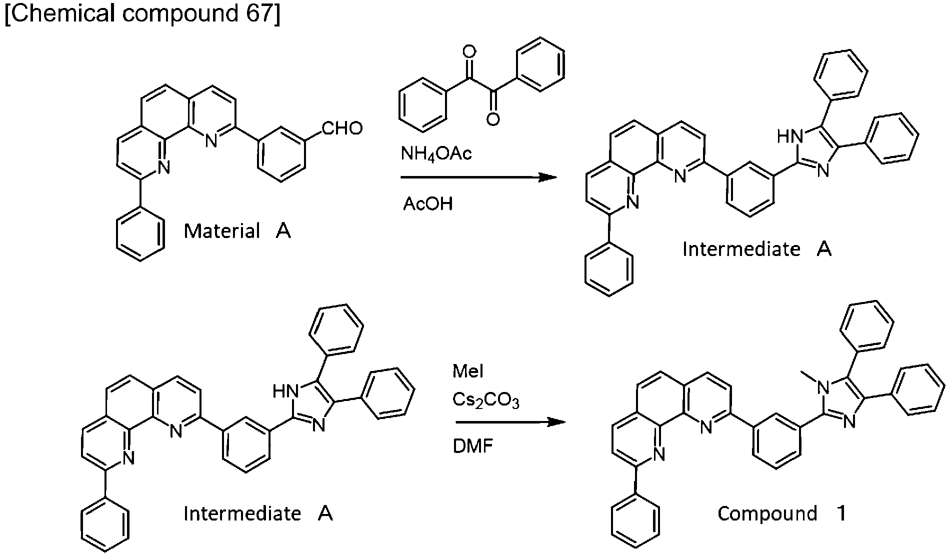

A mixed solution containing 6.0 g of a raw material A, 3.5 g of benzyl, 25.7 g of ammonium acetate, and 100 ml of acetic acid was heated while stirring at 80°C for 2 hours in a nitrogen flow. After cooling it to room temperature, water was added and filtration was performed, followed by washing with methanol and vacuum drying to provide 8.6 g of an intermediate A.

Then, a mixed solution containing 6.0 g of the intermediate A, 8.9 g of cesium carbonate, 2.3 g of iodomethane, and 110 ml of DMF was heated while stirring at 80°C for 2 hours in a nitrogen flow. After cooling it to room temperature, water was added and filtration was performed, followed by washing with methanol. Recrystallization was performed in pyridine and vacuum drying was conducted to provide 5.1 g of a compound 1.

The compound 1 thus obtained was subjected to sublimation purification using an oil diffusion pump at about 330°C under a pressure of 1 × 10⁻³ Pa. The HPLC purity (area% at measuring wavelength of 254 nm) of the compound 1 was 99.9% both before and after the sublimation purification.

After the sublimation purification, the structure of the compound 1 was identified by mass spectrometry (MS) analysis and ¹H-NMR analysis. Results are shown below.

MS (m/z): 565 [M+H]⁺
¹H-NMR (400 MHz, CDCl₃) δ: 9.09 (s, 1H), 8.54-8.65 (m, 4H), 8.45-8.48 (m, 2H), 8.40-8.42 (m, 1H), 8.00-8.05 (m, 3H), 7.81-7.85 (m, 1H), 7.48-7.63 (m, 7H), 7.40-7.45 (m, 3H), 7.15-7.27 (m, 3H), 3.70 (s, 3H).

### Synthesis example 2: synthesis of compound 9

A compound 9 was synthesized according to the synthesis procedure described below.

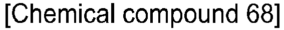

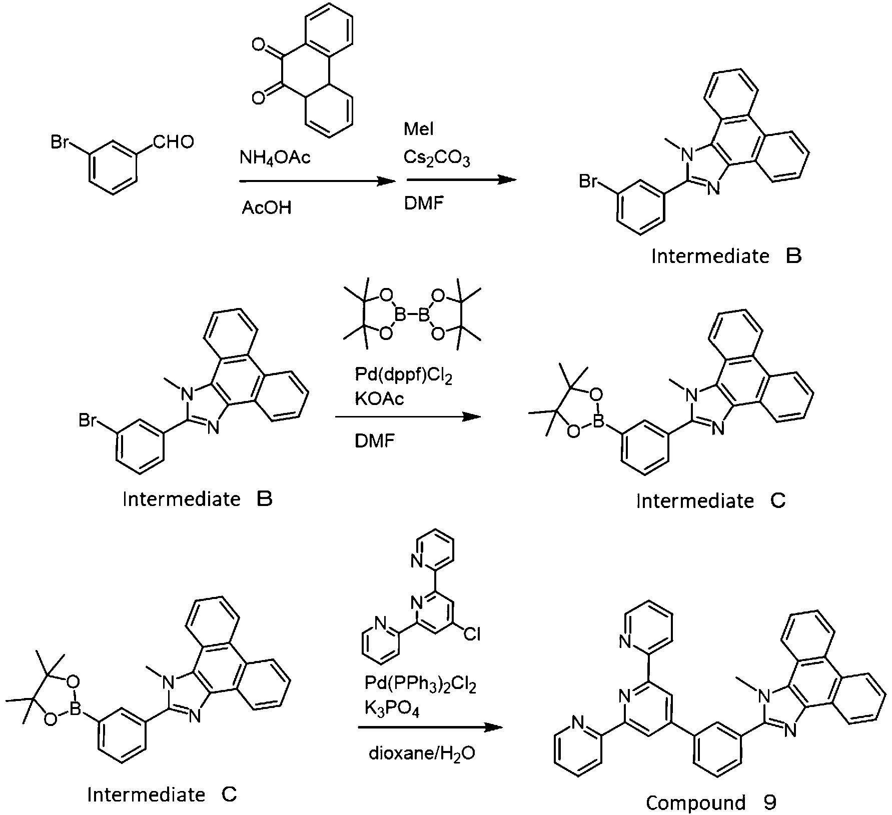

A mixed solution containing 3.7 g of 3-bromobenzaldehyde, 4.2 g of 9,10-phenanthrenequinone, 31.8 g of ammonium acetate, and 100 ml of acetic acid was heated while stirring at 80°C for 2 hours in a nitrogen flow. After cooling it to room temperature, water was added and filtration was performed, followed by washing with methanol and vacuum drying. To the resulting solid material, a mixed solution containing 8.9 g of cesium carbonate, 2.3 g of iodomethane, and 110 ml of DMF was added and heated while stirring at 80°C for 2 hours in a nitrogen flow. After cooling it to room temperature, water was added and filtration was performed, followed by vacuum drying to provide 6.9 g of an intermediate B.

Subsequently, a mixed solution of 6.9 g of the intermediate B, 6.3 g of bis(pinacolato)diboron, 5.2 g of potassium acetate, 260 mg of bis(diphenylphosphino)ferrocene dichloropalladium, and 120 ml of dimethyl formamide was heated while stirring at 100°C for 3 hours in a nitrogen flow. After cooling it to room temperature, water was added and filtration was performed, followed by washing with methanol and vacuum drying to provide 16.8 g of an intermediate

C.

Subsequently, a mixed solution of 4.4 g of the intermediate C, 2.6 g of 4'-chloro-2,2':6',2"-terpyridine, 4.2 g of tripotassium phosphate, 240 mg of dichlorobis(triphenylphosphine palladium)dichloride, 50 ml of dioxane, and 10 ml of water was heated under reflux for 8 hours in a nitrogen flow. After cooling it to room temperature, water was added and filtration was performed, followed by washing with methanol and vacuum drying. The catalyst was removed with activated carbon from the resulting solid material, and the solvent was removed by evaporation. The resulting solid material was recrystallized in pyridine and vacuum-dried to provide 4.0 g of a compound 9.

The compound 9 thus obtained was subjected to sublimation purification using an oil diffusion pump at about 340°C under a pressure of 1 × 10⁻³ Pa. The HPLC purity (area% at measuring wavelength of 254 nm) of the compound 9 was 99.9% both before and after the sublimation purification.

After the sublimation purification, the structure of the compound 9 was identified by mass spectrometry (MS) analysis and ¹H-NMR analysis. Results are shown below.

MS (m/z): 540 [M+H]⁺
¹H-NMR (400 MHz, CDCl₃) δ: 8.98-9.02 (m, 1H), 8.85-8.91 (m, 3H), 8.78-8.80 (m, 2H), 8.71-8.74 (m, 2H), 8.64-8.68 (m, 2H), 8.38-8.40 (m, 1H), 8.15-8.18 (m, 1H), 8.05-8.09 (m, 3H), 7.85-7.89 (m, 1H), 7.65-7.80 (m, 4H), 7.55-7.58 (m, 2H), 4.38 (s, 3H).

### Synthesis example 3: synthesis of compound 10

An intermediate C was synthesized in the same way as in Synthesis example 2, and then a compound 10 was synthesized according to the synthesis procedure described below.

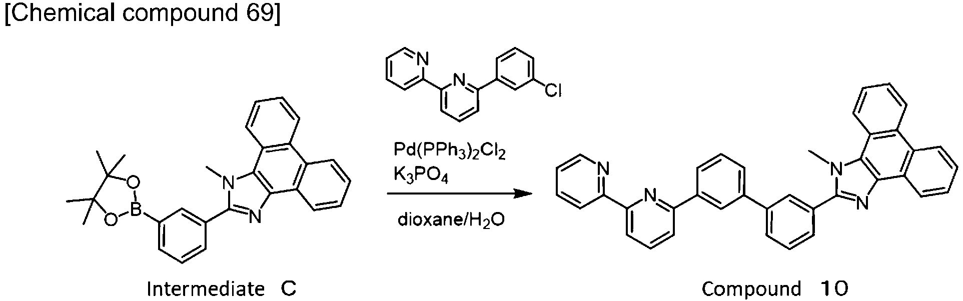

A mixed solution containing 3.2 g of the intermediate C, 5.6 g of 4'-chloro-2,2':6',2"-terpyridine, 5.0 g of tripotassium phosphate, 150 mg of dichlorobis(triphenylphosphine palladium)dichloride, 60 ml of dioxane, and 10 ml of water was heated under reflux for 8 hours in a nitrogen flow. After cooling to room temperature, water was added and filtration was performed, followed by washing with methanol and vacuum drying. The catalyst was removed with activated carbon from the resulting solid material, and the solvent was removed by evaporation. The resulting solid material was recrystallized in pyridine and vacuum-dried to provide 2.8 g of a compound 10.

The compound 10 thus obtained was subjected to sublimation purification using an oil diffusion pump at about 340°C under a pressure of 1 × 10⁻³ Pa. The HPLC purity (area% at measuring wavelength of 254 nm) of the compound 10 was 99.9% both before and after the sublimation purification.

After the sublimation purification, the structure of the compound 10 was identified by mass spectrometry (MS) analysis and ¹H-NMR analysis. Results are shown below.

MS (m/z): 539 [M+H]⁺
¹H-NMR (400 MHz, CDCl₃) δ: 8.98-9.02 (m, 1H), 8.86-8.91 (m, 1H), 8.72-8.75 (m, 1H), 8.58-8.65 (m, 4H), 8.38-8.41 (m, 1H), 8.28-8.34 (m, 2H), 8.23-8.26 (m, 1H), 7.98-8.09 (m, 3H), 7.92-7.95 (m, 1H), 7.65-7.80 (m, 5H), 7.48-7.52 (m, 1H), 7.14-7.25 (m, 2H), 4.40 (s, 3H).

Next, the evaluation procedures used in the examples given below are described. It should be noted that only one measurement is taken (n = 1) unless otherwise specified.

### (Driving voltage)

### - For single charge elements

The single charge elements obtained in Examples 1 to 25 and Comparative examples 1 to 12 were each driven by direct current at 10 mA/cm², and the initial driving voltage was measured. Then, the voltage was measured while driving each of them with a current density of 10 mA/cm² for 100 hours in an environment at 70°C, and the increase in voltage from the initial driving voltage was calculated.

### - For organic EL elements

The organic EL elements obtained in Examples 26 to 75 and Comparative examples 13 to 36 were each activated at a luminance of 1,000 cd/m², and the initial driving voltage was measured. Then, the voltage was measured while driving each of them with a current density of 10 mA/cm² for 100 hours in a room temperature environment, and the increase in voltage from the initial driving voltage was calculated.

### - Evaluation of measured values

A smaller initial driving voltage suggests that the device can be driven at a lower voltage and accordingly is superior in terms of luminance efficiency (luminance/power). Furthermore, a smaller increase in voltage suggests that the device is superior in terms of service life.

### (External quantum efficiency)

The external quantum efficiency of each of the organic EL elements obtained in Examples 26 to 75 and Comparative examples 13 to 36 was measured while it was activated with a current density of 10 mA/cm², and its evaluation was made in terms of luminance efficiency. A device with a higher external quantum efficiency is considered to be higher in luminance efficiency. Measurement of external quantum efficiency was performed with a spectral radiance meter (manufactured by Konica Minolta Inc.).

### (Durability)

The organic EL elements obtained in Examples 26 to 75 and Comparative examples 13 to 36 were each driven continuously at a constant current of 10 mA/cm², and the time required until the luminance decreased by 20% from the initial luminance, which was adopted to represent durability.

### Example 1

A transparent, electrically conductive ITO film with a thickness of 125 nm was evaporated on a glass substrate (manufactured by Geomatec Co., Ltd., 11 Ω/□, sputtered product) to prepare an anode, and it was then cut to a size of 38 mm × 46 mm and etched. The resulting substrate was subjected to ultrasonic cleaning for 15 minutes using SEMICOCLEAN (registered trademark) 56 (trade name, manufactured by Furuuchi Chemical Corporation) and then rinsed with ultrapure water. Immediately before subjecting it to single charge element production, this substrate was subjected to UV ozone treatment for 1 hour and placed in a vacuum evaporation instrument, which was then evacuated to an internal vacuum of 5 × 10⁻⁴ Pa or less. The compound 1 and metallic element Yb, which was used as dopant material, were evaporated to a thickness of 100 nm by resistance heating in such a manner that the evaporation rate ratio between the compound 1 and Yb was 9:1, thereby forming an organic layer with a weight ratio of 9:1 between them. Then, aluminum was evaporated to 60 nm to form a cathode, thereby providing a single charge element of a 5 × 5 mm square. The film thickness as referred to herein is the reading on the monitor of a quartz oscillator type film thickness instrument, and this is common throughout other Examples and Comparative examples. The use of a single charge element enables the evaluation of the charge generation efficiency and electron transport ability of a compound co-evaporated with a metal.

The above single charge element was examined by the evaluation methods described above, and results showed that the initial driving voltage was 0.071 V and the voltage increase in a test for driving it at 70°C for 100 hours was 0.022 V.

### Examples 2 to 25 and Comparative examples 1 to 12

Except for using the compound, metallic element, and evaporation rate ratio between the compound and the metallic element given in Table 1, the same procedure as in Example 1 was carried out to produce a single charge element. Results obtained in each Example and Comparative example are given in Table 1. Here, the compounds listed below were used as the compounds 1 to 16.

The compounds other than the compounds 1, 9, and 10 were synthesized by appropriately changing the raw materials or the like. A commercially available product was used as the compound 13.

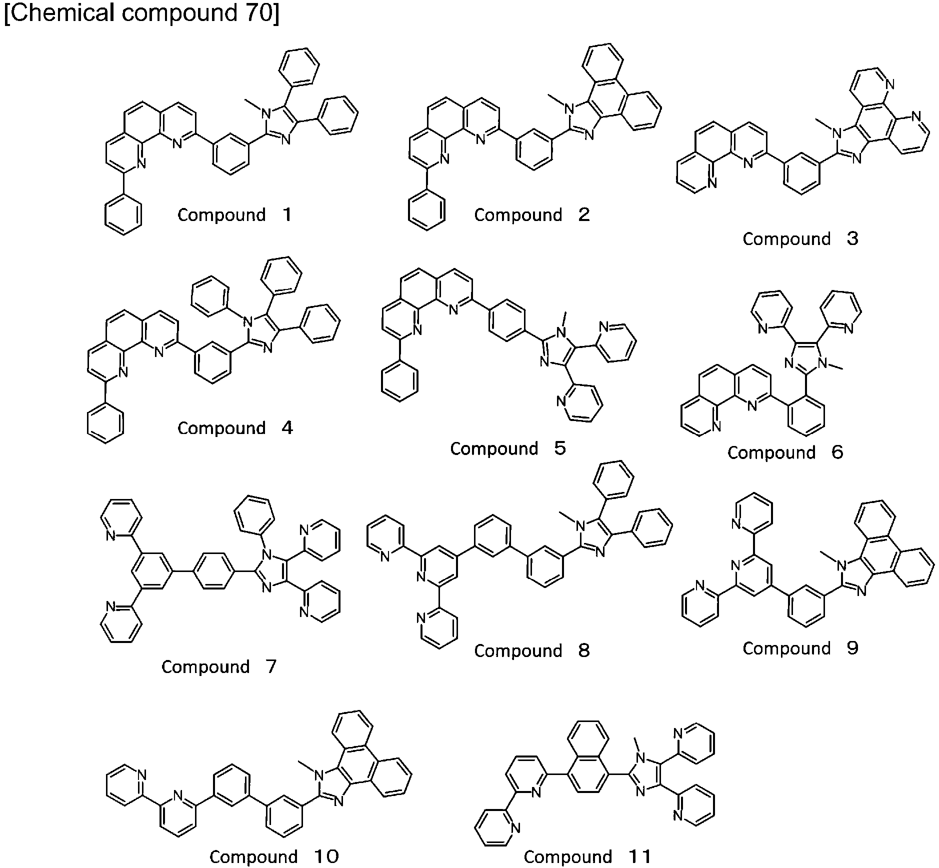

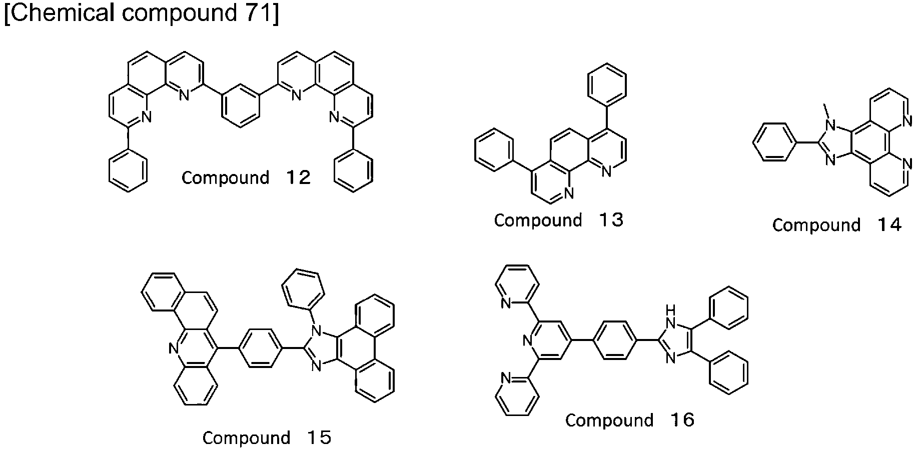

### [Table 1]

**[Table 1]**

| | compound | metallic element (dopant) | compound : metallic element evaporation rate ratio | voltage at 10 mA/cm² (V) | voltage increase during 100 hour driving at 70°C and 10 mA/cm² (V) |
|---|---|---|---|---|---|
| Example 1 | compound 1 | Yb | 9:1 | 0.071 | 0.022 |
| Example 2 | compound 2 | Yb | 9:1 | 0.068 | 0.014 |
| Example 3 | compound 3 | Yb | 9:1 | 0.074 | 0.019 |
| Example 4 | compound 4 | Yb | 9:1 | 0.084 | 0.031 |
| Example 5 | compound 5 | Yb | 9:1 | 0.077 | 0.026 |
| Example 6 | compound 6 | Yb | 9:1 | 0.092 | 0.038 |
| Example 7 | compound 7 | Yb | 9:1 | 0.103 | 0.036 |
| Example 8 | compound 8 | Yb | 9:1 | 0.068 | 0.013 |
| Example 9 | compound 9 | Yb | 9:1 | 0.064 | 0.012 |
| Example 10 | compound 10 | Yb | 9:1 | 0.070 | 0.016 |
| Example 11 | compound 11 | Yb | 9:1 | 0.089 | 0.020 |
| Example 12 | compound 9 | Yb | 99:1 | 0.088 | 0.025 |
| Example 13 | compound 9 | Yb | 19:1 | 0.073 | 0.020 |
| Example 14 | compound 9 | Yb | 7:3 | 0.081 | 0.027 |
| Example 15 | compound 1 | Li | 99:1 | 0.092 | 0.038 |
| Example 16 | compound 2 | Li | 99:1 | 0.075 | 0.024 |
| Example 17 | compound 3 | Li | 99:1 | 0.088 | 0.033 |
| Example 18 | compound 4 | Li | 99:1 | 0.091 | 0.044 |
| Example 19 | compound 5 | Li | 99:1 | 0.083 | 0.049 |
| Example 20 | compound 6 | Li | 99:1 | 0.101 | 0.050 |
| Example 21 | compound 7 | Li | 99:1 | 0.129 | 0.047 |
| Example 22 | compound 8 | Li | 99:1 | 0.077 | 0.025 |
| Example 23 | compound 9 | Li | 99:1 | 0.075 | 0.019 |
| Example 24 | compound 10 | Li | 99:1 | 0.083 | 0.021 |
| Example 25 | compound 11 | Li | 99:1 | 0.096 | 0.033 |
| Comparative example 1 | compound 12 | Yb | 9:1 | 0.177 | 0.095 |
| Comparative example 2 | compound 13 | Yb | 9:1 | 0.328 | 0.129 |
| Comparative example 3 | compound 14 | Yb | 9:1 | 0.299 | 0.161 |
| Comparative example 4 | compound 15 | Yb | 9:1 | 0.225 | 0.103 |
| Comparative example 5 | compound 16 | Yb | 9:1 | 0.319 | 0.155 |
| Comparative example 6 | compound 12 | Yb | 99:1 | 0.172 | 0.092 |
| Comparative example 7 | compound 12 | Yb | 19:1 | 0.180 | 0.099 |
| Comparative example 8 | compound 12 | Li | 99:1 | 0.192 | 0.088 |
| Comparative example 9 | compound 13 | Li | 99:1 | 0.341 | 0.123 |
| Comparative example 10 | compound 14 | Li | 99:1 | 0.372 | 0.226 |
| Comparative example 11 | compound 15 | Li | 99:1 | 0.237 | 0.133 |
| Comparative example 12 | compound 16 | Li | 99:1 | 0.296 | 0.207 |

### Example 26

A transparent, electrically conductive ITO film with a thickness of 165 nm was evaporated on a glass substrate (manufactured by Geomatec Co., Ltd., 11 Ω/□, sputtered product) to prepare an anode, and it was then cut to a size of 38 mm × 46 mm and etched. The resulting substrate was subjected to ultrasonic cleaning for 15 minutes using SEMICOCLEAN 56 (trade name, manufactured by Furuuchi Chemical Corporation) and then rinsed with ultrapure water. Immediately before subjecting it to organic EL element production, this substrate was subjected to UV ozone treatment for 1 hour and placed in a vacuum evaporation instrument, which was then evacuated to an internal vacuum of 5 × 10⁻⁴ Pa or less. Resistance heating was performed to first evaporate p-D1 to 5 nm to form a hole injection layer and then evaporate HT-1 to 50 nm to form a hole transporting layer. Subsequently, a light emitting layer was formed by evaporating a mixture layer of the host material H-1 and the dopant material D-1 in such a manner that the evaporated layer had a doping concentration of 5 wt% and a thickness of 20 nm. Then, an electron transporting layer was formed by evaporating ET-1 and 2E-1 to a thickness of 35 nm in such a manner that the evaporation rate ratio between ET-1 and 2E-1 was 1:1. Then, an electron injection layer was formed by evaporating the compound 1 and metallic element Yb, which was used as dopant material, to a thickness of 10nm in such a manner that the evaporation rate ratio between the compound 1 and Yb was 9:1. Subsequently, aluminum was evaporated to 60 nm to form a cathode, thereby providing an organic EL element of a 5 × 5 mm square.

This organic EL element was examined by the evaluation methods described above, and results showed an initial driving voltage of 4.40 V, an external quantum efficiency (luminance efficiency) of 5.39%, a service life of 1,030 hours, and a voltage increase of 0.022 V in a test for driving it for 100 hours at room temperature. It should be noted that p-D1, HT-1, H-1, D-1, ET-1, and 2E-1 denote the compounds listed below.

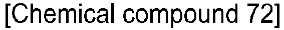

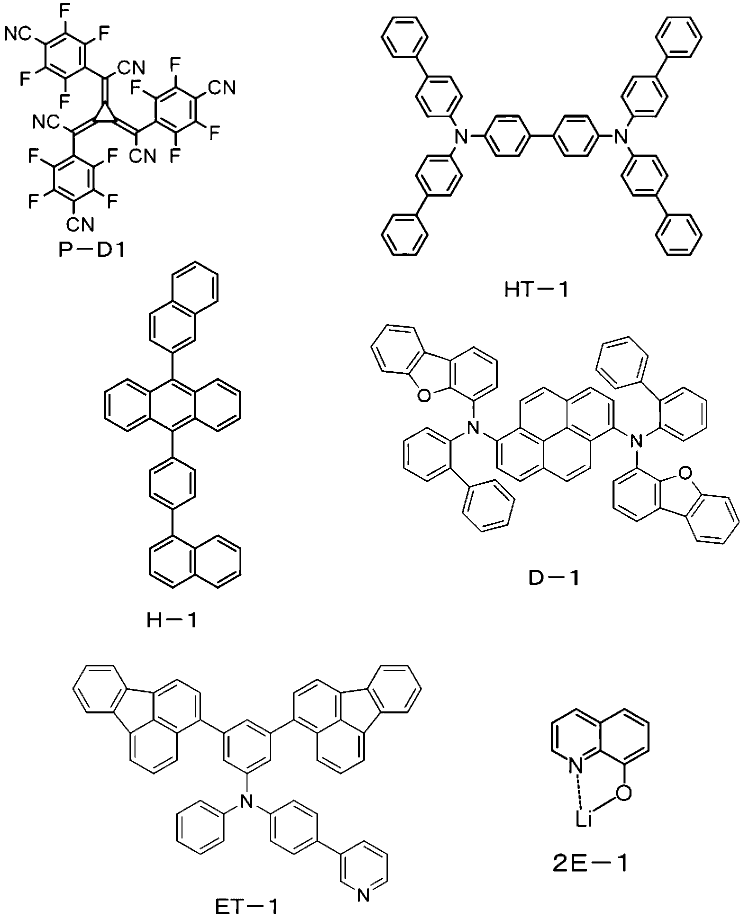

### Examples 27 to 50 and Comparative examples 13 to 24

Except for using the compound, metallic element, and evaporation rate ratio between the compound and the metallic element given in Table 2, the same procedure as in Example 26 was carried out to produce an organic EL element. Results obtained in each Example and Comparative example are given in Table 2.

### [Table 2]

**[Table 2]**

| | compound | metallic element (dopant) | compound : metallic element evaporation rate ratio | driving voltage (V) | external quantum efficiency (%) | durability (h) | voltage increase during 100 hour driving at 10 mA/cm² (V) |
|---|---|---|---|---|---|---|---|
| Example 26 | compound 1 | Yb | 9:1 | 4.40 | 5.39 | 1030 | 0.022 |
| Example 27 | compound 2 | Yb | 9:1 | 4.35 | 5.46 | 1120 | 0.017 |
| Example 28 | compound 3 | Yb | 9:1 | 4.44 | 5.28 | 1030 | 0.026 |
| Example 29 | compound 4 | Yb | 9:1 | 4.64 | 5.02 | 1020 | 0.043 |
| Example 30 | compound 5 | Yb | 9:1 | 4.51 | 5.22 | 1020 | 0.038 |
| Example 31 | compound 6 | Yb | 9:1 | 4.40 | 5.33 | 980 | 0.030 |
| Example 32 | compound 7 | Yb | 9:1 | 4.73 | 5.20 | 940 | 0.041 |
| Example 33 | compound 8 | Yb | 9:1 | 4.38 | 5.34 | 1150 | 0.014 |
| Example 34 | compound 9 | Yb | 9:1 | 4.32 | 5.41 | 1190 | 0.014 |
| Example 35 | compound 10 | Yb | 9:1 | 4.37 | 5.37 | 1100 | 0.019 |
| Example 36 | compound 11 | Yb | 9:1 | 4.42 | 5.28 | 1010 | 0.020 |
| Example 37 | compound 9 | Yb | 99:1 | 4.66 | 4.96 | 930 | 0.021 |
| Example 38 | compound 9 | Yb | 19:1 | 4.40 | 5.27 | 1090 | 0.018 |
| Example 39 | compound 9 | Yb | 7:3 | 4.53 | 5.04 | 950 | 0.024 |
| Example 40 | compound 1 | Li | 99:1 | 4.64 | 5.01 | 970 | 0.035 |
| Example 41 | compound 2 | Li | 99:1 | 4.68 | 5.11 | 1030 | 0.029 |
| Example 42 | compound 3 | Li | 99:1 | 4.75 | 4.88 | 1000 | 0.036 |
| Example 43 | compound 4 | Li | 99:1 | 4.92 | 4.72 | 960 | 0.040 |
| Example 44 | compound 5 | Li | 99:1 | 4.84 | 4.83 | 900 | 0.039 |
| Example 45 | compound 6 | Li | 99:1 | 4.72 | 4.90 | 920 | 0.052 |
| Example 46 | compound 7 | Li | 99:1 | 5.04 | 4.82 | 880 | 0.037 |
| Example 47 | compound 8 | Li | 99:1 | 4.68 | 5.01 | 990 | 0.037 |
| Example 48 | compound 9 | Li | 99:1 | 4.59 | 5.18 | 1070 | 0.028 |
| Example 49 | compound 10 | Li | 99:1 | 4.66 | 5.20 | 1020 | 0.031 |
| Example 50 | compound 11 | Li | 99:1 | 4.84 | 5.04 | 1000 | 0.039 |
| Comparative example 13 | compound 12 | Yb | 9:1 | 5.79 | 4.38 | 720 | 0.141 |
| Comparative example 14 | compound 13 | Yb | 9:1 | 6.52 | 4.18 | 400 | 0.231 |
| Comparative example 15 | compound 14 | Yb | 9:1 | 6.67 | 3.98 | 310 | 0.322 |
| Comparative example 16 | compound 15 | Yb | 9:1 | 5.52 | 4.41 | 730 | 0.171 |
| Comparative example 17 | compound 16 | Yb | 9:1 | 6.38 | 3.55 | 430 | 0.293 |
| Comparative example 18 | compound 12 | Yb | 99:1 | 6.01 | 4.53 | 750 | 0.152 |
| Comparative example 19 | compound 12 | Yb | 19:1 | 5.95 | 4.48 | 740 | 0.150 |
| Comparative example 20 | compound 12 | Li | 99:1 | 5.77 | 4.71 | 690 | 0.142 |
| Comparative example 21 | compound 13 | Li | 99:1 | 6.49 | 4.21 | 370 | 0.246 |
| Comparative example 22 | compound 14 | Li | 99:1 | 6.92 | 3.12 | 290 | 0.490 |
| Comparative example 23 | compound 15 | Li | 99:1 | 5.70 | 4.16 | 690 | 0.204 |
| Comparative example 24 | compound 16 | Li | 99:1 | 6.60 | 3.18 | 380 | 0.411 |

### Example 51

A transparent, electrically conductive ITO film with a thickness of 165 nm was evaporated on a glass substrate (manufactured by Geomatec Co., Ltd., 11 Ω/□, sputtered product) to prepare an anode, and it was then cut to a size of 38 mm × 46 mm and etched. The resulting substrate was subjected to ultrasonic cleaning for 15 minutes using SEMICOCLEAN 56 (trade name, manufactured by Furuuchi Chemical Corporation) and then rinsed with ultrapure water. Immediately before subjecting it to organic EL element production, this substrate was subjected to UV ozone treatment for 1 hour and placed in a vacuum evaporation instrument, which was then evacuated to an internal vacuum of 5 × 10⁻⁴ Pa or less. A hole injection layer was formed first by evaporating 5 nm of p-D1 by resistance heating. Then, on top of the hole injection layer, a light emitting unit (first light emitting unit) composed of a hole transporting layer, a light emitting layer, and an electron transporting layer was formed.

Specifically, HT-1 was evaporated to 50 nm to form a hole transporting layer, and then a light emitting layer was formed by evaporating a mixture layer of the host material H-1 and the dopant material D-1 in such a manner that the evaporated layer had a doping concentration of 5 wt% and a thickness of 20 nm, followed by forming an electron transporting layer by evaporating ET-1 and 2E-1 to a thickness of 35 nm in such a manner that the evaporation rate ratio between ET-1 and 2E-1 was 1:1.

Then, on top of the first light emitting unit, an N-type charge generation layer was formed by evaporating the compound 1 and metallic element Yb, which was used as dopant material, to a thickness of 10 nm in such a manner that the evaporation rate ratio between the compound 1 and Yb was 9:1, and then a p-type charge generation layer was formed by evaporating p-D1 to a thickness of 10 nm.

Following the formation of the charge generation layers, a second light emitting unit was formed in the same manner as the first light emitting unit. Subsequently, an electron injection layer was formed by evaporating the compound 1 and metallic element Yb, which was used as dopant material, to a thickness of 10 nm in such a manner that the evaporation rate ratio between the compound 1 and Yb was 9:1, and then a cathode was formed by evaporating aluminum to a thickness of 60 nm, thereby producing an organic EL element of a 5 mm × 5 mm square.

This organic EL element was examined by the evaluation methods described above, and results showed an initial driving voltage of 8.93 V, an external quantum efficiency (luminance efficiency) of 10.48%, a service life of 2,190 hours, and a voltage increase of 0.009 V in a test for driving it for 100 hours at room temperature.

### Examples 52 to 75 and Comparative examples 25 to 36

Except for using the compound and evaporation rate ratio between the compound and the metallic element given in Table 3, the same procedure as in Example 51 was carried out to produce an organic EL element. Results obtained in each Example and Comparative example are given in Table 3.

### [Table 3]

**[Table 3]**

| | compound in N-type charge generation layer | metallic element (dopant) | compound : metallic element evaporation rate ratio | driving voltage (V) | external quantum efficiency (%) | durability (h) | voltage increase during 100 hour driving at 10 mA/cm² (V) |
|---|---|---|---|---|---|---|---|
| Example 51 | compound 1 | Yb | 9:1 | 8.93 | 10.48 | 2190 | 0.009 |
| Example 52 | compound 2 | Yb | 9:1 | 8.89 | 10.55 | 2250 | 0.008 |
| Example 53 | compound 3 | Yb | 9:1 | 8.98 | 10.36 | 2090 | 0.011 |
| Example 54 | compound 4 | Yb | 9:1 | 9.42 | 10.03 | 2030 | 0.019 |
| Example 55 | compound 5 | Yb | 9:1 | 9.16 | 10.36 | 2100 | 0.013 |
| Example 56 | compound 6 | Yb | 9:1 | 9.01 | 10.41 | 2070 | 0.017 |
| Example 57 | compound 7 | Yb | 9:1 | 9.56 | 9.79 | 1980 | 0.024 |
| Example 58 | compound 8 | Yb | 9:1 | 8.80 | 10.54 | 2200 | 0.009 |
| Example 59 | compound 9 | Yb | 9:1 | 8.72 | 10.62 | 2240 | 0.006 |
| Example 60 | compound 10 | Yb | 9:1 | 8.86 | 10.51 | 2170 | 0.008 |
| Example 61 | compound 11 | Yb | 9:1 | 8.90 | 10.44 | 2180 | 0.010 |
| Example 62 | compound 9 | Yb | 99:1 | 9.51 | 9.66 | 1790 | 0.015 |
| Example 63 | compound 9 | Yb | 19:1 | 9.02 | 10.04 | 2030 | 0.010 |
| Example 64 | compound 9 | Yb | 7:3 | 9.24 | 9.89 | 1660 | 0.018 |
| Example 65 | compound 1 | Li | 99:1 | 9.05 | 10.33 | 2060 | 0.013 |
| Example 66 | compound 2 | Li | 99:1 | 8.97 | 10.27 | 2130 | 0.010 |
| Example 67 | compound 3 | Li | 99:1 | 9.12 | 10.15 | 1980 | 0.016 |
| Example 68 | compound 4 | Li | 99:1 | 9.63 | 10.01 | 1880 | 0.018 |
| Example 69 | compound 5 | Li | 99:1 | 9.18 | 10.22 | 2000 | 0.020 |
| Example 70 | compound 6 | Li | 99:1 | 9.07 | 9.97 | 1930 | 0.017 |
| Example 71 | compound 7 | Li | 99:1 | 9.77 | 9.67 | 1760 | 0.033 |
| Example 72 | compound 8 | Li | 99:1 | 9.19 | 9.85 | 1930 | 0.014 |
| Example 73 | compound 9 | Li | 99:1 | 8.90 | 10.26 | 2120 | 0.009 |
| Example 74 | compound 10 | Li | 99:1 | 8.99 | 10.19 | 2050 | 0.011 |
| Example 75 | compound 11 | Li | 99:1 | 9.06 | 10.06 | 2020 | 0.013 |
| Comparative example 25 | compound 12 | Yb | 9:1 | 11.52 | 8.56 | 1520 | 0.112 |
| Comparative example 26 | compound 13 | Yb | 9:1 | 13.52 | 8.10 | 820 | 0.193 |
| Comparative example 27 | compound 14 | Yb | 9:1 | 14.21 | 7.71 | 580 | 0.293 |
| Comparative example 28 | compound 15 | Yb | 9:1 | 11.03 | 8.92 | 1630 | 0.106 |
| Comparative example 29 | compound 16 | Yb | 9:1 | 12.84 | 6.16 | 710 | 0.204 |
| Comparative example 30 | compound 12 | Yb | 99:1 | 11.98 | 8.54 | 1400 | 0.089 |
| Comparative example 31 | compound 12 | Yb | 19:1 | 11.83 | 8.39 | 1420 | 0.133 |
| Comparative example 32 | compound 12 | Li | 99:1 | 11.67 | 9.21 | 1490 | 0.132 |
| Comparative example 33 | compound 13 | Li | 99:1 | 13.49 | 8.22 | 770 | 0.232 |
| Comparative example 34 | compound 14 | Li | 99:1 | 14.98 | 6.52 | 530 | 0.341 |
| Comparative example 35 | compound 15 | Li | 99:1 | 11.76 | 8.35 | 1550 | 0.142 |
| Comparative example 36 | compound 16 | Li | 99:1 | 13.13 | 5.99 | 640 | 0.308 |

For Examples 1 to 25, evaluation results of single charge elements including compounds as represented by the general formula (1) along with alkali metallic element Li or rare earth metallic element Yb are shown. For Comparative examples 1 to 12, evaluation results of light emitting elements including compounds 12 to 16 other than those represented by the general formula (1) along with alkali metallic element Li or rare earth metallic element Yb are shown. As compared to Comparative examples, the initial driving voltage was lower and the increase in driving voltage was smaller in Examples. It is considered that this is because the compounds 1 to 11 represented by the general formula (1) undergo stronger interactions such as coordination to alkali metal elements or rare earth metal elements as compared with the compounds 12 to 16, thereby realizing a higher charge generation efficiency and higher electron transport ability to serve for stabilizing the carrier balance and suppressing the voltage increase during driving.

Furthermore, Examples 1 to 14 gave evaluation results of single charge elements including compounds as represented by the general formula (1) along with rare earth metal element Yb, whereas Examples 15 to 25 gave evaluation results of single charge elements including alkali metallic element Li instead of Yb. As can be seen from these results, compounds as represented by the general formula (1) serve to form layers that are higher in stability and smaller in increase in driving voltage, when they are used along with a rare earth metal element.

Examples 26 to 50 gave evaluation results of light emitting elements that were produced by applying the organic layers used in Examples 1 to 25. Comparative examples 13 to 24 gave evaluation results of light emitting elements that were produced by applying the organic layers used in Comparative examples 1 to 12. As compared with Comparative examples, each Example exhibited a lower driving voltage, higher external quantum efficiency, improved durability, and smaller increase in driving voltage. It can be seen from this that, as in the case of the single charge elements, compounds as represented by the general formula (1) serve to produce light emitting elements that are high in efficiency, small in increase in driving voltage, and high in stability.

Furthermore, Examples 25 to 39 gave evaluation results of light emitting elements including compounds as represented by the general formula (1) along with rare earth metallic element Yb, whereas Examples 40 to 50 gave evaluation results of light emitting elements including alkali metallic element Li instead of Yb. As can be seen from these results, compounds as represented by the general formula (1) serve to form layers that are higher in stability and smaller in increase in driving voltage, when they are used along with rare earth metal elements.

Examples 51 to 75 gave evaluation results of tandem type light emitting elements that were produced by applying the light emitting elements used in Examples 26 to 50. Comparative examples 25 to 36 gave evaluation results of tandem type light emitting elements that were produced by applying the light emitting elements used in Comparative examples 13 to 24. As compared with Comparative examples, each Example exhibited a lower driving voltage, higher external quantum efficiency, improved durability, and smaller increase in driving voltage. It can be seen from this that, as in the case of the light emitting elements used in Examples 26 to 50 and Comparative examples 13 to 24, compounds as represented by the general formula (1) serve to produce tandem type light emitting elements that are high in efficiency, small in increase in driving voltage, and high in stability.

Furthermore, Examples 51 to 64 gave evaluation results of tandem type light emitting elements including compounds as represented by the general formula (1) along with rare earth metallic element Yb, whereas Examples 65 to 75 gave evaluation results of tandem type light emitting elements including alkali metallic element Li instead of Yb. As can be seen from these results, compounds as represented by the general formula (1) serve to form layers that are higher in stability and smaller in increase in driving voltage, when they are used along with rare earth metal elements.

## Claims

1. A compound as represented by the general formula (1) given below: 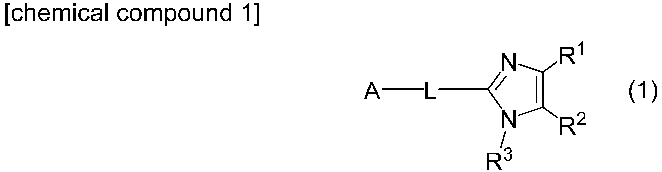 wherein, in the general formula (1), R¹ and R² are each independently one selected from the group consisting of substituted or unsubstituted aryl groups and substituted or unsubstituted heteroaryl groups, R¹ and R² may be linked to each other to form a ring structure, R³ is selected from the group consisting of substituted or unsubstituted alkyl groups, substituted or unsubstituted aryl groups, and substituted or unsubstituted heteroaryl groups, L denotes a substituted or unsubstituted arylene group or a substituted or unsubstituted heteroarylene group, and A denotes a structure as represented by the general formula (2) or (3) given below: 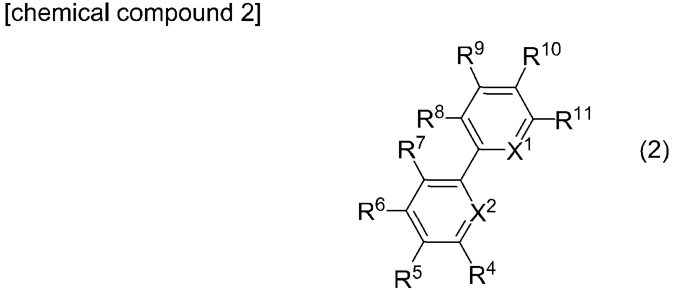 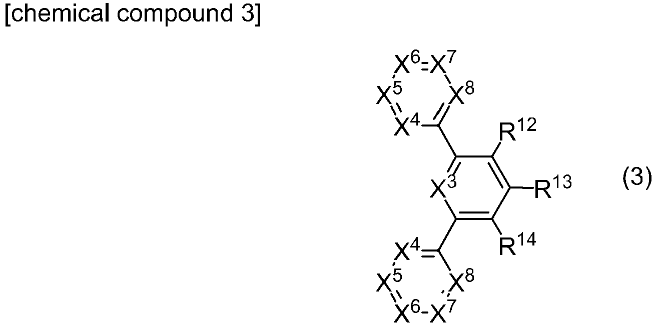 wherein, in the general formulae (2) and (3), R⁴ to R¹⁴ are each independently one selected from the group consisting of a hydrogen atom, substituted or unsubstituted alkyl groups, substituted or unsubstituted alkoxy groups, substituted or unsubstituted aryl groups, and substituted or unsubstituted heteroaryl groups, where one of R⁴ to R¹¹ is directly bonded to L, and one of R¹² to R¹⁴ is directly bonded to L, R⁷ and R⁸ may be linked to each other to form a ring structure, and X¹ to X⁸ are each independently a carbon atom or a nitrogen atom, where at least either of X¹ and X² is a nitrogen atom and at least one of X³ to X⁸ is a nitrogen atom.

2. A compound as set forth in claim 1, wherein the total number of carbon atoms present in R¹ and R² in the general formula (1) is 8 to 20.

3. A compound as set forth in claim 1, wherein the number of carbon atoms present in R³ in the general formula (1) is 1 to 12.

4. A compound as set forth in claim 1, wherein L in the general formula (1) is a phenylene group, a naphthylene group, or a biphenylene group.

5. A compound as set forth in claim 1, wherein A in the general formula (1) is represented by any one of the general formulae (4) to (6) given below: 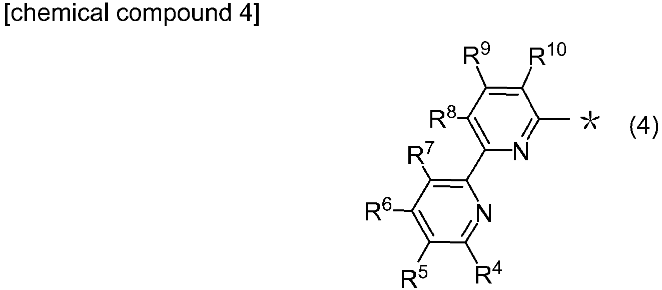 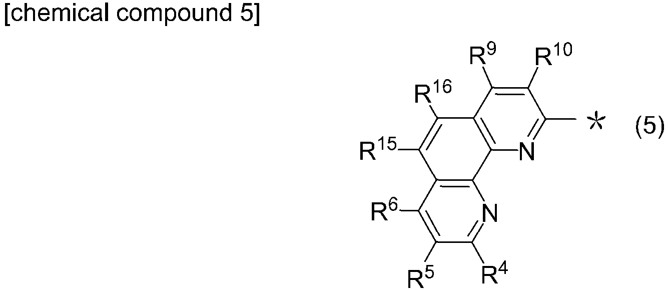 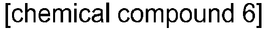 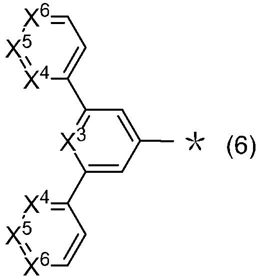 wherein, in the general formulae (4) to (6), R⁴ to R¹⁰, R¹⁵, and R¹⁶ are each independently one selected from the group consisting of a hydrogen atom, substituted or unsubstituted alkyl groups, substituted or unsubstituted alkoxy groups, substituted or unsubstituted aryl groups, and substituted or unsubstituted heteroaryl groups, X³ to X⁶ are each independently a carbon atom or a nitrogen atom, where at least one of X³ to X⁶ is a nitrogen atom, and the symbol * represents a site for bonding to L.

6. A compound as set forth in claim 5, wherein R⁵ to R¹⁰, R¹⁵, and R¹⁶ in the general formulae (4) and (5) are hydrogen atoms.

7. A compound as set forth in claim 5, wherein, in the general formula (6), X³ is a nitrogen atom and any one of X⁴ to X⁶ is a nitrogen atom.

8. A light emitting element comprising at least an electron transporting layer and a light emitting layer located between an anode and a cathode to emit light by using electrical energy, wherein the electron transporting layer contains a compound as set forth in claim 1.

9. A light emitting element as set forth in claim 8, wherein the electron transporting layer further contains an alkali metal atom or a rare earth metal atom.

10. A light emitting element comprising at least a charge generation layer and a light emitting layer located between an anode and a cathode to emit light by using electrical energy, wherein the charge generation layer contains a compound as set forth in claim 1.

11. A light emitting element as set forth in claim 10, wherein the charge generation layer further contains a phenanthroline derivative.

12. A light emitting element as set forth in claim 10, wherein the charge generation layer further contains an alkali metal atom, a copper group element atom, or a rare earth metal atom.

13. A light emitting element as set forth in claim 10, wherein the charge generation layer further contains an alkali metal atom, with the alkali metal atom being Li.

14. A light emitting element as set forth in claim 10, wherein the charge generation layer further contains a rare earth metal atom, with the rare earth metal atom being Yb.

15. A light emitting element comprising at least an electron injection layer and a light emitting layer located between an anode and a cathode to emit light by using electrical energy, wherein the electron injection layer contains a compound as set forth in claim 1.

16. A light emitting element as set forth in claim 15, wherein the electron injection layer further contains an alkali metal atom or a rare earth metal atom.

17. A display device comprising a light emitting element as set forth in any one of claims 8 to 11, 15, and 16.

18. An illumination device comprising a light emitting element as set forth in any one of claims 8 to 11, 15, and 16.

19. A photosensitizer comprising a light emitting element as set forth in any one of claims 8 to 11, 15, and 16.
